# EUROPÄISCHE PATENTSCHRIFT

(11) **EP 1 556 695 B1**
(45) Veröffentlichungstag und Bekanntmachung des Hinweises auf die Patenterteilung: **10.08.2011**
(21) Anmeldenummer: 03793767.9
(22) Anmeldetag: 28.08.2003
(51) Int. Cl.: G01N 33/543, G01N 21/77

(54) **ANALYTISCHE PLATTFORM UND NACHWEISVERFAHREN MIT GEGEBENENFALLS NACH FRAKTIONIERUNG IN EINER PROBE NACHZUWEISENDEN ANALYTEN ALS IMMOBILISIERTEN SPEZIFISCHEN BINDUNGSPARTNERN**
ANALYTICAL PLATFORM AND IDENTIFICATION METHOD WITH ANALYTES, WHICH ARE TO BE IDENTIFIED IN A SAMPLE OPTIONALLY AFTER FRACTIONATION AND WHICH SERVE AS IMMOBILIZED SPECIFIC BINDING PARTNERS
PLATE-FORME ANALYTIQUE ET PROCEDE D'IDENTIFICATION PAR DES ANALYTES A DECELER DANS UN ECHANTILLON EVENTUELLEMENT APRES FRACTIONNEMENT ET SE PRESENTANT SOUS FORME DE PARTENAIRES DE LIAISON SPECIFIQUES

(30) Priorität: 03.09.2002 CH 150302; 27.01.2003 CH 115002003
(43) Veröffentlichungstag der Anmeldung: 27.07.2005
(73) Patentinhaber: Bayer Technology Services GmbH, 51368 Leverkusen (DE)
(72) Erfinder: PAWLAK, Michael, 72147 Nehren (DE); SCHICK, Eginhard, 79618 Rheinfelden (DE); OROSZLAN, Peter, CH-4054 Basel (CH)
(86) Internationale Anmeldenummer: PCT/EP2003/009562
(87) Internationale Veröffentlichungsnummer: WO 2004/023143

(56) Entgegenhaltungen:
- WO-A-01/92870
- WO-A-02/20873
- WO-A-02/46756
- WO-A-95/33197
- WO-A-96/35940
- WO-A-98/21571
- WO-A-03/020488
- PAWLAK M ET AL: "Functional immobilization of biomembrane fragments on planar waveguides for the investigation of side-directed ligand binding by surface-confined fluorescence." FARADAY DISCUSSIONS. ENGLAND 1998, Nr. 111, 1998, Seiten 273-288;discussion 331 - 343, XP001156169 ISSN: 1359-6640
- PAWLAK M ET AL: "ZEPTOSENS' PROTEIN MICROARRAYS: A NOVEL HIGH PERFORMANCE MICROARRAY PLATFORM FOR LOW ABUNDANCE PROTEIN ANALYSIS" PROTEOMICS, XX, XX, Bd. 2, Nr. 4, April 2002 (2002-04), Seiten 383-393, XP009021061

## Beschreibung

Die vorliegende Erfindung betrifft eine analytische Plattform und ein damit ausgeführtes Verfahren zur Untersuchung einer Vielzahl von Proben auf in den Proben enthaltene, als Teilnehmer an spezifischen Bindungsreaktionen biologisch relevante Verbindungen als Analyten, dadurch gekennzeichnet, dass
- Besagte Proben oder Fraktionen besagter Proben mit den darin enthaltenen, nachzuweisenden Analyten, als einer ersten Vielzahl von spezifischen Bindungspartnern direkt oder nach zusätzlichen Verdünnungen der Proben oder der Fraktionen in mindestens einem ein- oder zweidimensisonalen Array in diskreten Messbereichen auf einer Evaneszentfeld-Sensorplattform als festem Träger aufgetragen werden, wobei unterschiedliche Proben oder Fraktionen oder unterschiedliche Verdünnungen von Proben oder Fraktionen in unterschiedlichen diskreten Messbereichen angeordnet werden,
- eine oder mehrere Nachweissubstanzen, als eine zweite Vielzahl spezifischer Bindungspartner, zum spezifischen Nachweis von einem oder mehreren in den Proben enthaltenen Analyten, aus besagter ersten Vielzahl spezifischer Bindungspartner, in einem einzigen oder mehreren Schritten einer spezifischen Bindungsreaktion mit den in besagten diskreten Messbereichen aufgetragenen Proben oder deren Fraktionen oder deren Verdünnungen in Kontakt gebracht werden,
- Änderungen von optoelektronischen Signalen als Folge der Bindung von Nachweissubstanzen an in diskreten Messbereichen enthaltene Analyten, im evaneszenten Feld der Evaneszentfeld-Sensorplattform, ortsaufgelöst gemessen werden und
aus der relativen Grösse der Änderungen besagter optoelektronischer Signale aus den jeweiligen Messbereichen qualitativ und / oder quantitativ das Vorhandensein der dort spezifisch nachzuweisenden Analyten bestimmt wird.

Dabei können besagte Änderungen von optoelektronischen Signalen, als Folge der Bindung von Nachweissubstanzen an in diskreten Messbereichen in den Proben enthaltene Analyten, im evaneszenten Feld der Evaneszentfeld-Sensorplattform, beispielsweise aus dem Vergleich der gleichzeitig gemessenen Signale aus unterschiedlichen Messbereichen, welche entsprechende nachzuweisende Analyten enthalten (in bekannter oder unbekannter Konzentration bzw. Menge) mit den Signalen aus Messbereichen, welche entsprechende nachzuweisende Analyten nicht enthalten, bestimmt werden. Für die Bestimmung besagter Signaländerungen können auch die Signale aus Messbereichen mit unbekannter Konzentration mit denjenigen Messbereichen mit darin in bekannter Konzentration enthaltenen Analyten benutzt werden. Für den Fall der kontinuierlichen Signalerfassung vor und während der Zugabe der entsprechenden Nachweissubstanzen und deren Bindung an die entsprechenden Analyten enthaltenden Messbereiche kann eine entsprechende Änderung auch aus dem zeitlichen Verlauf der Signale aus den entsprechenden Messbereichen bestimmt werden.

Im folgenden bezieht sich die Bezeichnung "einer Probe" bzw. "einer Immobilisierungsprobe" bzw "immoblisierte Probe" jeweils auch auf zwei oder mehr, d.h. auf eine Vielzahl solcher Proben bzw. "Immobiliserungsproben", sofern nicht ausdrücklich anders vermerkt.

Für zahlreiche Anwendungsgebiete ist es erforderlich, eine Vielzahl von biologisch relevanten Analyten in einer komplexen Probe zu bestimmen, beispielsweise in diagnostischen Verfahren zur Bestimmung des Gesundheitszustandes eines Individuums oder in der Pharmaforschung und -entwicklung zur Bestimmung der Beeinflussung eines Organismus und dessen komplexer Funktionsweise durch Zuführung biologisch aktiver Verbindungen.

Während bekannte analytische Trennverfahren im allgemeinen dahingehend optimiert sind, innerhalb möglichst kurzer Zeit eine möglichst grosse Anzahl von in einer gegebenen Probe enthaltenen Verbindungen gemäss eines vorgegebenen physikalisch-chemischen Parameters, wie beispielsweise des Molekulargewichts oder des Quotienten aus molekularer Ladung und Masse, aufzutrennen, beruhen Bioaffinitäts-Nachweisverfahren darauf, mit jeweils einem biologischen oder biochemischen oder synthetischen Erkennungselement möglichst hoher Spezifizität den entsprechenden (einzelnen) Analyten hochselektiv in einer komplex zusammengesetzten Probe zu erkennen und zu binden. Der Nachweis einer Vielzahl unterschiedlicher Verbindungen erfordert also die Verwendung einer entsprechenden Anzahl unterschiedlicher spezifischer Erkennungselemente.

Ein Nachweisverfahren basierend auf Bioaffinitätsreaktionen kann sowohl in einer homogenen Lösung als auch an der Oberfläche eines festen Trägers erfolgen. Je nach dem spezifischen Aufbau des Verfahrens sind nach Bindung der Analyten an die entsprechenden Erkennungselemente und gegebenenfalls weitere Nachweissubstanzen sowie gegebenenfalls zwischen verschiedenen Verfahrensschritten jeweils Waschschritte notwendig, um die gebildeten Komplexe aus den Erkennungselementen und den nachzuweisenden Analyten sowie gegebenenfalls weiteren Nachweissubstanzen vom Rest der Probe und der gegebenenfalls eingesetzten zusätzlichen Reagentien zu trennen.

Relativ weit verbreitet sind inzwischen Verfahren zum gleichzeitigen Nachweis einer Vielzahl unterschiedlicher Nukleinsäuren in einer Probe mithilfe entsprechender komplementärer, auf einem festen Träger in diskreten, räumlich getrennten Messbereichen immobiliserter Nukleinsäuren als Erkennungselementen. Beispielsweise sind, basierend auf einfachen Glas- oder Mikroskop-Plättchen, Arrays von Oligonukleotiden als Erkennungselementen mit einer sehr hohen Feature-Dichte (Dichte von Messbereichen auf einem gemeinsamen festen Träger) bekannt. Beispielsweise werden in der US-Patentschrift No. 5,445,934 (Affymax Technologies) Arrays von Oligonukleotiden mit einer Dichte von mehr als 1000 Features pro Quadratzentimeter beschrieben und beansprucht.

Seit kurzem häufen sich auch Beschreibungen von Arrays und damit ausgeführter Nachweisverfahren ähnlicher Art zur gleichzeitigen Bestimmung einer Vielzahl von Proteinen, beispielsweise in der US-Patentschrift No. 6,365,418 B1.

In den Patentschriften für derartige sogenannte "Mikroarrays", sowohl für den Nachweis von Nukleinsäuren als auch anderer Biopolymere, wie beispielsweise Proteine, wird jeweils beschrieben, dass eine Vielzahl unterschiedlicher spezifischer Erkennungselemente in diskreten Messbereichen zur Erzeugung eines Arrays für die Analyterkennung immobilisiert und anschliessend damit die zu untersuchende Probe mit den darin in einer (gegebenenfalls komplexen) Mischung vorhandenen Analyten in Kontakt gebracht wird. Den bekannten Beschreibungen folgend, liegen unterschiedliche spezifische Erkennungselemente dabei jeweils in einer möglichst hochreinen Form in unterschiedlichen diskreten Messbereichen vor, so dass an Messbereiche mit unterschiedlichen Erkennungselementen im allgemeinen unterschiedliche Analyten aus der Probe binden.

Diese Art bekannter Assays erfordert, dass die in möglichst hochreiner Form zu immobilisierenden spezifischen Erkennungselemente mittels teilweise sehr aufwendiger Arbeitsschritte angereichert werden. Da sich unterschiedliche Erkennungselemente mehr oder minder stark in ihren physikalisch-chemischen Eigenschaften (z. B. in ihrer Polarität) unterscheiden, gibt es auch entsprechende Unterschiede in den Bedingungen für eine optimale Immobilisierung dieser Erkennungselemente, beispielsweise durch Adsorption oder kovalente Bindung, in diskreten Messbereichen auf einem gemeinsamen festen Träger, gegebenenfalls auf einer darauf aufgebrachten Haftvermittlungsschicht. Demzufolge können die zur Immobilisierung einer Vielzahl unterschiedlicher Erkennungselemente gewählten Immobilisierungsbedingungen (wie z. B. Art der Haftvermittlungsschicht) kaum gleichzeitig für alle Erkennungselemente ein Optimum, sondern lediglich einen Kompromiss zwischen den Immobilisierungseigenschaften der verschiedenen Erkennungselemente darstellen.

Bei dieser Art des Assays ist ausserdem nachteilig, dass zum Nachweis von Analyten in einer Vielzahl unterschiedlichen Proben im allgemeinen die Bereitstellung einer entsprechenden Anzahl diskreter Arrays von Erkennungselementen, denen die unterschiedlichen Proben zugeführt werden, auf gemeinsamen oder diskreten Trägem erforderlich ist. Zur Untersuchung einer Vielzahl unterschiedlicher Proben bedeutet dieses den Bedarf einer hohen Anzahl diskreter Arrays, deren Herstellung relativ aufwendig ist.

Zwar ist beschrieben worden, dass beispielsweise die von immobilisierten Oligonukleotiden und in einer zugeführten Probe enthaltenen, zu den immobilisierten komplementären Oligonukleotide gebildeten Hybride unter geeigneten Dissoziationsbedingungen mit hoher Effizienz wieder dissoziiert werden können und so eine Erkennungsoberfläche regeneriert werden kann, jedoch kann kaum eine hundertprozentige Regenerierungsfähigkeit garantiert werden. Im Falle von Bioaffinitätskomplexen mit Proteinen besteht oft sogar keine Reversibilität des Bindungschrittes, d.h. keine Möglichkeit zur Regenerierung der Erkennungsoberfläche.

Es besteht daher das Bedürfnis nach einem geänderten Assay-Aufbau, welcher es ermöglicht, eine Vielzahl von Proben in einem Array auf einem gemeinsamen Träger gleichzeitig auf in den Proben enthaltene Analyten zu untersuchen. Dazu wäre es zweckmässig, nicht die unterschiedlichen spezifischen Erkennungselemente, sondern die zu untersuchenden Proben selbst direkt, d.h. unbehandelt, oder nach möglichst wenigen Vorbereitungsschritten in diskreten Messbereichen in einem Array auf einem Träger aufzutragen. Ein solcher Assay-Aufbau soll nachfolgend als eine "invertierte Assay-Architektur" bezeichnet werden.

In der US-Patentschrift No. 6,316,267 wird ein Verfahren beschrieben, in dem PolyAminosäuren in einer Probenmischung (wobei es sich offensichtlich auch um eine komplex zusammengesetzte Probe handeln kann) beispielsweise auf einer festen oder quasi-festen ("semi-solid") Probenmatrix aufgetragen werden. Der Nachweisschritt erfolgt jedoch nicht durch ein Bioaffinitätsassay, sondern durch Anfärbung mit einer Reagentienmischung, welche bestimmte, in der Patentschrift benannte Metallkomplexe enthält. Hierbei handelt es sich nicht um einen spezifischen Analytnachweis.

In der US-Patentschrift 6,287,768 wird ein Verfahren beschrieben, dem zufolge verschiedenartige nachzuweisende RNA-Moleküle aus einer biologischen Probe isoliert, durch der Grösse nach aufgetrennt, auf einem festen Träger aufgebracht und anschliessend dort, beispielsweise in einem Hybridisierungsassay durch Hybridisierung mit bekannten, komplementären Polynukleotiden, nachgewiesen werden. Gemäss der Beschreibung können die aus einem Organismus isolierten nachzuweisenden RNA-Moleküle dabei entweder direkt dem weiteren Nachweisverfahren unterzogen werden, wenn sie in hoher Konzentration vorhanden sind, oder müssen vor dem eigentlichen Nachweisverfahren noch mit bekannten Vervielfältigungsverfahren (z. B. mittels PCR, "Polymerase Chain Reaction") vermehrt werden. Dieses bedeutet, dass mit dem dort beschriebenen Verfahren, ohne zusätzliche Vervielfätigungsverfahren, eine vollständige Analyse der verschiedenen erzeugten Fraktionen nicht möglich ist.

Auch wenn das in dem US-Patent 6,287,768 vorgeschlagene Verfahren die Möglichkeit eröffnet, RNA aus verschiedenen Proben gleichzeitig zu bestimmen, erfordert es immer noch eine Reihe aufwendiger Probenaufbereitungsschritte und insbesondere die Isolation aus der biologischen Probenmatrix und eine nachfolgende Trennung nach der Molekülgrösse. Indem das beanspruchte Verfahren, welches nur am Beispiel von RNA beschrieben wird, mindestens die Isolation aus der ursprünglichen Probenmatrix und nachfolgende Auftrennung der nachzuweisenden Biopolymere nach deren Grösse voraussetzt, ist klar, dass diese nach diesem Auftrennungsschritt, vor dem Nachweisschritt, in einem von der ursprünglichen Probenmatrix (wie beispielsweise Blut oder Serum) deutlich unterschiedlichen, weniger komplexen Medium vorliegen.

Die Empfindlichkeit der vorangehend im Stand der Technik beschriebenen Verfahren ist offensichtlich nicht ausreichend, um eine Vielzahl in einer Probe enthaltener Analyten mit einer "inversen Assay-Architektur" mit einer ausreichenden Nachweisgrenze zu bestimmen.

Die Anregung von zum Analytnachweis eingesetzten "Nachweiskomponenten" (wie beispielsweise radioaktiven Isotopen oder Chromophoren mit einer charakteristischen Absorption und / oder Lumineszenz bzw. Fluoreszenz) und das Auslesen der Signale von Arrays der genannten Art beruht auf klassischen, beispielweise optischen Anordnungen und Detektionsmethoden. Die klassischen Messmethoden, wie beispielsweise Absorptions- oder Fluoreszenzmessungen, beruhen im allgemeinen auf der direkten Beleuchtung eines Probevolumens in einem Probenbehältnis oder eines Messfeldes auf einer Innenwand eines Probenbehältnisses einer flüssigen Probe. Diese Anordnungen haben den Nachteil, dass neben dem Anregungsvolumen oder der Anregungsfläche, innerhalb derer ein Signal zum Nachweis eines Analyten erzeugt werden soll, im allgemeinen ein erheblicher Anteil der Umgebung von Anregungslicht erfasst wird, was zur nachteiligen Erzeugung von störenden Untergrundsignalen führen kann.

Zur Erreichung tieferer Nachweisgrenzen sind zahlreiche Messanordnungen entwickelt worden, in denen der Nachweis des Analyten auf dessen Wechselwirkung mit dem evaneszenten Feld beruht, welches mit der Lichtleitung in einem optischen Wellenleiter verbunden ist. Koppelt man eine Lichtwelle in einen optischen Wellenleiter ein, der von optisch dünneren Medien, d.h. Medien mit niedrigerem Brechungsindex, umgeben ist, so wird sie durch Totalreflexion an den Grenzflächen der wellenleitenden Schicht geführt. In die optisch dünneren Medien tritt dabei ein Bruchteil des geführten Lichts ein. Diesen Anteil bezeichnet man als evaneszentes oder quergedämpftes Feld. Die Stärke des evaneszenten Feldes ist sehr stark abhängig von der Dicke der wellenleitenden Schicht selbst sowie vom Verhältnis der Brechungsindices der wellenleitenden Schicht und der sie umgebenden Medien. Bei dünnen Wellenleitern, d. h. Wellenleitern mit Schichtdicken von derselben oder niedrigerer Dicke als der zu führenden Wellenlänge, können diskrete Moden des geleiteten Lichts unterschieden werden. Derartige Verfahren haben den Vorteil, dass die Wechselwirkung des Anregungslichts mit dem Analyten auf die Eindringtiefe des evaneszenten Feldes ins angrenzende Medium, in der Grössenordnung von einigen hundert Nanometern, beschränkt ist und Störsignale aus der Tiefe des Mediums weitgehend vermieden werden können. Die ersten vorgeschlagenen derartigen Messanordnungen beruhten auf hochmultimodalen, selbsttragenden Einschichtwellenleitem, wie beispielsweise Fasern oder Plättchen aus transparentem Kunststoff oder Glas, mit Stärken von einigen hundert Mikrometern bis zu mehreren Millimetern.

Zur Verbesserung der Empfindlichkeit und gleichzeitig einfacheren Herstellung in Massenfabrikation wurden planare Dünnschichtwellenleiter vorgeschlagen. Ein planarer Dünnschichtwellenleiter besteht im einfachsten Fall aus einem Dreischichtsystem: Trägermaterial, wellenleitende Schicht, Superstrat (bzw. zu untersuchende Probe), wobei die wellenleitende Schicht den höchsten Brechungsindex besitzt.

Es sind verschiedene Verfahren für die Einkopplung von Anregungslicht in einen planaren Wellenleiter bekannt. Die am frühesten benutzten Verfahren beruhten auf Stirnflächenkopplung oder Prismenkopplung, wobei zur Verminderung von Reflexionen infolge von Luftspalten im allgemeinen eine Flüssigkeit zwischen Prisma und Wellenleiter aufgebracht wird. Diese beiden Methoden sind vor allem in Verbindung mit Wellenleitern relativ grosser Schichtdicke, d. h. insbesondere selbsttragenden Wellenleitern, sowie bei einem Brechungsindex des Wellenleiters von deutlich unter 2 geeignet. Zur Einkopplung von Anregungslicht in sehr dünne, hochbrechende wellenleitende Schichten ist demgegenüber die Verwendung von Koppelgittern eine wesentlich elegantere Methode.

Es können verschiedene Methoden zum Analytnachweis im evaneszenten Feld geführter Lichwellen in optischen Schichtwellenleitern unterschieden werden. Aufgrund des eingesetzten Messprinzips kann man beispielsweise zwischen Fluoreszenz- oder allgemeiner Lumineszenzmethoden auf der einen Seite und refraktiven Methoden andererseits unterscheiden. Hierbei können Verfahren zur Erzeugung einer Oberflächenplasmonenresonanz in einer dünnen Metallschicht auf einer dielektrischen Schicht mit niedrigerem Brechungsindex in die Gruppe der refraktiven Methoden mit einbezogen werden, sofern als Basis zur Bestimmung der Messgrösse der Resonanzwinkel des eingestrahlten Anregungslichts zur Erzeugung der Oberflächenplasmonenresonanz dient. Die Oberflächenplasmonenresonanz kann aber auch zur Verstärkung einer Lumineszenz oder zur Verbesserung des Signal-zu-Hintergrund-Verhältnisses in einer Lumineszenzmessung verwendet werden. Die Bedingungen zur Erzeugung einer Oberflächenplasmonenresonanz sowie zur Kombination mit Lumineszenzmessungen sowie mit wellenleitenden Strukturen sind vielfach in der Literatur beschrieben, beispielsweise in den US-Patenten US-P 5,478,755, US-P 5,841,143, US-P 5,006,716 und US-P 4,649,280.

Mit dem Begriff "Lumineszenz" wird in dieser Anmeldung die spontane Emission von Photonen im ultravioletten bis infraroten Bereich nach optischer oder nichtoptischer, wie beispielsweise elektrischer oder chemischer oder biochemischer oder thermischer Anregung, bezeichnet. Beispielsweise sind Chemilumineszenz, Biolumineszenz, Elektrolumineszenz und insbesondere Fluoreszenz und Phosphoreszenz unter dem Begriff "Lumineszenz" mit eingeschlossen.

Bei den refraktiven Messmethoden wird die Änderung des sogenannten effektiven Brechungsindex aufgrund molekularer Adsorption oder Desorption auf dem Wellenleiter zum Nachweis des Analyten benutzt. Diese Änderung des effektiven Brechungsindex wird, im Falle von Gitterkoppler-Sensoren, bestimmt aus der Änderung des Koppelwinkels für die Ein- oder Auskopplung von Licht in oder aus dem Gitterkoppler-Sensor, und im Falle von interferometrischen Sensoren aus der Änderung der Phasendifferenz zwischen dem in einem Sensorarm und einem Referenzarm des Interferometers geführten Messlichts.

Die genannten refraktiven Methoden haben den Vorteil, dass sie ohne Verwendung zusätzlicher Markierungsmoleküle, sogenannter molekularer Labels, eingesetzt werden können. Der Nachteil dieser labelfreien Methoden ist jedoch, dass die damit erzielbaren Nachweisgrenzen aufgrund der geringen Selektivität des Messprinzips, in Abhängigkeit von dem Molekulargewicht des Analyten auf pico- bis nanomolare Konzentrationsbereiche beschränkt sind, was für viele Anwendungen der modernen Spurenanalytik, beispielsweise für diagnostische Applikationen, nicht ausreichend ist.

Zur Erreichung noch tieferer Nachweisgrenzen erscheinen lumineszenz-basierende Methoden aufgrund grösserer Selektivität der Signalerzeugung besser geeignet. Dabei ist die Lumineszenzanregung auf die Eindringtiefe des evaneszenten Feldes in das optisch dünnere Medium, also auf die unmittelbare Umgebung des wellenleitenden Bereichs mit einer Eindringtiefe in der Grössenordnung von einigen hundert Nanometern ins Medium beschränkt. Dieses Prinzip wird evaneszente Lumineszenzanregung genannt.

Mittels hochbrechender Dünnschichtwellenleiter, in Kombination mit Lumineszenzdetektion, basierend auf einem nur einige hundert Nanometer dünnen wellenleitenden Film auf einem transparenten Trägermaterial, konnte in den letzten Jahren die Empfindlichkeit deutlich gesteigert werden. Beispielsweise wird in der WO 95/33197 eine Methode beschrieben, in der das Anregungslicht über ein Reliefgitter als diffraktives optisches Element in den wellenleitenden Film eingekoppelt wird. Die isotrop abgestrahlte Lumineszenz in der Eindringtiefe des evaneszenten Feldes befindlicher lumineszenzfähiger Substanzen wird mittels geeigneter Messvorrichtungen, wie zum Beispiel Photodioden, Photomultiplier oder CCD-Kameras, gemessen. Es ist auch möglich, den in den Wellenleiter rückgekoppelten Anteil der evaneszent angeregten Strahlung über ein diffraktives optisches Element, zum Beispiel ein Gitter, auszukoppeln und zu messen. Diese Methode ist zum Beispiel in der WO 95/33198 beschrieben.

In den vergangenen Jahren sind Weiterentwicklungen von planaren Dünnschichtwellenleitern als Sensorplattformen für "Mikroarrays" bekannt geworden, beispielsweise in den Internationalen Patentanmeldungen WO 01/13096, WO 01/43875, teilweise in Kombination mit entsprechend angepassten Fluidikstrukturen. Diese Patentanmeldeschriften werden hiermit vollumfänglich als Bestandteil der vorliegenden Anmeldung eingeführt. In der WO 01/79821 wird eine Dünnschicht-Wellenleiterstruktur beschrieben, welche eine Zweiphotonen-Anregung an der Oberfläche des Wellenleiters ermöglicht. In der WO 01/88511 werden eine Gitter-Wellenleiter-Struktur und ein damit ausgeführtes Messverfahren beschrieben, welche ein bildgebendes Verfahren zur Analytbestimmung mithilfe einer refraktiven Messmethode ermöglichen. Beide Patentanmeldeschriften werden hiermit ebenfalls als Bestandteil der vorliegenden Anmeldung eingeführt.- Den genannten Anordnungen ist gemeinsam, dass jeweils bekannte biologische oder biochemische oder synthetische Erkennungselemente für den Nachweis einer Vielzahl von Analyten in diskreten Messbereichen bekannter Lage auf dem Trägersubstrat, als Bestandteile eines oder mehrerer Arrays von Messbereichen, immobilisiert sind.

Es wurde nun überraschend gefunden, dass, bei geeigneter Wahl der physikalisch-chemischen Parameter (wie Schichtdicken, Brechungsindices der beteiligten Schichten) einer Evaneszentfeld-Sensorplattform, infolge der hohen Anregungslichtintensität an der Oberfläche und der gleichzeitigen Beschränkung dieses starken Anregungsfeldes auf die Eindringtiefe des evaneszenten Feldes in die benachbarten Medien, die erreichbare Empfindlichkeit für die Detektion molekularer Wechselwirkungen an der Oberfläche einer Evaneszentfeld-Sensorplattform ausreichend hoch ist, um eine Vielzahl von Proben, gegebenenfalls nach Fraktionierung und gegebenenfalls zusätzlichen Verdünnungen dieser Proben oder Fraktionen auf die darin enthaltenen Analyten, ohne zusätzliche Verfahrensschritte für eine Isolation von der restlichen Probenmatrix oder eine Vervielfältigung der nachzuweisenden Analyten (bezüglich ihrer Menge), nach direkter Auftragung dieser Fraktionen oder von Verdünnungen dieser Fraktionen auf besagter Evaneszentfeld-Sensorplattform zu analysieren. Damit wird ein einfaches Verfahren mit "invertierter Assay-Architektur bereitgestellt, welches es ermöglicht, eine Vielzahl von Analyten in einer Probe zu bestimmen, ohne weitere Änderungen der relativen molekularen Zusammensetzung der Probe nach einem Fraktionierungs- oder Auftrennschritt vorzunehmen.

Bei den zu untersuchenden Proben kann es sich beispielsweise (siehe auch weiter unten) um eine oder mehrere Zellen handeln, die zuvor aus einer grösseren Anzahl von Zellen selektiert werden, beispielsweise durch Zentrifugation, Filtration oder durch "Laser Capture Microdissection".

Im folgenden bezieht sich die Bezeichnung einer (einzelnen) Zelle für die auszuführenden Probenbehandlungsschritte jeweils auch auf eine Vielzahl von Zellen, sofern dieses nicht ausdrücklich anders vermerkt ist. Ebenso kann die Bezeichnung einer "Probe" auch jeweils deren mit einem geeigneten Trennverfahren hergestellte Fraktionen umfassen.

In einem ersten, für weitere Untersuchungsschritte im allgemeinen notwendigen Aufbereitungsschritt kann die Zelle lysiert werden. Das Lysat kann in einem geeigneten Lösungsmittel, beispielsweise einer Pufferlösung, gelöst sein und bekannte zugesetzte Beimischungen enthalten, beispielsweise Stabilisatoren wie Enzym-Inhibitoren, um einen Abbau enthaltener Biopolymere zu verhindern. Eine Probe kann auch Zusätze von bekannten Konzentrationen gleichartiger Verbindungen (als Standards) wie der nachzuweisenden Analyten, vergleichbar mit einem "Spiken" von Proben in der Chromatographie, enthalten. Solche Zusätze können beispielsweise für Kalibrationszwecke dienlich sein. Ausserdem können die Proben Zusätze von der Probenmatrix ähnlichen, aber von den nachzuweisenden Analyten verschiedenen Verbindungen, wie beispielsweise Rinderserumalbumin (BSA), enthalten, welche beispielsweise der kontrollierten Einstellung der Oberflächendichte immobilisierter Analytmoleküle in einem Messbereich dienen können. In den Proben oder deren Fraktionen oder den Verdünnungen besagter Proben oder Fraktionen enthaltene Analyten d.h. insbesondere Biopolymere wie beispielsweise Nukleinsäuren oder Proteine, können in nativer Form oder denaturierter Form, nach Behandlung beispielsweise mit Harnstoff oder Tensiden (z. B. SDS), vorliegen.

Bevorzugt liegen die in den Proben oder deren Fraktionen oder in den Verdünnungen besagter Proben oder Fraktionen enthaltenen Analyten d.h. insbesondere Biopolymere wie beispielsweise Nukleinsäuren oder Proteine, nach Behandlung mit Harnstoff, in denaturierter Form vor, wobei die Epitope dieser Analyten für die Bindung ihrer jeweiligen Nachweissubstanzen, beispielsweise von Antikörpern, frei zugänglich sind. Dieses wird ermöglicht durch die Zerstörung der tertiären oder quarternären Struktur infolge der Behandlung mit Harnstoff.

Überraschenderweise ist die Empfindlichkeit des erfindungsgemässen Verfahrens derart gross, dass eine Probe vor oder nach Fraktionierung sogar noch stark verdünnt werden kann, und in der Mischung enthaltene Verbindungen, trotz teilweiser nur sehr niedriger vorliegender Konzentration und entsprechend geringer verfügbarer Menge in einem einzelnen Messbereich, noch mit hoher Genauigkeit nachgewiesen werden können, was mit den bekannten herkömmlichen Methoden nicht möglich ist.

Als "Analyt" soll im Rahmen der vorliegenden Erfindung eine solche molekulare Spezies bezeichnet werden, welche mithilfe einer hierfür eingesetzten spezifischen Nachweissubstanz von anderen in einer zu analysierenden Probe enthaltenen Verbindungen unterschieden und gebunden wird. Erfolgt beispielsweise die Bindung einer entsprechenden Nachweissubstanz nur an die phosphorylierte, aber nicht an die unphosphorylierte Form einer nachzuweisenden Verbindung oder Spezies, so stellen gemäss dieser Definition beide Formen dieser Verbindung bzw. Spezies zwei unterschiedliche Analyten dar. Werden von einer entsprechenden Nachweissubstanz jegliche Verbindungen oder Spezies erkannt und gebunden, wenn sie phosphoryliert sind, so stellen entsprechend unter dieser Bedingung die entsprechenden phosphorylierten Verbindungen oder Spezies gemeinsam einen Analyten dar. Spezifische Bindungspartner als Nachweissubstanzen eines Analyten gemäss dieser Definition können beispielsweise so ausgewählt sein, dass sie ausschliesslich die phosphorylierte Form oder die glykolisierte Form (oder entsprechend die nicht phosphorylierte bzw. nicht glykolisierte Form) einer nachzuweisenden Verbindung erkennen und daran binden. Die Aktivität eines biologischen Signalwegs in einer Zelle oder einem Organismus kann mit dem Anteil phosphorylierter oder glykolisierter Verbindungen (abhängig von der Natur des Signalwegs), welche diesen Signalweg steuern, korreliert werden. Der relative Anteil der phosphorylierten bzw. glykolisierten Form an der Gesamtmenge, d.h. der Quotient der Menge einer Verbindung in ihrer phosphorylierten bzw. glykolisierten Form und der Gesamtmenge dieser Verbindung in phosphorylierter und nicht phosphorylierter Form bzw. glykolisierter und nicht glykolisierter Form, in einer Probe wird nachfolgend als Phosphorylierungssgrad bzw. Glykolisierungsgrad dieser Verbindung in der Probe bezeichnet. Phosphorylierungsgrad und Glykolisierungsgrad können unter dem Oberbegriff des Aktivierungsgrads einer Verbindung zusammengefasst werden, dessen numerischer Wert beispielsweise dem Phosphorylierungsgrad oder dem Glykolisierungsgrad entspricht. Der Aktivierungsgrad einer Verbindung kann aber auch andere chemisch veränderte Formen einer Verbindung bezeichnen.

Spezifische Bindungspartner als Nachweissubstanzen können auch so ausgewählt sein, dass sie nur dann an eine nachzuweisende Verbindung binden, wenn diese in einer bestimmten dreidimensionalen Struktur vorliegt. Beispielsweise erkennen und binden viele Antikörper nur an spezielle Teilbereiche (Epitope) einer nachzuweisenden Substanz mit einer speziellen dreidimensionalen Struktur. Je nach Konfonnationszustand der entsprechenden nachzuweisenden Verbindung können diese Teilbereiche (Epitope) für die Bindung der entsprechenden Nachweissubstanz zugänglich oder verborgen sein. Die spezifischen Bindungspartner können jedoch auch so ausgewählt sein, dass sie an Bereiche der nachzuweisenden Verbindung binden, deren Zugänglichkeit unabhängig von der dreidimensionalen Struktur dieser Verbindung ist. Durch den Einsatz entsprechend ausgewählter Nachweissubstanzen ist es daher möglich, den relativen Anteil an der Gesamtmenge einer in einer Probe nachzuweisenden Verbindung, welche einen spezifischen Konformationszustand aufweist, zu bestimmen.
Als "biologisch relevant" sollen solche Verbindungen bezeichnet werden, welche bekannt sind als Teilnehmer an spezifischen Bindungsreaktionen zu Molekülen oder Verbindungen biologischen Ursprungs oder zu deren synthetisch erzeugten Analogen. Beispiele für "biologisch relevante" Verbindungen sind daher nicht nur natürliche Proteine, wie Antikörper oder Rezeptoren, oder Nukleinsäuren, sondern auch deren Bindungspartner, wie beispielsweise Antigene, bei denen es sich auch um synthetische Verbindungen, auch sehr niedrigen Molekulargewichts, handeln kann.

Im Sinne der vorliegenden Erfindung sollen räumlich getrennte oder diskrete Messbereiche durch die geschlossene Fläche definiert werden, die dort immobilisierte Bindungspartner, zum Nachweis eines oder mehrerer Analyten in einer oder mehreren Proben in einem Bioaffinitätsassay, einnehmen. Diese Flächen können dabei eine beliebige Geometrie, beispielsweise die Form von Kreisen, Rechtecken, Dreiecken, Ellipsen etc., haben.

Unterschiedliche derartige Messbereiche können beispielsweise verschiedene Proben oder verschiedene Fraktionen einer einzigen aufgetrennten Probe umfassen, oder es kann sich um Fraktionen unterschiedlicher Proben oder auch um eine Vielzahl unterschiedlicher Verdünnungen von Fraktionen handeln. Dabei kann die Auftrennung in Fraktionen mit beliebigen bekannten Trennverfahren, wie beispielsweise Zentrifugation, Flüssig-Chromatographie (LC), HPLC, Dünnschich-Chromatographie, Gel-Chromatographie, Kapillarelektrophorese etc. oder durch Kombination dieser Trennverfahren erfolgt sein. Das Material für die diskreten Messbereiche kann auch durch selektive Mikropräparationen, wie beispielsweise das selektive Herauslösen einzelner Zellen aus einem Zellverband durch "Laser Capture Micro Dissection". bereitgestellt sein.

Allgemeiner kann die ursprüngliche Probe mit den darin nachzuweisenden Analyten ausgewählt sein aus der Gruppe von Extrakten gesunder oder krankhafter Zellen, (z. B. von menschlichen, tierischen, bakteriellen oder pflanzlichen Zellextrakten), Extrakten von tierischem oder menschlichem Gewebe, wie beispielsweise Organ-, Haut-, Haar- oder Knochengewebe, oder von Pflanzengewebe, sowie von Körperflüssigkeiten oder deren Bestandteilen, wie beispielsweise Blut, Serum oder Plasma, Gelenkflüssigkeiten, Tränenflüssigkeit, Urin, Speichel, Gewebeflüssigkeit, Lymphe. Insbesondere kann eine ursprüngliche Probe auch ausgewählt sein aus der Gruppe, welche Extrakte stimulierter oder unbehandelter Zellen und Extrakte gesunden oder krankhaften Gewebes umfasst.

Entsprechend kann, ausser durch "Laser Capture Micro Dissection", eine solche "Ursprungsprobe" auch einem Organismus oder Gewebe- oder Zellverband oder Zelle mittels einer Methode aus der Gruppe von Gewebeschnitten, Biopsie entnommen sein.

In einem Messbereich werden also im allgemeinen mehrere unterschiedliche Bindungspartner gleichzeitig immobilisiert sein. Meistens wird es sich um eine Vielzahl, d. h. mehrere hundert oder sogar mehrere tausend unterschiedliche Analyten in einem Messbereich immobilisierte Analyten handeln.

Erster Gegenstand der Erfindung ist ein Verfahren zur Untersuchung einer Vielzahl von Proben auf in den Proben enthaltene, als Teilnehmer an spezifischen Bindungsreaktionen biologisch relevante Verbindungen als Analyten, dadurch gekennzeichnet, dass
- Besagte Proben oder Fraktionen besagter Proben mit den darin enthaltenen, nachzuweisenden Analyten, als einer ersten Vielzahl von spezifischen Bindungspartnern direkt oder nach zusätzlichen Verdünnungen der Fraktionen in mindestens einem ein- oder zweidimensisonalen Array in diskreten Messbereichen auf einer Evaneszentfeld-Sensorplattform als festem Träger aufgetragen werden, wobei unterschiedliche Proben oder Fraktionen oder unterschiedliche Verdünnungen von Proben oder Fraktionen in unterschiedlichen diskreten Messbereichen angeordnet werden,
- eine oder mehrere Nachweissubstanzen, als eine zweite Vielzahl spezifischer Bindungspartner, zum spezifischen Nachweis von einem oder mehreren in den Proben oder deren Fraktionen enthaltenen Analyten, aus besagter ersten Vielzahl spezifischer Bindungspartner, in einem einzigen oder mehreren Schritten einer spezifischen Bindungsreaktion mit den in besagten diskreten Messbereichen aufgetragenen Proben oder Fraktionen oder deren Verdünnungen in Kontakt gebracht werden,
- Änderungen von optoelektronischen Signalen als Folge der Bindung von Nachweissubstanzen an in diskreten Messbereichen enthaltene Analyten, im evaneszenten Feld der Evaneszentfeld-Sensorplattform, ortsaufgelöst gemessen werden und
aus der relativen Grösse der Änderungen besagter optoelektronischer Signale aus den jeweiligen Messbereichen qualitativ und / oder quantitativ das Vorhandensein der dort spezifisch nachzuweisenden Analyten bestimmt wird.

Das Trennverfahren zur Auftrennung einer Probe in besagte Fraktionen kann ausgewählt sein aus der Gruppe von Verfahren, die von Zentrifugation, HPLC und micro-HPLC ("High Pressure Liquid Chromatography") mittels der Methode von "Normal Phase"-, "Reverse Phase"-, Ionenaustausch- oder Hydrophober Interaktionschromatographie (HIC), "Size Exclusion Chromatography", Gelchromatographie, Elektrophorese, Kapillarelektrophorese, Elektrochromatographie, "Free-Flow Elecktrophoresis", etc. gebildet wird.

Das erfindungsgemässe Verfahren zeichnet sich durch eine so grosse Empfindlichkeit aus, dass es möglich ist, dass eine Probe oder eine Fraktion einer Probe vor der Auftragung auf besagter Evaneszentfeld-Sensorplattform als festem Träger mindestens um einen Faktor 10 zu verdünnen. Es ist sogar möglich, eine Probe oder eine Fraktion einer zu untersuchenden Probe um einen Faktor 30 oder 100 zu verdünnen und trotzdem eine Vielzahl von Analyten in einem Messbereich, der durch die Aufbringung einer solchen stark verdünnten Probe oder deren Fraktion erzeugt wird, quantitativ nachzuweisen.

Nachfolgend sollen die in diskreten Messbereichen aufzubringenden Proben oder deren Fraktionen und die aufzubringenden Verdünnungen von von Proben oder Probenfraktionen unter dem Begriff "Immobilisierungsproben" zusammengefasst werden.

Die zu untersuchenden Proben mit den darin, gegebenenfalls nach einer Fraktionierung, nachzuweisenden Analyten können ausgewählt sein aus der Gruppe von Extrakten gesunder oder krankhafter Zellen, (z. B. von menschlichen, tierischen, bakteriellen oder pflanzlichen Zellextrakten), Extrakten von tierischem oder menschlichem Gewebe, wie beispielsweise Organ-, Haut-, Haar- oder Knochengewebe, oder von Pflanzengewebe, sowie von Körperflüssigkeiten oder deren Bestandteilen, wie beispielsweise Blut, Serum oder Plasma, Gelenkflüssigkeiten, Tränenflüssigkeit, Urin, Speichel, Gewebeflüssigkeit, Lymphe.

Um eine optimale Zugänglichkeit der in den Messbereichen immobilisierten Analyten als erster Vielzahl spezifischer Bindungspartner Bindungspartnern für die damit in Kontakt zu bringenden Nachweissubstanzen zu gewährleisten, ist es von Vorteil, wenn das Material einer in einem Messbereich aufzubringenden "Immobilisierungsprobe" gleich gross oder weniger ist, als zur Ausbildung einer Monoschicht auf der Evaneszentfeld-Sensorplattform als festem Träger erforderlich ist. Die Zugänglichkeit kann noch dadurch verbessert werden, dass eine zuvor aufgebrachte Haftvermittlungsschicht (wie sie weiter unten beschrieben wird) zu einer orientierten Immobilisierung führt, beispielsweise indem in der aufgebrachten Probe enthaltene Antikörper an ihrem F_{C}-Teil gebunden immobilisiert werden, so dass ihre spezifischen Bindungsepitope zugänglich sind.

Aufgrund der hohen Empfindlichkeit des erfindungsgemässen Verfahrens ist es möglich, auch nur geringe die eingesetzte Probevolumina und -mengen mit hoher Geanauigkeit zu analysieren. Als Probenmenge soll dabei die Gesamtmenge verstanden werden, welche in einem diskreten Messbereich aufgebracht ist. Beispielsweise kann eine "Immobilisierungsprobe" das Material von weniger als 20000 Zellen umfassen. Eine aufzubringende "Immobilisierungsprobe" kann sogar das Material von weniger als 1000 Zellen umfassen. Die erforderliche Probenmenge kann sogar das Material von weniger als 100 Zellen, oder sogar von nur 1 - 10 Zellen umfassen und noch verlässlich analysiert werden. Das Material.welches dem Inhalt einer Zelle entspricht, soll auch als ein Zell-Äquivalent bezeichnet werden. Die Notwendigkeit einer nur so kleinen Menge für eine Analyse benötigter Zell-Äquivalente ist dann gegeben, wenn es sich bei den nachzuweisenden Analyten um in relativ hoher Konzentration auftretende Inhaltsstoffe handelt. Ausserdem ist es möglich, dass eine "Immobilisierungsprobe" ein Volumen von weniger als 1 µl hat. Eine aufzubringende "Immobilisierungsprobe" kann sogar ein Volumen von weniger als 10 nl oder sogar weniger als 1 nl haben.

Das erfindungsgemässe Verfahren ermöglicht, dass die in einer "Immobilisierungsprobe"enthaltenen relativen Gesamtmengen von einer oder mehreren Verbindungen als Analyten, als Summe von deren Vorkommen in phosphorylierter oder nicht phosphorylierter Form und / oder glykolisierter und / oder nicht glykolisierter Form, bestimmt werden. Vorzugsweise werden die in einer "Immobilisierungsprobe"enthaltenen relativen Mengen von einer oder mehreren Verbindungen als Analyten, jeweils von deren Vorkommen in phosphorylierter und / oder nicht phosphorylierter Form und / oder glykolisierter und / oder nicht glykolisierter Form, für eine oder mehrere besagter Formen bestimmt.

Das erfindungsgemässe Verfahren ermöglicht es, gemäss obiger Definition den Aktivierungsgrad eines oder mehrerer in einer "Immobilisierungsprobe" enthaltenen Analyten zu bestimmen. Insbesonder kann mit besagtem Verfahren der Phosphorylierunsgrad und / oder Glykolisierungsgrad eines oder mehrerer in einer "Immobilisierungsprobe" enthaltenen Analyten bestimmt werden. Charakteristisch fügt das erfindungsgemässe Vefahren ist ausserdem aufgrund seiner hohen Empfindlichkeit und hohen Genauigkeit und Reproduzierberkait, insbesondere aufgrund einer Vielzahl gleichzeitig oder alternativ einsetzbarer, voneinander unabhängiger Referenzierungs- und Kalibrationsmethoden, dass Unterschiede von weniger als 20 %, bevorzugt von weniger als 10 %, von den in einer "Immobilisierungsprobe" und in einer oder mehreren Vergleichsproben enthaltenen relativen Mengen von einer oder mehreren Verbindungen in phosphorylierter und / oder nicht phosphorylierter Form und / oder glykolisierter und / oder nicht glykolisierter Form als Analyten, für eine oder mehrere besagter Formen nachgewiesen werden.

Ein grosser Vorteil des erfindungsgemässen Verfahrens aufgrund seiner inhärenten, methodenspezifischen hohen Empfindlichkeit und vielfältigen Möglichkeiten der Referenzierung und / oder Kalibration unter Benutzung ein- und derselben analytischen Plattform (bzw. Evaneszentfeld-Sensorplattform) ist, dass die Variation der damit gewonnnen Messergebnisse sehr niedrig ist. Das erfindungsgemässe Verfahren ist daher auch geeignet zur Untersuchung des zeitlichen Verlaufs (d. h. der Veränderungen) der relativen Mengen oder Konzentrationen von biologisch relevanten Substanzen unter Einfluss der Erkrankung eines biologischen Organismus oder einer Zellkultur und / oder der äusseren Beeinflussung eines Organismus oder einer Zellkultur.

Eine weitere Ausführungsform des erfindungsgemässen Verfahrens ist daher dadurch gekennzeichnet, dass besagte "naturidentische" Probe und eine oder mehrere Vergleichsproben dem gleichen Ursprungsort zu unterschiedlichen Zeitpunkten entnommen sind und dass zeitliche Veränderungen der in diesen Proben enthaltenen relativen Mengen von einer oder mehreren Verbindungen in phosphorylierter und / oder nicht phosphorylierter Form und / oder glykolisierter und / oder nicht glykolisierter Form als Analyten, bestimmt werden. Unter "dem gleichen Ursprungsort" soll dabei der gleiche Organismus oder ein gleichartiger Organismus oder die gleiche Zellkultur bzw. gleichartige Zellkultur (jeweils nach unterschiedlich langer gleichartiger Erkrankung oder Beeinflussung) verstanden werden. Vorzugsweise ermöglicht das erfindungsgemässe Verfahren, zeitliche Änderungen der relativen Konzentration bzw. Menge besagter Analyten von weniger als 20 % , bevorzugt von weniger als 10 %, nachzuweisen.

Unterschiedliche Proben können dem gleichen Organismus oder der gleichen Zellkultur entnommen werden. Dann kann durch die Analyse auf mehreren Messbereichen mit darin enthaltenem Material aus dem gleichen (oder einem gleichartigen) Organismus bzw, der gleichen Zellkultur (bzw. gleichartigen Zellkulturen) beispielsweise statistische Information über die Reproduzierbarkeit der in diesen Messbereichen bestimmten relativen molekularen Zusammensetzung der aufgebrachten Proben gewonnen werden.

Unterschiedliche Proben können insbesondere an verschiedenen Positionen des gleichen Organismus entnommen werden. Dann kann aus den Analysen auf den entsprechenden diskreten Messbereichen beispielsweise Information über Inhomogenitäten der relativen molekularen Zusammensetzung der nachzuweisenden Analyten in dem Organismus, aus dem besagte Proben entnommen wurden, gewonnen werden. Ein solches Vorgehen ist beispielsweise für die Untersuchung krebsbehafteter Organismen von grosser Bedeutung.

Unterschiedliche Proben können aber auch verschiedenen Organismen oder verschiedenen Zellkulturen entnommen sein. Beispielsweise kann es sich dabei um Proben von mit einem pharmazeutischen Wirkstoff behandelten und unbehandelten Organismen handeln. Ähnlich einer Expressionsanalyse in der Nukleinsäure-Analytik kann dann der Einfluss des jeweiligen Wirkstoffes auf die relative molekulare Zusammensetzung der Proben untersucht werden.

Die einfachste Form der Immobilisierung von spezifischen Bindungspartnern für einen Analytnachweis in einer spezifischen Bindungsreaktion besteht in physikalischer Adsorption, beispielsweise infolge hydrophober Wechselwirkungen zwischen den zu immobilisierenden spezifischen Bindungspartnern und der Evaneszentfeld-Sensorplattform als festem Träger. Diese Wechselwirkungen können jedoch durch die Zusammensetzung des Mediums und dessen physikalisch-chemische Eigenschaften, wie beispielsweise Polarität und Ionenstärke, in ihrem Ausmass stark verändert werden. Insbesondere im Falle sequentieller Zugabe verschiedener Reagentien in einem mehrstufigen Assay ist das Haftvermögen der Erkennungselemente nach rein adsorptiver Immobilisierung auf der Oberfläche oft unzureichend. dass die Evaneszentfeld-Sensorplattform, zur Verbesserung des Haftvermögens der in diskreten Messbereichen aufzubringenden "Immobilisierungsproben", eine Haftvermittlungsschicht umfasst, auf welcher besagte Fraktionen von Proben oder Verdünnungen dieser Fraktionen aufgetragen werden.

Vorzugsweise beträgt dabei die Dicke der Haftvermittlungsschicht weniger als 200 nm, besonders bevorzugt weniger als 20 nm.

Für die Herstellung der Haftvermittlungsschicht eignen sich eine Vielzahl von Materialien. Beispielsweise kann die Haftvermittlungsschiht Verbindungen umfassen aus der Gruppe von Silanen, funktionalisierten Silanen, Epoxiden, funktionalisierten, geladenen oder polaren Polymeren und "selbstorganisierten passiven oder funktionalisierten Mono- oder Mehrfachschichten", Thiolen, Alkylphosphaten und - phosphonaten, multifunktionellen Block-Copolymeren, wie beispielsweise Poly(L)lysin/Polyethylenglycolen.

Es ist auch möglich, dass besagte Haftvermittlungsschicht Verbindungen umfasst aus der Gruppe von Organophosphorsäuren der allgemeinen Formel I (A)

**Y-B-OPO₃ H₂** **(IA)**

oder von Organophosphonsäuren der allgemeinen Formel I (B)

**Y-B-PO₃ H₂** **(IB)**

und deren Salzen, in denen B einen Alkyl-, Alkenyl-, Alkinyl-, Aryl-, Aralkyl-, Hetaryl- oder Hetarylalkylrest und Y Wasserstoff oder eine funktionelle Gruppe aus der Reihe Hydroxy, Carboxy, Amino, gegebenfalls durch Niederalkyl substituiertes Mono-oder Dialkylamino, Thiol, oder eine negative Säuregruppe aus der Reihe Ester, Phosphat, Phosphonat, Sulfat, Sulfonat, Maleimid, Succinimydyl, Epoxy oder Acrylat bedeutet Diese Verbindungen sind genauer beschrieben in der internationalen Anmeldung PCT/EP 01/10077, welche hiermit vollumfänglich als Bestandteil dieser Anmeldung eingeführt wird.

Eine besondere Ausführungsform des erfindungsgemässen Verfahrens ist dadurch gekennzeichnet, dass eine oder mehrere "Immobilisierungsproben"vor ihrer Auftragung auf der Evaneszentfeld-Sensorplattform als festem Träger (zur Verbesserung des Haftvermögens auf besagtem festem Träger und Erhöhung der Gleichmässigkeit der Auftragung) mit einer Lösung von Polymeren oder polymerisierbaren Monomeren, gegebenenfalls in Anwesenheit von Initiatoren, oder von chemischen "Cross-Linkern" (z. B. Glutaraldehyd) gemischt werden. Diese Variante des Verfahrens kann beispielsweise dazu beitragen, die Ausbildung von Inhomogenitäten der Verteilung des Probenmaterials innerhalb eines Messbereichs beim Prozess der Verdampfung der Probenflüssigkeit zu vermeiden, was zu einer besseren "Spot-Morphologie" führt und damit die Auswertung der Ergebnisse erleichtert. Dabei wird bevorzugt, dass besagte Lösung von Polymeren oder polymerisierbaren Monomeren oder chemischen "Cross-Linkern" ausgewählt ist aus der Gruppe, welche Lösungen von Polysaccharidea, wie z. B. Agarose, oder von Acrylamiden oder von Glutaralehyd etc.umfasst.

Diese besondere Variante des erfindungsgemässen Verfahrens ist auch dadurch gekennzeichnet, dass die Mischung der einen oder mehreren Proben mit einer Lösung von Polymeren oder polymerisierbaren Monomeren, gegebenenfalls in Anwesenheit von Initiatoren, oder von chemischen "Cross-Linkern" (z. B. Glutaraldehyd) in der Immobilisierung einer dreidimensionalen Netzwerkstruktur mit darin eingebundenen, für Nachweissubstanzen in dem nachfolgenden Schritt einer Bioaffinitätsreaktion zugänglichen Probenbestandteilen, auf der Evaneszentfeld-Sensorplattform als festem Träger resultiert. Damit kann ein höherer Grad der Oberflächenbedeckung der Evaneszentfeld-Sensorplattform als eine Monoschicht erreicht werden, was zu einer weiteren Erhöhung der messbaren Signale beim Schritt des Analytnachweises führen kann. Wichtig ist dabei, dass die geschaffene Polymeren-Netzwerkstruktur sich nicht über die Eindringtiefe des evaneszenten Feldes ins Medium hinaus erstreckt, da ein Analytnachweis jenseits dieser Entfernung von der Oberfläche der Evaneszentfeld-Sensorplattform nicht möglich ist.

Die "Immobilisierungsproben" können in diskreten Messbereichen auf der Evaneszentfeld-Sensorplattform direkt oder auf einer darauf aufgebrachten Haftvermittlungsschicht räumlich selektiv mithilfe eines Verfahrens aufgebracht werden, welches ausgewählt ist aus der Gruppe von Verfahren, die von "Ink j et spotting", mechanischem Spotting mittels Stift, Feder oder Kapillare, "Micro contact printing", fluidischer Kontaktierung der Messbereiche mit besagten Proben durch deren Zufuhr in parallelen oder gekreuzten Mikrokanälen, unter Einwirkung von Druckunterschieden oder elektrischen oder elektromagnetischen Potentialen sowie photochemischen oder photolithographischen Immobilisierungsverfahren gebildet wird.

Es ist von Vorteil, wenn Bereiche zwischen den diskreten Messbereichen zur Minimierung unspezifischer Bindung von Nachweissubstanzen "passiviert werden", d.h. dass zwischen den räumlich getrennten Messbereichen gegenüber den Analyten und anderen Inhaltsstoffen der aufgebrachten Proben sowie gegenüber den Nachweissubstanzen für besagte Analyten "chemisch neutrale", d.h. diese nicht bindende, Komponenten aufgebracht sind.

Besagte gegenüber den Analyten und anderen Inhaltsstoffen der aufgebrachten "Immobilisierungsproben" sowie gegenüber den Nachweissubstanzen für besagte Analyten "chemisch neutrale", d.h. diese nicht bindende, Komponenten können ausgewählt sein aus den Gruppen, die von Albuminen, insbesondere Rinderserumalbumin oder Humanserumalbumin, Casein, unspezifischen, polyklonalen oder monoklonalen, artfremden oder empirisch für den oder die nachzuweisenden Analyten unspezifischen Antikörpern (insbesondere für Immunoassays), Detergentien - wie beispielsweise Tween 20 -, nicht mit zu analysierenden Polynukleotiden hybridisierender, fragmentierter natürlicher oder synthetischer DNA, wie beispielsweise ein Extrakt von Herings- oder Lachssperma (insbesondere für Polynukleotid-Hybridisierungsassays), oder auch ungeladenen, aber hydrophilen Polymeren, wie beispielsweise Polyethylenglycolen oder Dextranen, gebildet werden.

Ohne Beschränkung der Allgemeinheit kann es sich bei den in diskreten Messbereichen aufgetragenen "Immobilisierungsproben"enthaltenen, nachzuweisenden Analyten beispielsweise um Verbindungen aus der Gruppe handeln, die von Proteinen, beispielsweise mono- oder polyklonalen Antikörpern und Antikörperfragmenten, Peptiden, Enzymen, Glycopeptiden, Oligosacchariden, Lektinen, Antigenen für Antikörper, mit zusätzlichen Bindungsstellen funktionalisierten Proteinen ("Tag-Proteinen", wie beispielsweise "Histidin-Tag-Proteinen") sowie Nukleinsäuren (beispielsweise DNA, RNA) gebildet wird.. Bei den in diskreten Messbereichen aufgetragenen Proben enthaltenen, nachzuweisenden Analyten kann es sich auch um Verbindungen aus der Gruppe handeln, welche cytosolische oder membrangebundene Zellproteine, insbesondere an den Prozessen der Signaltransduktion in Zellen beteiligte Proteine, wie z. B. Kinasen, umfasst. Die Analyten können auch biotechnisch modifizierte, beispielsweise mit lumineszenten bzw. fluoreszenten Gruppen und biologisch expremierte Biopolymere, wie beispielsweise mit "blue fluorescent proteins" (BFP), "green fluorescent proteins" (GFP) oder "red fluorescent proteins" (RFP) sein.

Abhängig von der physikalischen Ausgestaltung der Evaneszentfeld-Sensorplattform gibt es für die messtechnische Art der Signalerzeugung beim Analytnachweis verschiedene Möglichkeiten. Eine mögliche Variante ist dadurch gekennzeichnet, dass die ortaufgelöst zu messenden Änderungen optoelektronischer Signale, als Folge der Bindung von Nachweissubstanzen an in diskreten Messbereichen in den "Immobilisierungsproben" enthaltene Analyten, durch lokale Änderungen der Resonanzbedingungen zur Erzeugung eines Oberflächenplasmons in einer dünnen Metallschicht als Teil besagter Evaneszentfeld-Sensorplattform hervorgerufen werden.

Messtechnisch sind für die Bestimmung von Änderungen der Resonanzbedingungen der Resonanzwinkel (bei Variation des Einstrahlungswinkels bei konstanter Wellenlänge des eingestrahlten Lichts) und die Resonanzwellenlänge (bei konstantem Einstrahlungswinkel und Variation der eingestrahlten Anregungswellenlänge) zugänglich. Entsprechend kann dass besagte Änderung der Resonanzbedingungen in einer Änderung des Resonanzwinkels für die Einstrahlung eines Anregungslichts zur Erzeugung eines Oberflächenplasmons in einer dünnen Metallschicht als Teil besagter Evaneszentfeld-Sensorplattform bestehen. Entsprechend kann auch besagte Änderung der Resonanzbedingungen in einer Änderung der Resonanzwellenlänge eines eingestrahlten Anregungslichts zur Erzeugung eines Oberflächenplasmons in einer dünnen Metallschicht als Teil besagter Evaneszentfeld-Sensorplattform bestehen.

Die ortaufgelöst zu messenden Änderungen optoelektronischer Signale, als Folge der Bindung von Nachweissubstanzen an in diskreten Messbereichen in den "Immobilisierungsproben" enthaltene Analyten, können durch lokale Änderungen des effektiven Brechungsindex in diesen Bereichen auf besagter Evaneszentfeld-Sensorplattform hervorgerufen werden.

Eine andere wichtige Ausführungsform des erfindungsgemässen Verfahrens ist dadurch gekennzeichnet, dass die ortaufgelöst zu messenden Änderungen optoelektronischer Signale, als Folge der Bindung von Nachweissubstanzen an in diskreten Messbereichen in den "Immobilisierungsproben" enthaltene Analyten, durch lokale Änderungen einer oder mehrerer Lumineszenzen von innerhalb des evaneszenten Feldes besagter Evaneszentfeld-Sensorplattform befindlichen lumineszenzfähigen Molekülen hervorgerufen werden.

Es wird bevorzugt, dass besagte Änderungen einer oder mehrerer Lumineszenzen von lumineszenzfähigen Molekülen oder lumineszenzfähigen Nanopartikeln stammen, welche als Lumineszenzlabel an eine oder mehrere Nachweissubstanzen für die in diskreten Messbereichen enthaltenen Analyten gebunden sind.

Von besonderem Vorteil ist, wenn zum Analytnachweis zwei oder mehr Lumineszenzlabel mit unterschiedlichen Emissionswellenlängen und / oder unterschiedlichen Anregungsspektren, bevorzugt mit unterschiedlichen Emissionswellenlängen und gleicher Anregungswellenlänge, eingesetzt werden. Wenn mehrere Lumineszenzlabel mit unterschiedlichen spektralen Eigenschaften, insbesondere unterschiedlichen Emissionswellenlängen an unterschiedliche Nachweissubstanzen aus der zweiten Vielzahl spezifischer Bindungspartner, welche mit den Messbereichen in Kontakz gebracht wird, gebunden sind, können beispielsweise unterschiedliche Analyten in einem einzigen Nachweisschritt, d.h. Kontaktierung der Messbereiche mit besagten Nachweissubstanzen und gleichzeitige oder nachfolgende Detektion der erzeugten Lumineszenzen, bestimmt werden.

Beispielsweise ist eine solche Variante des erfindungsgemässen Verfahrens besonders gut dafür geeignet, gleichzeitig zum Beispiel die phoshorylierte und die nicht phosphorylierte Form einer Verbindung, insbesondere auch innerhalb eines einzelnen (gemeinsamen) Messbereiches nachzuweisen, mithilfe zweier entsprechender unterschiedlicher, in diesem Fall direkt (z. B. mit grün bzw. rot emittierenden Lumineszenzlabeln) markierter spezifischer Bindungspartner als Nachweissubstanzen.

In ähnlicher Weise können gleichzeitig zwei oder mehr verschiedene Analyten detektiert werden, wenn zum Analytnachweis zwei oder mehr Lumineszenzlabel mit unterschiedlichen Emissionsabklingzeiten eingesetzt werden.

Für das erfindungsgemässe Verfahren wird also bevorzugt, dass zum Nachweis unterschiedlicher Analyten in einer "Immobilisierungsprobe" zwei oder mehr Lumineszenzlabel verwendet werden. Ebenso wird bevorzugt, dass zum Nachweis unterschiedlicher Analyten in einem Messbereich zwei oder mehr Lumineszenzlabel verwendet werden.

Von Vorteil ist ausserdem, wenn die Einstrahlung des Anregungslichts in Pulsen mit einer Dauer zwischen 1 fsec und 10 Minuten erfolgt und das Emissionslicht aus den Messbereichen zeitlich aufgelöst gemessen wird.

Vorzugsweise umfasst die Evaneszentfeld-Sensorplattform als fester Träger einen optischen Wellenleiter, umfassend eine oder mehrere Schichten. Es kann sich dabei beispielsweise um einen faseroptischen Wellenleiter, bestehend aus mehreren Schichten handeln. Bevorzugt handelt es sich jedoch úm einen planaren optischen Wellenleiter, welcher durchgehend über eine Oberfläche der Evaneszentfeld-Sensorplattform ausgebildet ist oder auch in diskrete wellenleitende Bereiche aufgeteilt sein kann, wie dieses beispielsweise in der Patentanmeldung WO 96/35940 beschrieben ist, .

Besonders bevorzugt wird eine solche Ausführungsform des erfindungsgemässen Verfahrens, welche dadurch gekennzeichnet ist, dass die Evaneszentfeld-Sensorplattform als fester Träger einen planaren optischen Dünnschichtwellenleiter mit einer im wesentlichen optisch transparenten, wellenleitenden Schicht auf einer zweiten, ebenfalls im wesentlichen optisch transparenten Schicht mit niedrigerem Brechungsindex als die wellenleitende Schicht und gegebenenfalls einer ebenfalls im wesentlichen optisch transparenten Zwischenschicht zwischen der wellenleitender Schicht und der zweiten Schicht mit ebenfalls niedrigerem Brechungsindex als die wellenleitende Schicht umfasst.

Das Anregungslicht von einer oder mehreren Lichtquellen kann in eine wellenleitende Schicht der Evaneszentfeld-Sensorplattform über ein oder mehrere optische Koppelemente eingekoppelt werden, welche ausgewählt sind aus der Gruppe, die von Prismenkopplern, evaneszenten Kopplern mit zusammengebrachten optischen Wellenleitern mit überlappenden evaneszenten Feldern, Stirnflächenkopplern mit vor einer Stirnseite der wellenleitenden Schicht angeordneten fokussierenden Linsen, vorzugsweise Zylinderlinsen, und Gitterkopplern gebildet wird.

Es wird bevorzugt, dass die Einkopplung von Anregungslicht in eine wellenleitende Schicht der Evaneszentfeldsensorplattform mithilfe von einer oder mehreren Gitterstrukturen erfolgt, die in der besagten wellenleitenden Schicht ausgeprägt sind.

Ausserdem wird bevorzugt, dass die Auskopplung von in einer wellenleitenden Schicht der Evaneszentfeld-Sensorplattform geführtem Licht mithilfe von einer oder mehreren Gitterstrukturen zur Auskopplung erfolgt, die in besagter wellenleitender Schicht ausgeprägt sind und gleiche oder unterschiedliche Periode und Gittertiefe wie die Gitterstrukturen zur Einkopplung haben.

Eine besonders bevorzugte Ausführungsform des erfindungsgemässen Verfahrens ist dadurch gekennzeichnet, dass Anregungslicht von einer oder mehreren Lichtquellen über eine Gitterstruktur zur Einkopplung in eine wellenleitende Schicht besagter Evaneszentfeld-Sensorplattform eingekoppelt und zu auf der Evaneszentfeld-Sensorplattform befindlichen Messbereichen als geführte Welle geleitet wird, dass weiterhin die im evaneszenten Feld besagter geführter Welle erzeugte Lumineszenz von lumineszenzfähigen Molekülen mit einem oder mehreren Detektoren ortsaufgelöst erfasst und die relative Konzentration eines oder mehrerer Analyten aus der relativen Intensität dieser Lumineszenzsignale bestimmt wird.

Eine besondere Variante besteht dabei darin, dass neben der Bestimmung einer oder mehrerer Lumineszenzen Änderungen des effektiven Brechungsindex auf den Messbereichen bestimmt werden.

Für eine weitere Verbesserung der Empfindlichkeit kann dabei vorteilhaft sein, wenn die einen oder mehreren Lumineszenzen und / oder Bestimmungen von Lichtsignalen bei der Anregungswellenlänge polarisationsselektiv vorgenommen werden. Dabei wird bevorzugt, dass die einen oder mehreren Lumineszenzen bei einer anderen Polarisation als der des Anregungslichts gemessen werden.

Ein weiterer Gegenstand der vorliegenden Erfindung ist eine analytische Plattform zur Untersuchung einer Vielzahl von Proben auf in den Proben enthaltene, als Teilnehmer an Bioaffinitätsreaktionen biologisch relevante Verbindungen als Analyten, umfassend
- eine Evaneszentfeld-Sensorplattform als festen Träger
- mindestens ein ein- oder zweidimensionales Array von diskreten Messbereichen mit darin immobilisierten Bindungspartnern auf besagter Evaneszentfeld-Sensorplattform für den Nachweis besagter Analyten in einer Bioaffinitätsreaktion,
dadurch gekennzeichnet,
dass besagte diskrete Messbereiche durch die Auftragung von besagten Proben oder Fraktionen besagter Proben direkt oder nach zusätzlichen Verdünnungen dieser Proben oder von deren Fraktionen, mit den darin nachzuweisenden Analyten, als einer ersten Vielzahl von spezifischen Bindungspartnern, erzeugt wurden,
dass unterschiedliche Proben oder Fraktionen oder unterschiedliche Verdünnungen der Proben oder von deren Fraktionen in unterschiedlichen diskreten Messbereichen angeordnet sind und
dass es sich bei dem einen oder den mehreren immobilisierten Bindungspartnern, welche besagte erste Vielzahl von spezifischen Bindungspartnern bilden, um den einen oder um die mehreren Analyten selbst handelt, welche in den zu untersuchenden Proben enthalten sind.

Dabei kann eine in besagte Fraktionen aufgetrennte, zu untersuchende Probe durch ein Trennverfahren fraktioniert worden sein, welches ausgewählt sein kann aus der Gruppe von Verfahren, die von Zentrifugation, HPLC und micro-HPLC ("High Pressure Liquid Chromatography") mittels der Methode von "Normal Phase"-, "Reverse Phase"-, Ionenaustausch- oder Hydrophober Interaktionschromatographie (HIC), "Size Exclusion Chromatography", Gelchromatographie, Elektrophorese, Kapillarelektrophorese, Elektrochromatographie, "Free-Flow Electrophoresis", etc. gebildet wird.

Eine oder mehrere besagter Proben können biologischen Organismen oder Gewebe-oder Zellverbänden oder Zellen entnommen und direkt (d.h. nach Lyse der Zellen) ohne weitere Verdünnung, auf besagtem festen Träger aufgetragen worden sein.

Die erfindungsgemässe analytische Plattform zeichnet sich durch eine so grosse Empfindlichkeit aus, dass es möglich ist, eine Probe oder eine Fraktion einer Probe vor der Auftragung auf besagter Evaneszentfeld-Sensorplattform als festem Träger mindestens um einen Faktor 10 zu verdünnen. Es ist sogar möglich, eine Probe oder eine Fraktion einer zu untersuchenden Probe um sogar einen Faktor 30 oder 100 zu verdünnen und trotzdem eine Vielzahl von Analyten in einem Messbereich, der durch die Aufbringung einer solchen stark verdünnten Fraktion erzeugt wird, quantitativ nachzuweisen.

Nachfolgend sollen wiederum die in diskreten Messbereichen aufzubringenden Fraktionen von Proben und die aufzubringenden Verdünnungen von Probenfraktionen unter dem Begriff "Immobilisierungsproben" zusammengefasst werden.

Die zu untersuchenden Proben mit den darin gegebenenfalls nach einer Fraktionierung nachzuweisenden Analyten können ausgewählt sein aus der Gruppe von Extrakten gesunder oder krankhafter Zellen, (z. B. von menschlichen, tierischen, bakteriellen oder pflanzlichen Zellextrakten), Extrakten von tierischem oder menschlichem Gewebe, wie beispielsweise Organ-, Haut-, Haar- oder Knochengewebe, oder von Pflanzengewebe, sowie von Körperflüssigkeiten oder deren Bestandteilen, wie beispielsweise Blut, Serum oder Plasma, Gelenkflüssigkeiten, Tränenflüssigkeit, Urin, Speichel, Gewebeflüssigkeit, Lymphe.

Insbesondere kann eine zu untersuchende Probe ("Immobilisierungsprobe") Probe auch ausgewählt sein aus der Gruppe, welche Extrakte stimulierter oder unbehandelter Zellen und Extrakte gesunden oder krankhaften Gewebes umfasst.

In den Proben oder deren Fraktionen bzw. deren Verdünnungen enthaltene Analyten, d.h. insbesondere Biopolymere, wie beispielsweise Nukleinsäuren oder Proteine, können in nativer Form oder denaturierter Form, nach Behandlung der "Ursprungsprobe" beispielsweise mit Harnstoff oder Tensiden (z. B. SDS) vorliegen.

Bevorzugt liegen die in den "Immobilisierungsproben" enthaltenen Analyten, d.h. insbesondere Biopolymere wie beispielsweise Nukleinsäuren oder Proteine, nach Behandlung mit Harnstoff, in denaturierter Form vor, wobei die Epitope der besagten Analyten für die Bindung ihrer jeweiligen Nachweissubstanzen, beispielsweise von Antikörpern, frei zugänglich sind. Dieses wird ermöglicht durch die Zerstörung der tertiären oder quartemären Struktur infolge der Behandlung mit Harnstoff.

Entsprechend kann, ausser durch "Laser Micro Dissection", eine Probe auch einem Organismus oder Gewebe- oder Zellverband oder Zelle mittels einer Methode aus der Gruppe von Gewebeschnitten, oder Biopsie entnommen sein.

Eine aufgebrachte Probe kann das Material von weniger als 20000 Zellen, ja sogar von weniger als 1000 Zellen umfassen. Die Probe kann ein Volumen von weniger als 1 µl, ja sogar von weniger als 10 nl haben.

Die erforderliche Probenmenge kann sogar das Material von weniger als 100 Zellen umfassen und noch verlässlich analysiert werden. Dieses ist dann der Fall, wenn es sich bei den nachzuweisenden Analyten um in relativ hoher Konzentration auftretende Inhaltsstoffe handelt.

Unterschiedliche aufgetragene Proben können dem gleichen Organismus entnommen sein. Dabei können die Proben an verschiedenen Positionen des gleichen Organismus entnommen sein. Unterschiedliche aufgetragene Proben können auch der gleichen oder einer gleichartigen Zellkultur entnommen sein.

Unterschiedliche aufgetragene Proben können aber auch verschiedenen Organismen oder verschiedenen Zellkulturen entnommen sein.

Es wird bevorzugt, dass die Evaneszentfeld-Sensorplattform, zur Verbesserung des Haftvermögens der in diskreten Messbereichen aufgetragenen "Immobilisierungsproben", eine Haftvermittlungsschicht umfasst, auf welcher besagte Proben oder deren Fraktionen oder Verdünnungen aufgetragen werden.

Vorzugsweise beträgt dabei die Dicke der Haftvermittlungsschicht weniger als 200 nm, besonders bevorzugt weniger als 20 nm.

Die Haftvermittlungsschicht kann Verbindungen umfassen aus der Gruppe von Silanen, funktionalisierten Silanen, Epoxiden, funktionalisierten, geladenen oder polaren Polymeren und "selbstorganisierten passiven oder funktionalisierten Mono-oder Mehrfachschichten", Thiolen, Alkylphosphaten und -phosphonaten, multifunktionellen Block-Copolymeren, wie beispielsweise Poly(L)lysin/Polyethylenglycolen.

Als besonders vorteilhaft hat sich erwiesen, wenn besagte Haftvermittlungsschicht Verbindungen umfasst aus der Gruppe von Organophosphorsäuren der allgemeinen Formel I (A)

**Y-B-OPO₃ H₂** **(IA)**

oder von Organophosphonsäuren der allgemeinen Formel I (B)

**Y-B-PO₃ H₂** **(IB)**

und deren Salzen, in denen B einen Alkyl-, Alkenyl-, Alkinyl-, Aryl-, Aralkyl-, Hetaryl- oder Hetarylalkylrest und Y Wasserstoff oder eine funktionelle Gruppe aus der Reihe Hydroxy, Carboxy, Amino, gegebenfalls durch Niederalkyl substituiertes Mono-oder Dialkylamino, Thiol, oder eine negative Säuregruppe aus der Reihe Ester, Phosphat, Phosphonat, Sulfat, Sulfonat, Maleimid, Succinimydyl, Epoxy oder Acrylat bedeutet.

Eine besondere Ausführungsfonn der erfindungsgemässen analytischen Plattform ist dadurch gekennzeichnet, dass eine oder mehrere "Immobilisierungsproben"vor ihrer Auftragung auf der Evaneszentfeld-Sensorplattform als festem Träger (zur Verbesserung des Haftvermögens auf besagtem festem Träger und Erhöhung der Gleichmässigkeit der Auftragung) mit einer Lösung von Polymeren oder polymerisierbaren Monomeren, gegebenenfalls in Anwesenheit von Initiatoren, oder von chemischen "Cross-Linkern" (z. B. Glutaraldehyd) gemischt werden. Diese Variante des Verfahrens kann beispielsweise dazu beitragen, die Ausbildung von Inhomogenitäten der Verteilung des Probenmaterials innerhalb eines Messbereichs beim Prozess der Verdampfung der Probenflüssigkeit zu vermeiden, was zu einer besseren "Spot-Morphologie" führt und damit die Auswertung der Ergebnisse erleichtert. Dabei wird bevorzugt, dass besagte Lösung von Polymeren oder polymerisierbaren Monomeren oder chemischen "Cross-Linkern" ausgewählt ist aus der Gruppe, welche Lösungen von Polysacchariden, wie z. B. Agarose, oder von Acrylamiden oder von Glutaralehyd etc.umfasst

Eine solche spezielle Ausführungsform einer erfindungsgemässen analytischen Plattform ist auch dadurch gekennzeichnet, dass die Bestandteile der einen oder mehreren "Immobilisierungsproben" nach deren Mischung mit einer Polymerenlösung, eingebunden in eine dreidimensionale Netzwerkstruktur immobilisiert auf der Evaneszentfeld-Sensorplattform vorliegen und dort zugänglich für ihre Nachweissubstanzen sind, mit denen sie in einem nachfolgenden Schritt einer Bioaffinitätsreaktion in Kontakt gebracht werden. Damit kann ein höherer Grad der Oberflächenbedeckung der Evaneszentfeld-Sensorplattform als eine Monoschicht erreicht werden, was zu einer weiteren Erhöhung der messbaren Signale beim Schritt des Analytnachweises führen kann. Dabei sollte sich die geschaffene Polymeren-Netzwerkstruktur jedoch nicht über die Eindringtiefe des evaneszenten Feldes ins Medium hinaus erstrecken, da ein Analytnachweis jenseits dieser Entfernung von der Oberfläche der Evaneszentfeld-Sensorplattform nicht möglich ist.

Vorteilhaft sind solche Ausführungsformen einer erfindungsgemässen analytischen Plattform, bei denen ein Array mehr als 50, bevorzugt mehr als 500, besonders bevorzugt mehr als 5000 Messbereiche umfasst.

Dabei kann jeder Messbereich eine zu anderen Messbereichen gleiche oder unterschiedliche immobilisierte Probe enthalten.

Die Messbereiche eines Arrays sind in einer Dichte von mehr als 10, bevorzugt von mehr als 100, besonders bevorzugt von mehr als 1000 Messbereichen pro Quadratzentimeter angeordnet.

Vorteilhaft ist auch eine solche Ausführungsform einer erfindungsgemässen analytischen Plattform, welche dadurch gekennzeichnet ist, dass auf der Evaneszenzfeld-Sensorplattform als festem Träger eine Vielzahl von Arrays von Messbereichen angeordnet sind. Insbesondere können auf der Evaneszenzfeld-Sensorplattform mindestens 5, bevorzugt mindestens 50 Arrays von Messbereichen angeordnet sein. Besonders vorteihaft ist es, wenn unterschiedliche Arrays von Messbereichen einer solchen Variante einer erfindungsgemässen analytischen Plattform in unterschiedlichen Probenbehältnissen angeordnet sind. Beispielsweise in den Internationalen Patentanmeldungen WO 01/13096 und WO 01/43875 ist beschrieben, wie eine Evaneszentfeld-Sensorplattform, welche für eine erfindungsgemässe analytische Plattform geeignet ist, als Bodenplatte mit einem geeigneten Aufsatzkörper zur Erzeugung eines geeigneten Arrays von Probenbehältnissen, jeweils für die Aufnahme eines Arrays von Messbereichen, kombiniert werden kann.

Mit einer solchen Ausführungsform einer erfindungsgemässen analytischen Plattform wird eine experimentelle Konzeption ermöglicht, welche man als "multidimensional" bezeichnen kann: Beispielsweise können in den Zeilen und Spalten eines Arrays verschiedene Proben, beispielsweise von unterschiedlichen Organismen (z.·B. entsprechend Spalten), in unterschiedlicher Verdünnung (z. B. entsprechend Zeilen) aufgebracht sein. Unterschiedliche Arrays von Messbereichen, in unterschiedlichen Probenbehältnissen, können dann mit unterschiedlichen zweiten Vielzahlen von spezifischen Bindungspartnern als Nachweissubstanzen zur Bestimmung unterschiedlicher immobilisierter Analyten in unterschiedlichen Arrays in Kontakt gebracht werden. Es ist offensichtlich, dass sich mit einer solchen Variante einer erfindungsgemässen analytischen Plattform eine nahezu unbegrenzte Anzahl verschiedener Experimente durchführen lässt.

Von Vorteil ist weiterhin, wenn Bereiche zwischen den diskreten Messbereichen zur Minimierung unspezifischer Bindung von Nachweissubstanzen "passiviert sind", d.h. dass zwischen den räumlich getrennten Messbereichen gegenüber den Analyten und anderen Inhaltsstoffen der aufgebrachten "Immobilisierungsproben" sowie gegenüber den Nachweissubstanzen für besagte Analyten "chemisch neutrale", d.h. diese nicht bindende, Komponenten aufgebracht sind.

Besagte gegenüber den Analyten und anderen Inhaltsstoffen der aufgebrachten "Immobilisierungsproben" sowie gegenüber den Nachweissubstanzen für besagte Analyten "chemisch neutrale", d.h. diese nicht bindende, Komponenten können ausgewählt sein aus den Gruppen, die von Albuminen, insbesondere Rinderserumalbumin oder Humanserumalbumin, Casein, unspezifischen, polyklonalen oder monoklonalen, artfremden oder empirisch für den oder die nachzuweisenden Analyten unspezifischen Antikörpern (insbesondere für Immunoassays), Detergentien - wie beispielsweise Tween 20 -, nicht mit zu analysierenden Polynukleotiden hybridisierender, fragmentierter natürlicher oder synthetischer DNA, wie beispielsweise ein Extrakt von Herings- oder Lachssperma (insbesondere für Polynukleotid-Hybridisierungsassays), oder auch ungeladenen, aber hydrophilen Polymeren, wie beispielsweise Polyethylenglycolen oder Dextranen, gebildet werden.

Ohne Beschränkung der Allgemeinheit kann dass es sich bei den in diskreten Messbereichen einer erfindungsgemässen analytischen Plattform aufgetragenen "Immobilisierungsproben" enthaltenen, nachzuweisenden Analyten beispielsweise um Verbindungen aus der Gruppe handeln, die von Proteinen, beispielsweise mono- oder polyklonalen Antikörpern und Antikörperfragmenten, Peptiden, Enzymen, Glycopeptiden, Oligosacchariden, Lektinen, Antigenen für Antikörper, mit zusätzlichen Bindungsstellen funktionalisierten Proteinen ("Tag-Proteinen", wie beispielsweise "Histidin-Tag-Proteinen") sowie Nukleinsäuren (beispielsweise DNA, RNA) gebildet wird.

Insbesondere kann es sich bei den in diskreten Messbereichen aufgetragenen Proben enthaltenen, nachzuweisenden Analyten um Verbindungen aus der Gruppe handeln, welche cytosolische oder membrangebundene Zellproteine, insbesondere an den Prozessen der Signaltransduktion in Zellen beteiligte Proteine, wie z. B. Kinasen, umfasst. Die Analyten können auch biotechnisch modifizierte, beispielsweise mit lumineszenten bzw. fluoreszenten Gruppen und biologisch expremierte Biopolymere, wie beispielsweise mit "blue fluorescent proteins" (BFP), "green fluorescent proteins" (GFP) oder "red fluorescent proteins" (RFP) sein.

Eine spezielle Variante einer erfindungsgemässen analytischen Plattform ist dadurch gekennzeichnet, dass die Evaneszentfeld-Sensorplattform, als Bestandteil der analytischen Plattform, eine dünne Metallschicht, gegebenenfalls auf einer darunter befindlichen Zwischenschicht mit Brechungsindex vorzugsweise < 1.5, wie beispielsweise Siliciumdioxid oder Magnesiumfluorid, umfasst, wobei die Dicke der Metallschicht und der eventuellen Zwischenschicht so ausgewählt ist, dass ein Oberflächenplasmon bei der Wellenlänge eines eingestrahlten Anregungslichts und / oder bei der Wellenlänge einer erzeugten Lumineszenz angeregt werden kann.

Dabei wird bevorzugt, dass das Metall ausgewählt ist aus der Gruppe, welche Gold und Silber umfasst. Ausserdem wird bevorzugt, dass die Metallschicht eine Dicke zwischen 10 nm und 1000 nm, bevorzugt zwischen 30 nm und 200 nm, hat.

Vorzugsweise umfasst die Evaneszentfeld-Sensorplattform als fester Träger einen optischen Wellenleiter, umfassend eine oder mehrere Schichten. Es kann sich dabei beispielsweise um einen faseroptischen Wellenleiter, bestehend aus mehreren Schichten handeln. Bevorzugt handelt es sich jedoch úm einen planaren optischen Wellenleiter, welcher durchgehend über eine Oberfläche der Evaneszentfeld-Sensorplattform ausgebildet ist oder auch in diskrete wellenleitende Bereiche aufgeteilt sein kann, wie dieses beispielsweise in der Patentanmeldung WO 96/35940 beschrieben ist.

Besonders bevorzugt wird eine solche Ausführungsform des erfindungsgemässen Verfahrens, welche dadurch gekennzeichnet ist, dass die Evaneszentfeld-Sensorplattform als fester Träger einen planaren optischen Dünnschichtwellenleiter mit einer im wesentlichen optisch transparenten, wellenleitenden Schicht auf einer zweiten, ebenfalls im wesentlichen optisch transparenten Schicht mit niedrigerem Brechungsindex als die wellenleitende Schicht und gegebenenfalls einer ebenfalls im wesentlichen optisch transparenten Zwischenschicht zwischen der werllenleitenden Schicht (a) und der zweiten Schicht mit ebenfalls niedrigerem Brechungsindex als die wellenleitende Schicht umfasst.

Vorzugsweise ist eine erfindungsgemässe analytische Plattform so gestaltet, dass eine wellenleitende Schicht der Evaneszenzfeld-Sensorplattform mit einem oder mehreren optischen Koppelelementen in optischem Kontakt steht, welche die Einkopplung von Anregungslicht von einer oder mehreren Lichtquellen in die besagte wellenleitende Schicht ermöglichen, wobei besagte optische Koppelelemente ausgewählt sind aus der Gruppe von Prismenkopplern, evaneszenten Kopplern mit zusammengebrachten optischen Wellenleitern mit überlappenden evaneszenten Feldern, Stirnflächenkopplern mit vor einer Stirnseite der besagten wellenleitenden Schicht der Evaneszentfeld-Sensorplattform angeordneten fokussierenden Linsen, vorzugsweise Zylinderlinsen, und Gitterkopplern.

Besonders bevorzugt wird, wenn in einer wellenleitenden Schicht der Evaneszentfeld-Sensorplattform eine oder mehrere Gitterstrukturen zur Auskopplung mit gleicher oder unterschiedlicher Gitterperiode und Gittertiefe wie Gitterstrukturen zur Einkopplung ausgeprägt sind, welche die Auskopplung von in besagter wellenleitender Schicht geführtem Licht ermöglichen.

Weitere Ausführungsformen von Evaneszenzfeld-Sensorplattformen, welche für eine erfindungsgemässe analytische Plattform geeignet sind, sind beispielsweise in den Patentanmeldungen WO 95/33197, WO 95/33198 und WO96/35940 beschrieben, welche hiermit ebenfalls vollumfänglich als Bestandteil der vorliegenden Erfindung eingeführt werden.

Ein weiterer Gegenstand der vorliegenden Erfindung ist die Verwendung eines erfindungsgemässen Verfahrens oder einer erfindungsgemässen analytischen Plattform zu quantitativen und / oder qualitativen Analysen zur Bestimmung chemischer, biochemischer oder biologischer Analyten in Screeningverfahren in der Pharmaforschung, der Kombinatorischen Chemie, der Klinischen und Präklinischen Entwicklung, zu Echtzeitbindungsstudien und zur Bestimmung kinetischer Parameter im Affinitätsscreening und in der Forschung, zu qualitativen und quantitativen Analytbestimmungen, insbesondere für die DNA- und RNA-Analytik und die Bestimmung von genomischen oder proteomischen Unterschieden im Genom, wie beispielsweise Einzelnukleotid-Polymorphismen, zur Messung von Protein-DNAwechselwirkungen, zur Bestimmung von Steuemngsmechanismen für die m-RNA-Expression und für die Protein(bio)synthese, für die Erstellung von Toxizitätsstudien sowie für die Bestimmung von Expressionsprofilen, insbesondere zur Bestimmung von biologischen und chemischen Markerstoffen, wie mRNA, Proteinen, Peptiden oder niedermolekularen organischen (Boten-)Stoffen, sowie zum Nachweis von Antikörpern, Antigenen, Pathogenen oder Bakterien in der pharmazeutischen Protduktforschung und -entwicklung, der Human- und Veterinärdiagnostik, der Agrochemischen Produktforschung und -entwicklung, der symptomatischen und präsymptomatischen Pflanzendiagnostik, zur Patientenstratifikation in der pharmazeutischehn Produktentwicklung und für die therapeutische Medikamentenauswahl, zum Nachweis von Pathogenen, Schadstoffen und Erregern, insbesondere von Salmonellen, Prionen, Viren und Bakterien, insbesondere in der Lebensmittel- und Umweltanalytik.

Die Erfindung wird nachfolgend anhand von Ausführungsbeispielen weiter erläutert, wobei die dort beschriebenen Ausführungsformen keine Einschränkung der Allgemeinheit bedeuten.

### Beispiele

### 1. Analytische Plattform

### 1.1. Evaneszentfeld-Sensorplattform

Als analytische Plattform dient eine Evaneszentfeld-Sensorplattform als fester Träger mit den Abmessungen 14 mm Breite x 57 mm Länge x 0.7 mm Dicke. Die Evaneszentfeld-Sensorplattform ist als ein Dünnschichtwellenleiter ausgeprägt, umfassend ein Glasssubstrat (AF 45) und eine darauf aufgebrachte 150·nm dünne, hochbrechende Schicht aus Tantalpentoxid. In dem Glassubstrat sind, parallel zur Länge, zwei Oberflächenreliefgitter (Gitterperiode: 318 nm, Gittertiefe: (12 +/- 2) nm) im Abstand von 9 mm moduliert. Bei der nachfolgenden Auftragung der hochbrechenden Schicht wurden diese Strukturen, die als diffraktive Gitter der Lichteinkopplung in die hochbrechende Schicht dienen sollen, in die Oberfläche der Tantalpentoxidschicht übertragen.

Nach sorgfältiger Reinigung der Evaneszentfeld-Sensorplattform wird auf der Oberfläche der Metalloxidschicht eine Monoschicht aus Mono-Dodecylphosphat (DDP) durch spontane Selbstorganisation als Haftvermittlungsschicht erzeugt, mittels Abscheidung aus wässriger Lösung (0.5 mM DDP). Diese Oberflächenmodifikation der zuvor hydrophilen Metalloxidoberfläche führt zu einer hydrophoben Oberfläche (mit einem Kontaktwinkel von etwa 100° gegenüber Wasser), auf der eine Vielzahl von "Immobilisierungsproben" mit darin enthaltenen Analyten als spezifischen Bindungspartnern für den Analytnachweis in einer spezifischen Bindungsreaktion aufgetragen werden sollen.

Auf den mit der hydrophoben Haftvermittlungsschicht versehenen Evaneszentfeld-Sensorplattformen werden 6 identische Mikroarrays von je 90 Messbereichen (Spots), ihrerseits in einer Anordnung von jeweils 10 Reihen und 9 Spalten, mit einem Inkjet-Spotter (Modell BCA1, Perkin Elmer, Boston, MA,USA) aufgetragen. Jeder Spot wird durch die Auftragung eines einzelnen Tröpfchens von 280 pL Volumen auf die Chipoberfläche erzeugt.

### 1.2. Reagentien und Erzeugung der Arrays von Messbereichen

Für den Nachweis von biologisch relevanten Proteinanalyten in "Immobilisierungsproben"werden humane T-Zell-Kulturen (Jurkat, DMZ # ACC282) benutzt. Diese Zellen werden in einer Lösung enthaltend RPMI 1640, 10% FCS (Fötales Rinderserum), 2mM Glutamin, 50 U/ml Penicillin, 50 µg/ml Streptamycin bei 37°C kultiviert (Zelldichte ca. 0.5-1.0 x 10⁶ Zellen/ml). Diese Zellen werden dann mit Antikörpern, nämlich "Maus-anti-human-CD3" ("mouse-α-human-CD3") bzw. "Maus-anti-human-CD28" ("mouse-α-human-CD28"), gegen die Oberflächenrezeptoren CD3 bzw. CD28 (in einer Lösung von je 1 µg/ml für 10 min) inkubiert. Für eine Vergleichsprobe zu den mit den Antikörpern behandelten Zellkulturen wird eine ansonsten gleichartige Zellkultur benutzt, die unbehandelt blieb und im analytischen Nachweisverfahren als Negativ-Kontrolle dienen wird. Eine weitere ansonsten gleichartige Zellkultur wird mit Staurosporin (Konzentration: 10 µM), einem starken Proteinkinase-Inhibitor, 180 min lang behandelt.

Anschliessend werden die so behandelten bzw. unbehandelten Zellkulturen auf 4°C gekühlt und 2 Minuten bei einer Zentrifugationskraft von 350x g pelletiert (Zellzahl ca. 10⁷). Dabei werden die Zellen lediglich, ohne Schädigung, vom Medium getrennt. Danach wird der Überstand dekantiert und Lysepuffer (7 M Harnstoff, 2 M Thioharnstoff, 4% CHAPS, 1% DTT, 4 mM Spermidin und Complete (Proteaseinhibitor, Roche AG, 1 Tablette/50 ml)) zugegeben, wobei die Totalproteinkonzentration auf etwa 10 mg/ml eingestellt wird.. Alle proteinhaltigen Zellbestandteile werden hierbei spontan und vollständig denaturiert und solubilisiert.

Durch Zentrifugation bei 13 000 x g wird das DNA enthaltende Material abzentrifugiert. Der Überstand stellt wird dann, nach nochmaliger Verdünnung um einen Faktor 10 (siehe unten) als "Immobilisierungsprobe" verwendet.

Die Behandlung mit den genannten Antikörpern dient als Modellsystem für die costimulatorische Aktivierung von humanen T-Zellen (M. Diehn et al., "Genomic expression programs and the integration of the CD28 costimulatory signal in T cell activation", Proceedings of the National Academy of Sciences 99 (2002) 11796 - 11801). Die Bindung besagter Antikörper an zellmembrangebundene Rezeptoren löst eine Phosphorylierungskaskade mit anschlossenen, unterschiedlichen Signalwegen innerhalb der betroffenen Zellen aus. Die Aktivität eines bestimmten Signalwegs lässt sich hierbei durch die Bestimmung des Phosphorylierungsgrades eines entsprechenden involvierten Schlüsselproteins (als einem sogenannten "Markerprotein") oder dessen Substrats nachweisen, was mithilfe einer erfindungsgemässen analytischen Plattform durchgeführt werden soll.

Die mithilfe der vorangehend beschriebenen Vorbereitungsschritte gewonnenen Proben werden nochmals um einen Faktor 10 auf eine Totalproteinkonzentration von etwa 1 mg/ml verdünnt und dann in diskreten Messbereichen zur Erzeugung eines Arrays von Messbereichen auf der mit der Haftvermittlungsschicht versehenen Evaneszentfeld-Sensorplattfonn aufgetragen.

Zusätzlich zu den Messbereichen mit darin aufgebrachten Proben enthält jedes Mikroarray weitere Messbereiche mit darin immobilisiertem Cy5-fluoreszenzmarkiertem Rinderserumalbumin (Cy5-BSA), die zur Referenzierung von lokalen Unterschieden und / oder zeitlichen Variationen der Anregungslichtintensität bei der Messung verwendet werden ("Referenz-Spots"). Cy5-BSA wird in einer Konzentration von 1.0 nM in phosphatgepufferter Kochsalzlösung (PBS, pH 7.4) aufgetragen (Markierungsrate: 3 Cy5-Moleküle pro BSA-Molekül).

Nach Aufbringen der "Immobilisierungsproben" und Cy5-BSA wird die analytische Plattform zwei Stunden lang bei Raumtemperatur und 100 % relativer Luftfeuchte gelagert und anschliessend bei Raumluft getrocknet. Dann werden die freien, nicht protein-bedeckten, hydrophoben Oberflächenbereiche der Evaneszentfeldsensor-Plattform mit Rinderserumalbumin (BSA) abgesättigt, indem die Oberfläche mit einer Lösung von BSA (30 mg/ml) in 50 mM Imidazol / 100 mM NaCl (pH 7.4) inkubiert wird. Danach wird die Evaneszentfeldsensor-Plattform mit den darauf erzeugten Messbereichen mit Wasser gewaschen und anschliessend in einem Stickstoffstrom getrocknet und bei 4°C bis zur Durchführung des erfindungsgemässen Nachweisverfahrens gelagert.

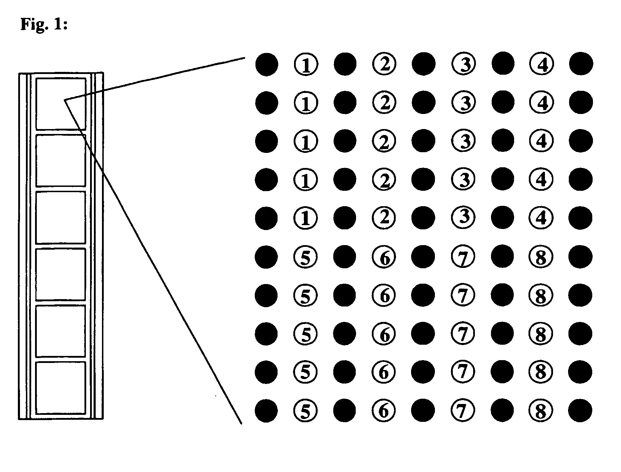

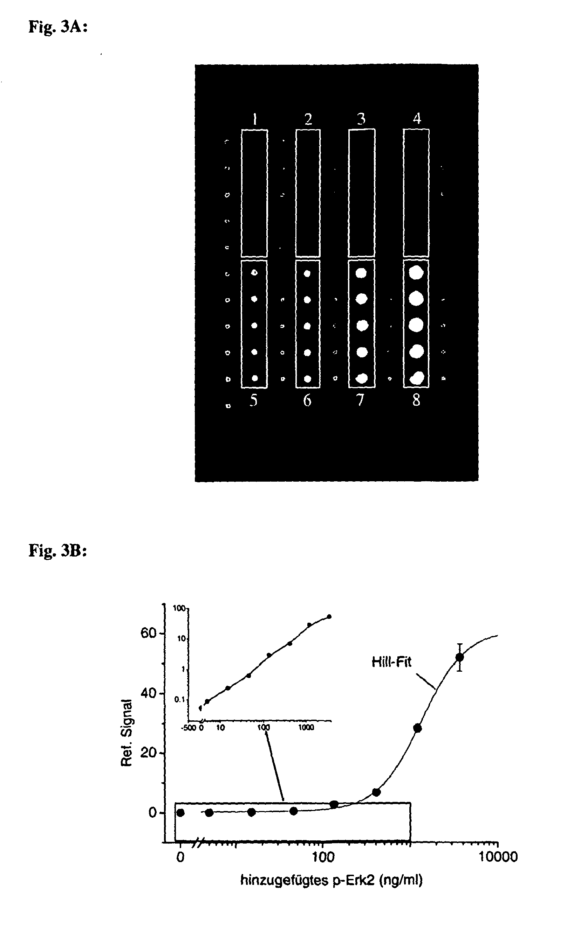

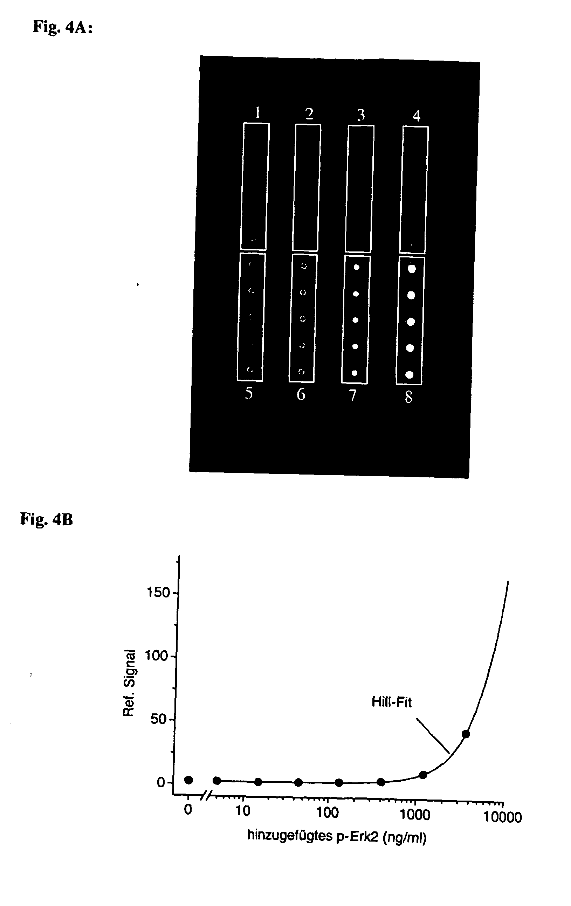

Die Geometrie einer typischen Anordnung der Messbereiche in einem zweidimensionalen Array und eine lineare Anordnung von sechs (identischen) Arrays auf einer Evaneszentfeldsensor-Plattform ist in Figur 1 dargestellt (für die Beispiele, die anhand von Figur 3A/B bzw. Figur 4A/B genauer besprochen werden). Der Durchmesser der Spots, mit einem Abstand (Zentrum-zu-Zentrum) von 600 µm, beträgt etwa 90 µm. Ein Array von Messbereichen umfasst für diese Beispiele jeweils eine Anordnung von Messbereichen mit 8 verschiedenen, in 5 Replikaten aufgetragenenen Proben, wobei die 5 gleichartigen Messbereiche jeweils in einer gemeinsamen Spalte senkrecht zur Aubreitungsrichtung des während des Detektionsschritts in der wellenleitenden Schicht der analytischen Plattform geführten Lichts angeordnet sind. Mithilfe der jeweils 5 gleichartigen Messbereiche soll die Reproduzierbarkeit der Mess-Signale innerhalb des Arrays von Messbereichen bestimmt werden. Zwischen und neben den Spalten von Messbereichen mit darin aufgebrachten zu anylsierenden Proben sind jeweils Spalten von Messbereichen mit darin aufgebrachtem Cy5-BSA (zu Referenzierungszwecken) angeordnet. Die erfindungsgemässe analytische Plattform umfasst in diesem Beispiel 6 gleichartige derartige Arrays von Messbereichen, wie in Figur 1 dargestellt.

### 2. Analytisches Nachweisverfahren

### 2.1. Assay-Architektur

Der Nachweis bestimmter Proteine allgemein (d. h. z. B. mit oder ohne Phosphorylierung) bzw. bestimmter Proteine speziell in aktivierter (z.B. phosphorylierter) Form in den immobilisierten Zell-Lysaten als in diskreten Messbereichen aufgetragenen "Immobilisierungsproben" erfolgt mithilfe sequentieller Zugabe entsprechender Nachweisreagentien als Assay-Schritten vor der Vermessung der resultierenden Fluoreszenzsignale: Zur Vorbereitung für einen ersten Assay-Schritt werden polyklonale analytspezifische Kaninchen-Antikörper (Antikörper A1 (#2261): Phospho-(Ser) PKC Substrat; Antikörper A2 (#9611): Phospho-(Ser/Thr) Akt Substrat; Antikörper A3 (#9101): Phospho-p44/42 MAP Kinase (Thr202/Tyr204); Antikörper A4 (#9102): p44/42 MAP Kinase (Thr202/Tyr204); alle Antikörper bezogen von Cell Signaling Technology, INC., Beverly, MA, USA) typischerweise im Verhältnis 1:500 in Assay-Puffer (50 mM Imidazol, 100 mM NaCl, 0.1% BSA, 0.05% Tween 20 pH 7.4) verdünnt. Von diesen vier verschiedenen Antikörperlösungen werden jeweils 30 µl auf jeweils eines der 6 identischen Arrays von Messbereichen aufgebracht, gefolgt von einer Inkubation bei Raumtemperatur über Nacht (Erster Assay-Schritt). Überschüssige, nicht spezifisch gebundene Antikörper werden durch Waschen eines jeden Arrays mit Assaypuffer (5x100 ul) entfernt.

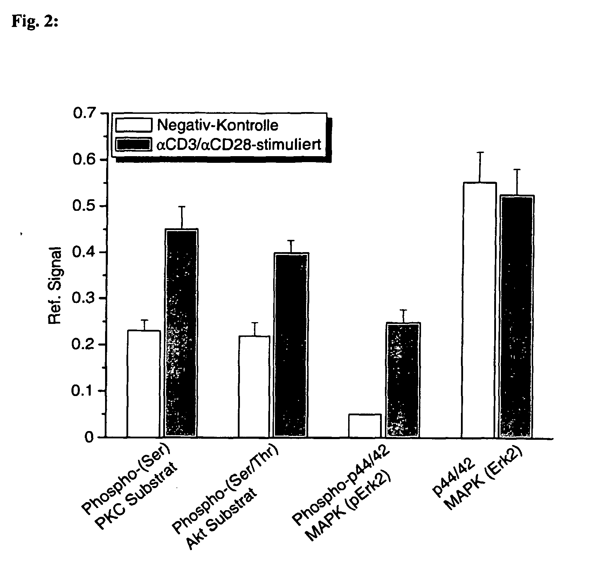

Die hier benutzten 4 verschiedenen Antikörper sind grundsätzlich unterschiedlicher Natur: Antikörper A1 und A2 erkennen und binden eine Reihe unterschiedlicher, an Serin bzw. Serin/Threonin phosphorylierten Proteinen, welche Proteinkinasen als Substrate dienen. Dieses ist an der Vielzahl der Banden im Western-Blot zu erkennen (Figur 2A und Abschnitt 2.4, "Ergebnisse"). Antikörper A3 und A4 erkennen und binden die gleiche Art von Verbindung, nämlich die p44/42 MAP-Kinase (auch Erk2 genannt), wobei jedoch Antikörper A3 nur dessen phosphorylierte "aktive" Form (pErk2) erkennt, während Antikörper A4 beide Formen (die nicht phosphorylierte Form Erk2 und die phosphorylierte Form pErk2) erkennt und daran bindet.

Für den Nachweis von in disketen Messbereichen an dort in den immobilisierten Proben enthaltene gebundene analytspezifische Antikörper erfolgt ein zweiter Assay-Schritt unter Benutzung eines Cy5-markierten anti-Kaninchen-Antikörpers (Amersham Biosciences, Dübendorf, CH), welches an alle vorangehend genannten Antikörper A1 - A4 bindet. Dieser Cy5-markierte Antikörper wird in einer Konzentration von typischerweise 10 nM in Assay-Puffer auf die Arrays aufgebracht (je 30 µl) und damit anschliessend 2 Stunden lang bei Raumtemperatur im Dunkeln inkubiert wird. Anschliessend werden die Arrays mit Assaypuffer (jeweils fünfmal mit 100 µl) gewaschen, um nicht spezifisch gebundene Cy5-anti-Kaninchen Antikörper zu entfernen. Danach werden die so vorbereiteten analytischen Plattformen bis zum Detektionsschritt mittels Anregung und Detektion resultierender Fluoreszenzsignale im ZeptoREADER^{™} (siehe unten) gelagert.

### 2.2. Bestimmung der Fluoreszenzsignale aus den Arrays von Messbereichen

Die Fluoreszenzsignale aus den verschiedenen Arrays von Messbereichen werden mit einem ZeptoREADER^{™} (Zeptosens AG, CH-4108 Witterswil, Schweiz) sequentiell automatisch gemessen Für jedes Array von Messbereichen wird die erfindungsgemässe analytische Plattform justiert zur Erfüllung der Resonanzbedingung für die Lichteinkopplung in die wellenleitende TantalpentoxidSchicht und zur Maximierung des in den Messbereichen verfügbaren Anregungslichts. Anschliessend wird von jedem Array eine vom Benutzer wählbare Anzahl von Bildern der Fluoreszenzsignale aus dem betreffenden Array erzeugt, wobei unterschiedliche Belichtungszeiten gewählt werden können. Die Anregungswellenlänge beträgt bei den Messungen für das vorliegende Beispiel 633 nm, die Detektion des Fluoreszenzlicht erfolgt mit einer gekühlten Kamera bei der Fluoreszenzwellenlänge von Cy5, unter Verwendung eines Interferenzfilters (Transmission 670±20 nm) zur Unterdrückung von Streulicht bei der Anregungswellenlänge, der vor dem Objektiv der Kamera positioniert ist. Die erzeugten Fluoreszenzbilder werden automatisch auf der Speicherplatte des Steuer-Computers abgespeichert. Weitere Details des optischen Systems (ZeptoREADER™) sind in der internationalen Patentanmeldung PCT/EP 01/10012 beschrieben, welche hiermit vollumfänglich als Bestandteil dieser Anmeldung eingeführt wird.

### 2.3. Auswertung und Referenzierung:

Die mittlere Signalintensität aus den Messbereichen (Spots) wird bestimmt mithilfe einer Bildanalyse-Software (ZeptoVIEW, Zeptosens AG, CH-4108 Witterswil), welche es ermöglicht, die Fluoreszenzbilder einer Vielzahl von Arrays von Messbereichen halbautomatisch auzuwerten.

Die Rohdaten der einzelnen Pixel der Kamera stellen eine zweidimensionale Matrix digitalisierter Messwerte dar, mit der gemessenen Intensität als Messwert eines einzelnen Pixels entsprechend der auf ihn abgebildeten Fläche auf der Sensorplattform. Für die Auswertung der Daten wird zunächst manuell ein zweidimensionales (Koordinaten-) Netz über die Bildpunkte (Pixelwerte) gelegt derart, dass das Teilbild jedes Spots in ein individuelles zweidimensionales Netzelement fällt. Innerhalb dieses Netzelements wird jedem Spot ein kreisförmiger möglichst gut anzupassender "Auswertebereich" (area of interest, AOI) mit einem vom Benutzer vorzugebenden Radius (typischerweise 90 µm) zugeordnet. Durch die Bildanalysesoftware wird der Ort der einzelnen AOIs individuell als Funktion der Signalintensität der einzelnen Pixel bestimmt. Dabei bleibt der zu Beginn vom Nutzer vorgegebene Radius der AOIs erhalten. Als mittlere Bruttosignalintensität eines jeden Spots wird das arithmetische Mittel der Pixelwerte (Signalintensitäten) innerhalb eines gewählten Auswertebereichs bestimmt.

Die Hintergrundsignale werden bestimmt aus den gemessenen Signalintensitäten zwischen den Spots. Dazu werden pro Spot vier weitere kreisförmige Flächen (mit typischerweise gleichem Radius wie für die Auswertebereiche der Spots) als Auswertebereiche zur Hintergrundsignalbestimmung definiert, welche vorzugsweise in der Mitte zwischen zwischen benachbarten Spots angeordnet sind. Aus diesen vier Kreisflächen wird die mittlere Hintergrundsignalintensität beispielsweise als das arithmetische Mittel der Pixelwerte (Signalintensitäten) innerhalb eines hierfür gewählten AOIs bestimmt. Die mittlere Nettosignalintensität aus den Messbereichen (Spots) wird dann als Differenz zwischen der lokalen mittleren Brutto- und der lokalen mittleren Hintergrundsignalintensität des jeweiligen Spots berechnet.

Die Referenzierung der Netto-Signalintensität aller Spots erfolgt geweils mithilfe von Referenzspots (Cy5-BSA) eines jeden Arrays von Messbereichen. Dazu wird die Netto-Signalintensität eines jeden Spots durch den Mittelwert der NettoSignalintensitäten der benachbarten Referenzspots derselben Reihe (angeordnet parallel zur Ausbreitungsrichtung des in der Evaneszentfeld-Sensorplattform geführten Lichts) dividiert. Durch diese Referenzierung werden die lokalen Unterschiede der verfügbaren Anregungslichtintensität orthogonal zur Lichtausbreitungsrichtung sowohl innerhalb eines jeden Mikroarrays als auch zwischen verschiedenen Mikroarrays kompensiert.

### 2.4. Ergebnisse

Figur 2 zeigt die Ergebnisse, welche mit dem erfindungsgemässen Verfahren mit der erfindungsgemässen analytischen Plattform erzielt wurden. Das Balkendiagramm zeigt jeweils im Vergleich die Ergebnisse der mit den Antikörpern gegen die Oberflächenrezeptoren CD23 ("αCD3") und CD28 ("αCD28") behandelten Zellkultur (ausgefüllte Balken) und der unbehandelten Kultur ("Negativ-Kontrolle", leere Balken), welche daraus gewonnenen "natur-identischen" Proben nach Aufbringung in jeweils 6 gleichartigen Arrays von Messbereichen auf einer oben beschriebenen Evaneszentfeld-Sensorplattform mit den Lösungen der verschiedenen Antikörper A1 - A4 zusammengebracht worden sind. Dabei wurden auf jeweils 4 gleichartige Arrays, die in unterschiedlichen Probenbehältnissen auf einer gemeinsamen Evaneszentfeld-Sensorplattform angeordnet waren, jeweils unterschiedliche, jeweils einen der 4 genannten Antikörper A1 - A4 enthaltene Lösungen aufgebracht und anschliessend, wie unter 2.1. beschrieben, Cy5-markierte anti-Kaninchen-Antikörper zugeführt. In Figur 2 sind jeweils die Mittelwerte der nach dem vorangehend beschriebenen Verfahren referenzierten Signalintensitäten von jeweils fünf gleichartigen Messbereichen innerhalb eines Arrays und deren Standardabweichungen dargestellt.

Dabei korrelieren die ermittelten Signalintensitäten mit der Konzentration eines jeweilig auftretenden bestimmten Analyten (hohe Signalintensität entsprechend hoher Konzentration). Es ist deutlich zu erkennen, dass sich durch Behandlung der Jurkat-Zellkulturen mit den Antikörpern gegen die Oberflächenrezeptoren CD23 ("αCD3") und CD28 ("αCD28") ("Stimulation") die relative intrazelluläre Konzentration an Phospho-(Ser) PKC- und Phospho-(Ser/Thr) Akt Substraten jeweils auf etwa das Doppelte, im Vergleich zur Negativ-Kontrolle erhöhte. Noch stärker, nämlich um den Faktor 5, stieg die Konzentration an pErk2, wobei die Summe des Gehaltes an Erk2 und pErk2 (detektiert mithilfe von Antikörper A4) innerhalb der Messgenauigkeit konstant blieb. Dieses bedeutet, dass innerhalb der Stimulationsdauer von 10 Minuten der Gesamtgehalt an Erk2 nicht durch Anstieg der Expression erhöht wurde, sondern nur der Gehalt an pErk2 durch Phosphorylierung gesteigert wurde.

Um die Empfindlichkeit des erfindungsgemässen Verfahrens für den Nachweis eines einzelnen interessierenden "Markerproteins" in einer in einem Messbereich aufgebrachten Probe, d.h. in dem in einem einzelnen Messbereich immobilisierten Proteom, zu prüfen, wird ein unbehandeltes Zell-Lysat (Negativ-Kontrolle), welches gemäss dem zuvor durchgeführtem Versuch (dessen Ergebnisse in Figur 2 dargestellt sind) nachweislich nur einen sehr geringen Gehalt an pErk2 (drittes Balkenpaar in Figur 2) enthielt, für einen zweiten Versuch in einzelne Teillösungen aufgeteilt, welche mit diesem "Markerprotein" in unterschiedlichen Konzentrationen von (0-3645 ng/ml) versetzt werden. Anschliessend werden diese Lösungen, wie zuvor beschrieben, auf dem planaren Wellenleiterchip immobilisiert und ein Assay gemäss 2.1 (unter Verwendung von Antikörper A3) durchgerührt. Figur 3A zeigt eine typische Signalverteilung aus einem Array von Messbereichen, wobei die markierten Rechtecke jeweils 5 Replikatspots einer zu dem unbehandelten Zell-Lysat hinzugegebenen pErk2-Konzentration darstellen (1-8: aufsteigende Konzentration, gemäss der in Figur 1 dargestellten geometrischen Anordnung).

Das Ergebnis dieser Messung für die Detektion von pErk2, in Abhängigkeit von dessen hinzugegebener Konzentration, kann durch eine typische Bindungskurve beschrieben werden, indem an den Konzentrationsverlauf der Signale eine Hill-Funktion angepasst ("gefittet") wird (Fig. 3B). Jeder Datenpunkt in Figur 3B stellt den Mittelwert der referenzierten Nettosignalintensitäten von 5 Replikat-Analytspots, jeweils mit der durch Fehlerbalken markierten zugehörigen Standardabweichung, dar. Der vergrösserte Bildausschnitt in Figur 3B zeigt den Konzentrationsverlauf der Signale für die niedrigeren Konzentrationen, dessen Anstieg in der Darstellung für den gesamten Konzentrationsverlauf nicht mehr aufgelöst werden kann. Als Empfindlichkeit des Assays (Limit of Detection) wird, basierend auf der Summe des Signals des 0-Wertes ("Blank", ohne hinzugefiigtes p-Erk2) und dessen zweifacher Standardabweichung, ein Wert von 2.0 ng/ml bestimmt, was einer Gewichtsfraktion von 2.3 x 10⁻⁶ g pro g Totalprotein entspricht.

Die Figuren 4A und 4B zeigen die Ergebnisse eines zu dem zweiten im wesentlichen analogen dritten Versuchs. Als Unterschied zum vorangehend beschriebenen zweiten Versuch mit den in Figur 3A und 3B dargestellten Ergebnissen wird in diesem dritten Versuch das Assay gemäss 2.1 unter Verwendung des Antikörpers A4 (d.h. unter Zugabe zu gleichartigen Arrays wie im zweiten Versuch) durchgeführt. In diesem Versuch wird also die Gesamtheit von phosphorylierter und nicht phosphorylierter Verbindung (pErk2 und Erk2), entsprechend der unterschiedlichen Zugabe von pErk2, bestimmt. Figur 4A zeigt eine typische Signalverteilung aus einem Array von Messbereichen, wobei die markierten Rechtecke wiederum jeweils 5 Replikatspots einer zu dem unbehandelten Zell-Lysat hinzugegebenen pErk2-Konzentration darstellen (1-8: aufsteigende Konzentration, gemäss der in Figur 1 dargestellten geometrischen Anordnung). In diesem Fall wird für die Emfindlichkeit des Assays (Limit of Detection) ein Wert von 110 ng/ml bestimmt, was einer Gewichtsfraktion von 1.1x10⁻⁴ g pro g Totalprotein entspricht.

In einem weiteren, vierten Experiment wird untersucht, ob sich auch unterschiedliche Änderungen der Konzentration an pErk2 infolge Co-Stimulation von Jurkat-Zellen mit αCD3/αCD28, mit unterschiedlicher Stimulationsdauer nachweisen lassen und ob die Unterschiede dieser Änderungen mit dem erfindungsgemässen Verfahren aufgelöst werden können.. Dazu werden Jurkat-Zellkulturen vor ihrer Lyse jeweils unterschiedlich lange (im Minutenbereich) mit je 1 µg/ml αCD3/αCD28 inkubiert. Weiterhin wird eine Jurkat Zell-Kultur mit Staurosporin (Proteinkinase-Inhibitor) behandelt. Letzere Zellkultur dient als eine Negativkontrolle, da hier aufgrund der Inhibition aller Proteinkinasen kein pErk2 vorhanden sein sollte (siehe auch Abschnitt 1.2), ein hierfür gemessenes Signal also dem Signal einer von pErk2 freien Probe entsprechen sollte. Anschliessend werden die so behandelten Zell-Lysate auf die Evaneszentfeld-Sensor-Plattform gespottet und ein Assay gemäss 2.1, unter Benutzung des Antikörpers A3 zum Nachweis der Änderungen der Konzentrationen von pErk2, durchgeführt.

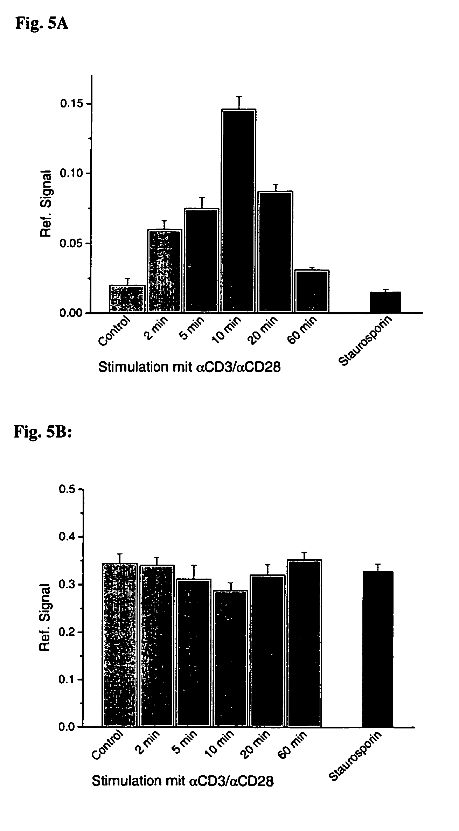

Das Ergebnis dieser Messung ist in Fig. 5A dargestellt. Jeder der im Graph gezeigten Balken stellt den referenzierten Mittelwert der Nettosignalintenstät von 5 Replikat-Analytspots mit der dazugehörigen Standardabweichung dar. Es ist gut zu erkennen, dass sich die Änderung der pErk2-Konzentration, detektiert mit dem Antikörper 3, sehr gut auflösen lässt, wobei die Zeitabhängigkeit, d.h. die Abhängigkeit von der Stimulationsdauer, durch einen schnellen Anstieg der pErk2-Konzentration gekennzeichnet ist, gefolgt von einem Abfall auf das Niveau der Anfangskonzentration nach einer Stimulationsdauer von 60 Minuten, wobei das Konzentrationsmaximum nach etwa 10 Minuten erreicht wird. Das Signal der nicht stimulierten Kontrollprobe liegt geringfügig höher als als dasjenige der mit Staurosporin behandelten Probe, was den natürlichen Anteil von pErk2 ohne Stimulation repräsentiert.

Als Kontrollmessung werden ein ansonsten gleichartiges Assay und Nachweisverfahren durchgeführt, wobei anstelle des Antikörpers 3 der Antikörper 4 zum Nachweis der Gesamtheit der entsprechenden phosphorylierten und nicht phosphorylierten Proteinform, also des relativen Gesamtgehalts an Erk2/pErk2 eingesetzt wird. Hierbei wird innerhalb der experimentellen Genauigkeit kein signifikanter Unterschied der Signale, d.h. keine Konzentrationsänderung für die unterschiedlichen Stimulationsdauern, von bis zu 60 Minuten, und auch kein Unterschied zur unbehandelten Kontrollprobe und zur mit Staurosporin behandelten Probe festgestellt (Fig. 5B).

## Patentansprüche

1. Verfahren zur Untersuchung einer Vielzahl von Proben auf in den Proben enthaltene, als Teilnehmer an spezifischen Bindungsreaktionen biologisch relevante Verbindungen als Analyten, **dadurch gekennzeichnet, dass**
- besagte Proben oder Fraktionen besagter Proben mit den darin enthaltenen, nachzuweisenden Analyten, als einer ersten Vielzahl von spezifischen Bindungspartnem direkt oder nach zusätzlichen Verdünnungen der Fraktionen in mindestens einem ein- oder zweidimensisonalen Array in diskreten Messbereichen auf einer Evaneszentfeld-Sensorplattform als festem Träger aufgetragen werden, wobei unterschiedliche Proben oder Fraktionen oder unterschiedliche Verdünnungen von Proben oder Fraktionen in unterschiedlichen diskreten Messbereichen angeordnet werden,
- eine oder mehrere Nachweissubstanzen, als eine zweite Vielzahl spezifischer Bindungspartner, zum spezifischen Nachweis von einem oder mehreren in den Proben oder deren Fraktionen enthaltenen Analyten, aus besagter ersten Vielzahl spezifischer Bindungspartner, in einem einzigen oder mehreren Schritten einer spezifischen Bindungsreaktion mit den in besagten diskreten Messbereichen aufgetragenen Proben oder Fraktionen oder deren Verdünnungen in Kontakt gebracht werden,
- Änderungen von optoelektronischen Signalen als Folge der Bindung von Nachweissubstanzen an in diskreten Messbereichen enthaltene Analyten, im evaneszenten Feld der Evaneszentfeld-Sensorplattform, ortsaufgelöst gemessen werden und
aus der relativen Grösse der Änderungen besagter optoelektronischer Signale aus den jeweiligen Messbereichen qualitativ und / oder quantitativ das Vorhandensein der dort spezifisch nachzuweisenden Analyten bestimmt wird.

2. Verfahren nach Anspruch 1, **dadurch gekennzeichnet, dass** ein Trennverfahren zur Aufteilung der Probe in eine oder mehrere besagte Fraktionen aus der Gruppe von Verfahren, die von Zentrifugation, High Pressure Liquid Chromatography und micro High Pressure Liquid Chromatography mittels der Methode von Normal Phase, Reverse Phase, Ionenaustausch- oder Hydrophober Interaktionschromatographie, Size Exclusion Chromatography, Gelchromatographie, Elektrophorese, Kapillarelektrophorese, Elektrochromatographie, Free-Flow Electrophoresis gebildet wird.

3. Verfahren nach einem der Ansprüche 1-2, **dadurch gekennzeichnet, dass** eine Fraktion einer Probe vor der Auftragung auf besagter Evaneszentfeld-Sensorplattform als festem Träger mindestens um einen Faktor 10 verdünnt wird.

4. Verfahren nach einem der Ansprüche 1-2, **dadurch gekennzeichnet, dass** eine Fraktion einer Probe vor der Auftragung auf besagter Evaneszentfeld-Sensorplattform als festem Träger mindestens um einen Faktor 30 verdünnt wird.

5. Verfahren nach einem der Ansprüche 1-4, **dadurch gekennzeichnet, dass** die Proben ausgewählt sind aus der Gruppe von Extrakten gesunder oder krankhafter Zellen, Extrakten von tierischem oder menschlichem Gewebe oder von Pflanzengewebe, sowie von Körperflüssigkeiten oder deren Bestandteilen.

6. Verfahren nach einem der Ansprüche 1-4, **dadurch gekennzeichnet, dass** die Proben ausgewählt sind aus der Gruppe, welche Extrakte stimulierter oder unbehandelter Zellen und Extrakte gesunden oder krankhaften Gewebes umfasst.

7. Verfahren nach einem der Ansprüche 1-6, **dadurch gekennzeichnet, dass** eine zu untersuchende Probe aus einem Organismus oder Gewebe- oder Zellverband oder Zelle mittels einer Methode aus der Gruppe von Gewebeschnitten, Biopsie oder "Laser Capture Micro Dissection" entnommen wurde.

8. Verfahren nach einem der Ansprüche 1-7, **dadurch gekennzeichnet, dass** eine immobilisierte Probe_das Material von weniger als 20000 Zellen umfasst.

9. V erfahren nach einem der Ansprüche 1-8, **dadurch gekennzeichnet, dass** eine immobilisierte Probe das Material von weniger als 1 000 Zellen umfasst.

10. Verfahren nach einem der Ansprüche 1-9, **dadurch gekennzeichnet, dass** in einer immobilisierten Probe enthaltene Analyten in nativer oder denaturierter Form vorliegen.

11. Verfahren nach einem der Ansprüche 1-9, **dadurch gekennzeichnet, dass** die in einer immobilisierten Probe enthaltenen Analyten, Nukleinsäuren oder Proteine, nach Behandlung mit Harnstoff, in denaturierter Form vorliegen, wobei die Epitope der besagten Analyten für die Bindung ihrer jeweiligen Nachweissubstanzen frei zugänglich sind.

12. Verfahren nach einem der Ansprüche 1-11, **dadurch gekennzeichnet, dass** die in einer immobilisierten Probe enthaltenen relativen Gesamtmengen von einer oder mehreren Verbindungen als Analyten, als Summe von deren Vorkommen in phosphorylierter oder nicht phosphorylierter Form und / oder glykolisierter und / oder nicht glykolisierter Form, bestimmt werden.

13. Verfahren nach einem der Ansprüche 1-11, **dadurch gekennzeichnet, dass** die in einer immobilisierten Probe enthaltenen relativen Mengen von einer oder mehreren Verbindungen als Analyten, jeweils von deren Vorkommen in phosphorylierter und / oder nicht phosphorylierter Form und / oder glykolisierter und / oder nicht glykolisierter Form, für eine oder mehrere besagter Formen bestimmt werden.

14. Verfahren nach einem der Ansprüche 1-11, **dadurch gekennzeichnet, dass** der Aktivierungsgrad eines oder mehrerer in einer immobilisierten Probe enthaltenen Analyten bestimmt wird.

15. Verfahren nach einem der Ansprüche 1-11, **dadurch gekennzeichnet, dass** der Phosphorylierunsgrad und / oder Glykolisierungsgrad eines oder mehrerer in einer immobilisierten Probe enthaltenen Analyten bestimmt wird.

16. Verfahren nach einem der Ansprüche 1-15, **dadurch gekennzeichnet, dass** Unterschiede von weniger als 20 %.bevorzugt von weniger als 10 %, von den in einer immobilisierten Probe und in einer oder mehreren Vergleichsproben enthaltenen relativen Mengen von einer oder mehreren Verbindungen in phosphorylierter und / oder nicht phosphorylierter Fonn und / oder glykolisierter und / oder nicht glykolisierter Fonn als Analyten, für eine oder mehrere besagter Formen nachgewiesen werden.

17. Verfahren nach einem der Ansprüche 1 - 16, **dadurch gekennzeichnet, dass** besagte immobilisierte Probe und eine oder mehrere Vergleichsproben dem gleichen Ursprungsort zu unterschiedlichen Zeitpunkten entnommen sind und dass zeitliche Veränderungen der in diesen Proben enthaltenen relativen Mengen von einer oder mehreren Verbindungen in phosphorylierter und / oder nicht phosphorylierter Fonn und / oder glykolisierter und / oder nicht glykolisierter Form als Analyten, bestimmt werden.

18. Verfahren nach einem der Ansprüche 1-17, **dadurch gekennzeichnet, dass** unterschiedliche Proben dem gleichen Organismus oder der gleichen Zellkultur entnommen wurden.

19. Verfahren nach Anspruch 18, **dadurch gekennzeichnet, dass** unterschiedliche Proben an verschiedenen Positionen des gleichen Organismus entnommen wurden.

20. Verfahren nach einem der Ansprüche 1-17, **dadurch gekennzeichnet, dass** unterschiedliche Proben verschiedenen Organismen oder verschiedenen Zellkulturen entnommen wurden.

21. Verfahren nach einem der Ansprüche 1-20, **dadurch gekennzeichnet, dass** die Evaneszentfeld-Sensorplattform, zur Verbesserung des Haftvermögens der in diskreten Messbereichen aufzubringenden immobilisierten Probe, eine Haftvermittlungsschicht umfasst, auf welcher besagte Fraktionen von Proben oder Verdünnungen dieser Fraktionen aufgetragen werden.

22. Verfahren nach Anspruch 21, **dadurch gekennzeichnet, dass** besagte Haftvermittlungsschicht eine Dicke von weniger als 200 nm, bevorzugt von weniger als 20 nm hat.

23. Verfahren nach einem der Ansprüche 21-22, **dadurch gekennzeichnet, dass** besagte Haftvermittlungsschicht Verbindungen umfasst aus der Gruppe von Silanen, funktionalisierten Silanen, Epoxiden, funktionalisierten, geladenen oder polaren Polymeren und selbstorganisierten passiven oder funktionalisierten Mono- oder Mehrfachschichten, Thiolen, Alkylphosphaten und -phosphonaten, multifunktionellen Block-Copolymeren.

24. Verfahren nach einem der Ansprüche 21-22, **dadurch gekennzeichnet, dass** besagte Haftvermittlungsschicht Verbindungen umfasst aus der Gruppe von Organophosphorsäuren der allgemeinen Formel I (A)
Y -B-OPO₃ H₂ (IB)
oder von Organophosphonsäuren der allgemeinen Formel I (B)
Y -B-PO₃ H₂ (IB)
und deren Salzen, in denen Beinen Alkyl-, Alkenyl-, Alkinyl-, Aryl-, Aralkyl-, Hetaryl-oder Hetarylalkylrest und Y Wasserstoff oder eine funktionelle Gruppe aus der Reihe Hydroxy, Carboxy, Amino, gegebenfalls durch Niederalkyl substituiertes Mono-oder Dialkylamino, Thiol, oder eine negative Säuregruppe aus der Reihe Ester, Phosphat, Phosphonat, Sulfat, Sulfonat, Maleimid, Succinimydyl, Epoxy oder Acrylat bedeutet.

25. Verfahren nach einem der Ansprüche 1-24, **dadurch gekennzeichnet, dass** eine oder mehrere immobilisierten Probe vor ihrer Auftragung auf der Evaneszentfeld-Sensorplattform als festem Träger mit einer Lösung von Polymeren oder polymerisierbaren Monomeren, gegebenenfalls in Anwesenheit von Initiatoren, oder von chemischen Cross-Linkem gemischt werden.

26. Verfahren nach Anspruch 25, **dadurch gekennzeichnet, dass** besagte Lösung von Polymeren oder polymerisierbaren Monomeren oder chemischen Cross-Linkem ausgewählt ist aus der Gruppe, welche Lösungen von Polysacchariden umfasst.

27. Verfahren nach einem der Ansprüche 25-26, **dadurch gekennzeichnet, dass** die Mischung der einen oder mehreren immobilisierten Proben mit einer Lösung von Polymeren oder polymerisierbaren Monomeren in der Immobilisierung einer dreidimensionalen Netzwerkstruktur mit darin eingebundenen, für Nachweissubstanzen in dem nachfolgenden Schritt einer Bioaffinitätsreaktion zugänglichen Probenbestandteilen, auf der Evaneszentfeld-Sensorplattform als festem Träger resultiert.

28. Verfahren nach einem der Ansprüche 1-20, **dadurch gekennzeichnet, dass** die immobilisierten Probe in diskreten Messbereichen auf der Evaneszentfeld-Sensorplattform direkt oder auf einer darauf aufgebrachten Haftvermittlungsschicht räumlich selektiv mithilfe eines Verfahrens aufgebracht werden, welches ausgewählt ist aus der Gruppe von Verfahren, die von "Inkjet spotting", mechanischem Spotting mittels Stift, Feder oder Kapillare, "Micro contact printing", fluidischer Kontaktierung der Messbereiche mit besagten Proben durch deren Zufuhr in parallelen oder gekreuzten Mikrokanälen, unter Einwirkung von Druckunterschieden oder elektrischen oder elektromagnetischen Potentialen sowie photochemischen oder photolithographischen Immobilisierungsverfahren gebildet wird.

29. Verfahren nach einem der Ansprüche 1-28, **dadurch gekennzeichnet, dass** Bereiche zwischen den diskreten Messbereichen zur Minimierung unspezifischer Bindung von Nachweissubstanzen zwischen den räumlich getrennten Messbereichen gegenüber den Analyten und anderen Inhaltsstoffen der aufgebrachten immobilisierten Probe sowie gegenüber den Nachweissubstanzen für besagte Analyten diese nicht bindende, Komponenten aufgebracht sind.

30. Verfahren nach Anspruch 29, **dadurch gekennzeichnet, dass** diese nicht bindende, Komponenten ausgewählt sind aus den Gruppen, die von Albuminen, insbesondere Rinderserumalbumin oder Humanserumalbumin, Casein, unspezifischen, polyklonalen oder monoklonalen, artfremden oder empirisch für den oder die nachzuweisenden Analyten unspezifischen Antikörpern, Detergentien, nicht mit zu analysierenden Polynukleotiden hybridisierender, fragmentierter natürlicher oder synthetischer DNA, oder auch ungeladenen, aber hydrophilen Polymeren gebildet werden.

31. Verfahren nach einem der Ansprüche 1-23, **dadurch gekennzeichnet, dass** es sich bei den in diskreten Messbereichen aufgetragenen immobilisierten Proben enthaltenen, nachzuweisenden Analyten um Verbindungen aus der Gruppe handelt, die von Proteinen, beispielsweise mono- oder polyklonalen Antikörpern und Antikörperfragmenten, Peptiden, Enzymen, Glycopeptiden, Oligosacchariden, Lektinen, Antigenen für Antikörper, mit zusätzlichen Bindungsstellen funktionalisierten Proteinen sowie Nukleinsäuren gebildet wird.

32. Verfahren nach Anspruch 31, **dadurch gekennzeichnet, dass** es sich bei den in diskreten Messbereichen aufgetragenen immobilisierten Proben enthaltenen, nachzuweisenden Analyten um Verbindungen aus der Gruppe handelt, welche cytosolische oder membrangebundene Zellproteine, insbesondere an den Prozessen der Signaltransduktion in Zellen beteiligte Proteine Kinasen, umfasst.

33. Verfahren nach einem der Ansprüche 1-32, **dadurch gekennzeichnet, dass** die ortaufgelöst zu messenden Änderungen optoelektronischer Signale, als Folge der Bindung von Nachweissubstanzen an in diskreten Messbereichen in den immobilisierten Proben enthaltene Analyten, durch lokale Änderungen der Resonanzbedingungen zur Erzeugung eines Oberflächenplasmons in einer dünnen Metallschicht als Teil besagter Evaneszentfeld-Sensorplattform hervorgerufen werden.

34. Verfahren nach Anspruch 33, **dadurch gekennzeichnet, dass** besagte Änderungen der Resonanzbedingungen in einer Änderung des Resonanzwinkels für die Einstrahlung eines Anregungslichts zur Erzeugung eines Oberflächenplasmons in einer dünnen Metallschicht als Teil besagter Evaneszentfeld-Sensorplattform bestehen.

35. Verfahren nach Anspruch 33, **dadurch gekennzeichnet, dass** besagte Änderungen der Resonanzbedingungen in einer Änderung der Resonanzwellenlänge eines eingestrahlten Anregungslichts zur Erzeugung eines Oberflächenplasmons in einer dünnen Metallschicht als Teil besagter Evaneszentfeld-Sensorplattform bestehen.

36. Verfahren nach einem der Ansprüche 1-35, **dadurch gekennzeichnet, dass** die ortaufgelöst zu messenden Änderungen optoelektronischer Signale, als Folge der Bindung von Nachweissubstanzen an in diskreten Messbereichen in den immobilisierten Proben enthaltene Analyten, durch lokale Änderungen des effektiven Brechungsindex in diesen Bereichen auf besagter Evaneszentfeld-Sensorplattform hervorgerufen werden.

37. Verfahren nach einem der Ansprüche 1-32, **dadurch gekennzeichnet, dass** die ortaufgelöst zu messenden Änderungen optoelektronischer Signale, als Folge der Bindung von Nachweissubstanzen an in diskreten Messbereichen in den immobilisierten Proben enthaltene Analyten, durch lokale Änderungen einer oder mehrerer Lumineszenzen von innerhalb des evaneszenten Feldes besagter Evaneszentfeld-Sensorplattform befindlichen lumineszenzfähigen Molekülen hervorgerufen werden.

38. Verfahren nach Anspruch 37, **dadurch gekennzeichnet, dass** besagte Änderungen einer oder mehrerer Lumineszenzen von lumineszenzfähigen Molekülen oder lumineszenzfähigen Nanopartikeln stammen, welche als Lumineszenzlabel an eine oder mehrere Nachweissubstanzen für die in diskreten Messbereichen enthaltenen Analyten gebunden sind.

39. Verfahren nach Anspruch 38, **dadurch gekennzeichnet, dass** zum Analytnachweis zwei oder mehr Lumineszenzlabel mit unterschiedlichen Emissionswellenlängen und / oder unterschiedlichen Anregungsspektren, bevorzugt mit unterschiedlichen Emissionswellenlängen und gleicher Anregungswellenlänge, eingesetzt werden.

40. Verfahren nach einem der Ansprüche 38-39, **dadurch gekennzeichnet, dass** zum Analytnachweis zwei oder mehr Lumineszenzlabel mit unterschiedlichen Emissionsabklingzeiten eingesetzt werden

41. Verfahren nach einem der Ansprüche 39-40, **dadurch gekennzeichnet, dass** zum Nachweis unterschiedlicher Analyten in einer immobilisierten Probe zwei oder mehr Lumineszenzlabel verwendet werden.

42. Verfahren nach einem der Ansprüche 39-41, **dadurch gekennzeichnet, dass** zum Nachweis unterschiedlicher Analyten in einem Messbereich zwei oder mehr Lumineszenzlabel verwendet werden.

43. Verfahren nach einem der Ansprüche 37-42, **dadurch gekennzeichnet, dass** die Einstrahlung des Anregungslichts in Pulsen mit einer Dauer zwischen 1 fsec und 10 Minuten erfolgt und das Emissionslicht aus den Messbereichen zeitlich aufgelöst gemessen wird.

44. Verfahren nach einem der Ansprüche 36-43, **dadurch gekennzeichnet, dass** die Evaneszentfeld-Sensorplattform als fester Träger einen optischen Wellenleiter, umfassend eine oder mehrere Schichten, umfasst.

45. Verfahren nach Anspruch 44, **dadurch gekennzeichnet, dass** die Evaneszentfeld-Sensorplattform als fester Träger einen durchgehenden oder in diskrete wellenleitende Bereiche aufgeteilten planaren optischen Wellenleiter, umfassend eine oder mehrere Schichten, umfasst.

46. Verfahren nach Anspruch 45, **dadurch gekennzeichnet, dass** die Evaneszentfeld-Sensorplattform als fester Träger einen planaren optischen Dünnschichtwellenleiter mit einer im wesentlichen optisch transparenten, wellenleitenden Schicht auf einer zweiten, ebenfalls im wesentlichen optisch transparenten Schicht mit niedrigerem Brechungsindex als Schicht und gegebenenfalls einer ebenfalls im wesentlichen optisch transparenten Zwischenschicht zwischen der wellenleitenden Schicht und zweiten Schicht mit ebenfalls niedrigerem Brechungsindex als die wellenleitende Schicht umfasst.

47. Verfahren nach einem der Ansprüche 1-46, **dadurch gekennzeichnet, dass** Anregungslicht von einer oder mehreren Lichtquellen in eine wellenleitende Schicht der Evaneszentfeld-Sensorplattform über ein oder mehrere optische Koppelemente eingekoppelt wird, welche ausgewählt sind aus der Gruppe, die von Prismenkopplern, evaneszenten Kopplern mit zusammengebrachten optischen Wellenleitern mit überlappenden evaneszenten Feldern, Stirnflächenkopplern mit vor einer Stirnseite der wellenleitenden Schicht angeordneten fokussierenden Linsen, vorzugsweise Zylinderlinsen, und Gitterkopplern gebildet wird.

48. Verfahren nach Anspruch 47, **dadurch gekennzeichnet, dass** die Einkopplung von Anregungslicht in eine wellenleitende Schicht der Evaneszentfeld-Sensorplattform mithilfe von einer oder mehreren Gitterstrukturen zur Einkopplung erfolgt, die in der besagten wellenleitenden Schicht ausgeprägt sind.

49. Verfahren nach einem der Ansprüche 1-48, **dadurch gekennzeichnet, dass** die Auskopplung von in einer wellenleitenden Schicht der Evaneszentfeld-Sensorplattform geführtem Licht mithilfe von einer oder mehreren Gitterstrukturen zur Auskopplung erfolgt, die in besagter wellenleitender Schicht ausgeprägt sind und gleiche oder unterschiedliche Periode und Gittertiefe wie Gitterstrukturen zur Einkopplung haben.

50. Verfahren nach einem der Ansprüche 48-49, **dadurch gekennzeichnet, dass** Anregungslicht von einer oder mehreren Lichtquellen über eine Gitterstruktur zur Einkopplung von Anregungslicht in eine wellenleitende Schicht besagter Evaneszentfeld-Sensorplattform eingekoppelt und zu auf der Evaneszentfeld-Sensorplattform befindlichen Messbereichen als geführte Welle geleitet wird, dass weiterhin die im evaneszenten Feld besagter geführter Welle erzeugte Lumineszenz von lumineszenzfähigen Molekülen mit einem oder mehreren Detektoren ortsaufgelöst erfasst und die relative Konzentration eines oder mehrerer Analyten aus der relativen Intensität dieser Lumineszenzsignale bestimmt wird.

51. Verfahren nach einem der Ansprüche 37-50, **dadurch gekennzeichnet, dass** neben der Bestimmung einer oder mehrerer Lumineszenzen Änderungen des effektiven Brechungsindex auf den Messbereichen bestimmt werden.

52. Verfahren nach einem der Ansprüche 33-51, **dadurch gekennzeichnet, dass** die einen oder mehreren Lumineszenzen und / oder Bestimmungen von Lichtsignalen bei der Anregungswellenlänge polarisationsselektiv vorgenommen werden.

53. Verfahren nach einem der Ansprüche 47-52, **dadurch gekennzeichnet, dass** die einen oder mehreren Lumineszenzen bei einer anderen Polarisation als der des Anregungslichts gemessen werden.

54. Analytische Plattform zur Untersuchung einer Vielzahl von Proben auf in den Proben enthaltene, als Teilnehmer an Bioaffinitätsreaktionen biologisch relevante Verbindungen als Analyten, umfassend
- eine Evaneszentfeld-Sensorplattform als festen Träger,
- mindestens ein ein- oder zweidimensionales Array von diskreten Messbereichen mit darin immobilisierten Bindungspartnern auf besagter Evaneszentfeld-Sensorplattform für den Nachweis besagter Analyten in einer Boaffinitätsreaktion,
**dadurch gekennzeichnet,**
- **dass** besagte diskrete Messbereiche durch die Auftragung von besagten Proben oder Fraktionen besagter Proben direkt oder nach zusätzlichen Verdünnungen dieser Proben oder von deren Fraktionen, mit den darin nachzuweisenden Analyten, als einer ersten Vielzahl von spezifischen Bindungspartnern, erzeugt wurden,
- **dass** unterschiedliche Proben oder Fraktionen oder unterschiedliche Verdünnungen der Proben oder von deren Fraktionen in unterschiedlichen diskreten Messbereichen angeordnet sind und
- **dass** es sich bei dem einen oder den mehreren immobilisierten Bindungspartnern, welche besagte erste Vielzahl von spezifischen Bindungspartnern bilden, um den einen oder um die mehreren Analyten selbst handelt, welche in den zu untersuchenden Proben enthalten sind.

55. Analytische Plattform nach Anspruch 54, **dadurch gekennzeichnet, dass** eine oder mehrere Proben oder Fraktionen einer Probe vor der Auftragung auf der Evaneszentfeld-Sensorplattform als festem Träger mindestens um einen Faktor 10 verdünnt und unterschiedliche Verdünnungen einer Fraktion in unterschiedlichen diskreten Messbereichen auf besagter Evaneszentfeld-Sensorplattform aufgetragen wurden.

56. Analytische Plattform nach Anspruch 54, **dadurch gekennzeichnet, dass** eine oder mehrere Proben oder Fraktionen einer Probe vor der Auftragung auf der Evaneszentfeld-Sensorplattform als festem Träger mindestens um einen Faktor 30 verdünnt und unterschiedliche Verdünnungen einer Fraktion in unterschiedlichen diskreten Messbereichen auf besagter Evaneszentfeld-Sensorplattform aufgetragen wurden.

57. Analytische Plattform nach einem der Ansprüche 54-56, **dadurch gekennzeichnet, dass** die Proben ausgewählt sind aus der Gruppe von Extrakten gesunder oder krankhafter Zellen, Extrakten von tierischem oder menschlichem Gewebe oder von Pflanzengewebe, sowie von Körperflüssigkeiten oder deren Bestandteilen.

58. Analytische Plattform nach einem der Ansprüche 54-56, **dadurch gekennzeichnet, dass** die Proben ausgewählt sind aus der Gruppe, welche Extrakte stimulierter oder unbehandelter Zellen und Extrakte gesunden oder krankhaften Gewebes umfasst.

59. Analytische Plattform nach einem der Ansprüche 54-58, **dadurch gekennzeichnet, dass** die zu untersuchenden Proben aus einem Organismus oder Gewebe- oder Zell verband oder Zelle mittels einer Methode aus der Gruppe von Gewebeschnitten, Biopsie oder "Laser Capture Micro Dissection" entnommen waren.

60. Analytische Plattform nach einem der Ansprüche 54-59, **dadurch gekennzeichnet, dass** eine immobilisierte Probe das Material von weniger als 20000 Zellen umfasst.

61. Analytische Plattform nach einem der Ansprüche 54-60, **dadurch gekennzeichnet, dass** eine immobilisierte Probe das Material von weniger als 1 000 Zellen umfasst.

62. Analytische Plattform nach einem der Ansprüche 54-61, **dadurch gekennzeichnet, dass** in einer immobilisierten Probe enthaltene enthaltene Analyten, in nativer oder denaturierter Form vorliegen.

63. Analytische Plattform nach einem der Ansprüche 54-61, **dadurch gekennzeichnet, dass** die in den immobilisierten Proben enthaltenen Analyten nach Behandlung mit Harnstoff, in denaturierter Form vorliegen, wobei die Epitope der besagten Analyten für die Bindung ihrer jeweiligen Nachweissubstanzen frei zugänglich sind.

64. Analytische Plattform nach einem der Ansprüche 54-63, **dadurch gekennzeichnet, dass** unterschiedliche aufgetragene Proben dem gleichen Organismus oder der gleichen Zellkultur entnommen wurden.

65. Analytische Plattform nach Anspruch 64, **dadurch gekennzeichnet, dass** unterschiedliche aufgetragene Proben an verschiedenen Positionen des gleichen Organismus entnommen wurden.

66. Analytische Plattform nach einem der Ansprüche 54-63, **dadurch gekennzeichnet, dass** unterschiedliche aufgetragene Proben verschiedenen Organismen oder verschiedenen Zellkulturen entnommen wurden.

67. Analytische Plattform nach einem der Ansprüche 54-66, **dadurch gekennzeichnet, dass** die Evaneszentfeld-Sensorplattfonn, zur Verbesserung des Haftvermögens der in diskreten Messbereichen aufgetragenen immobilisierten Proben, eine Haftvermittlungsschicht umfasst, auf welcher besagte Proben oder deren Fraktionen oder Verdünnungen aufgetragen werden.

68. Analytische Plattform nach Anspruch 67, **dadurch gekennzeichnet, dass** besagte Haftvermittlungsschicht eine Dicke von weniger als 200 nm, bevorzugt von weniger als 20 nm hat.

69. Analytische Plattform nach einem der Ansprüche 67-68, **dadurch gekennzeichnet, dass** besagte Haftvermittlungsschicht Verbindungen umfasst aus der Gruppe von Silanen, funktionalisierten Silanen, Epoxiden, funktionalisierten, geladenen oder polaren Polymeren und "selbstorganisierten passiven oder funktionalisierten Mono-oder Mehrfachschichten", Thiolen, Alkylphosphaten undphosphonaten, multifunktionellen Block-Copolymeren.

70. Analytische Plattform nach einem der Ansprüche 67-68, **dadurch gekennzeichnet, dass** besagte Haftvermittlungsschicht Verbindungen umfasst aus der Gruppe von Organophosphorsäuren der allgemeinen Formel I (A)
Y-B-OP0₃ H₂ (IA)
oder von Organophosphonsäuren der allgemeinen Fonnel I (B)
Y-B-P0₃ H₂ (IB)
und deren Salzen, in denen Beinen Alkyl-, Alkenyl-, Alkinyl-, Aryl-, Aralkyl-, Hetaryl-oder Hetarylalkylrest und Y Wasserstoff oder eine funktionelle Gruppe aus der Reihe Hydroxy, Carboxy, Amino, gegebenfalls durch Niederalkyl substituiertes Mono-oder Dialkylamino, Thiol, oder eine negative Säuregruppe aus der Reihe Ester, Phosphat, Phosphonat, Sulfat, Sulfonat, Maleimid, Succinimydyl, Epoxy oder Acrylat bedeutet

71. Analytische Plattform nach einem der Ansprüche 54-70, **dadurch gekennzeichnet, dass** eine oder mehrere immobilisierten Proben vor ihrer Auftragung auf der Evaneszentfeld-Sensorplattform als festem Träger mit einer Lösung von Polymeren oder polymerisierbaren Monomeren, gegebenenfalls in Anwesenheit von Initiatoren, oder von chemischen "Cross-Linkern gemischt werden.

72. Analytische Plattform nach Anspruch 71, **dadurch gekennzeichnet, dass** besagte Lösung von Polymeren oder polymerisierbaren Monomeren oder chemischen "CrossLinkern" ausgewählt ist aus der Gruppe, welche Lösungen von Polysacchariden umfasst.

73. Analytische Plattform nach einem der Ansprüche 71-72, **dadurch gekennzeichnet, dass** die Mischung der einen oder mehreren immobilisierten Proben mit einer Lösung von Polymeren oder polymerisierbaren Monomeren, gegebenenfalls in Anwesenheit von Initiatoren, oder von chemischen Cross-Linkem in der Immobilisierung einer dreidimensionalen Netzwerkstruktur mit darin eingebundenen, für Nachweissubstanzen in dem nachfolgenden Schritt einer Bioaffinitätsreaktion zugänglichen Probenbestandteilen, auf der Evaneszentfeld-Sensorplattform als festem Träger resultiert.

74. Analytische Plattform nach einem der Ansprüche 54-73, **dadurch gekennzeichnet, dass** ein Array mehr als 50, bevorzugt mehr als 500, besonders bevorzugt mehr als 5000 Messbereiche umfasst.

75. Analytische Plattform nach einem der Ansprüche 54-74, **dadurch gekennzeichnet, dass** die Messbereiche eines Arrays in einer Dichte von mehr als 10, bevorzugt von mehr als 100, besonders bevorzugt von mehr als 1000 Messbereichen pro Quadratzentimeter angeordnet sind.

76. Analytische Plattform nach einem der Ansprüche 54-75, **dadurch gekennzeichnet, dass** auf der Evaneszenzfeld-Sensorplattform als festem Träger eine Vielzahl von Arrays von Messbereichen angeordnet sind.

77. Analytische Plattform nach Anspruch 76, **dadurch gekennzeichnet, dass** auf der Evaneszenzfeld-Sensorplattform als festem Träger mindestens 5, bevorzugt mindestens 50 Arrays von Messbereichen angeordnet sind.

78. Analytische Plattform nach einem der Ansprüche 54-77, **dadurch gekennzeichnet, dass** Bereiche zwischen den diskreten Messbereichen zwischen den räumlich getrennten Messbereichen gegenüber den Analyten und anderen Inhaltsstoffen der aufgebrachten immobilisierten Proben sowie gegenüber den Nachweissubstanzen für besagte Analyten diese nicht bindende, Komponenten aufgebracht sind.

79. Analytische Plattform nach Anspruch 78, **dadurch gekennzeichnet, dass** diese nicht bindende, Komponenten ausgewählt sind aus den Gruppen, die von Albuminen, insbesondere Rinderserumalbumin oder Humanserumalbumin, Casein, unspezifischen, polyklonalen oder monoklonalen, artfremden oder empirisch für den oder die nachzuweisenden Analyten unspezifischen Antikörpern, Detergentien, nicht mit zu analysierenden Polynukleotiden hybridisierender, fragmentierter natürlicher oder synthetischer DNA, oder auch ungeladenen, aber hydrophilen Polymeren gebildet werden.

80. Analytische Plattform nach einem der Ansprüche 54-79, **dadurch gekennzeichnet, dass** es sich bei den in diskreten Messbereichen aufgetragenen immobilisierten Proben enthaltenen, nachzuweisenden Analyten um Verbindungen aus der Gruppe handelt, die von Proteinen sowie Nukleinsäuren gebildet wird.

81. Analytische Plattform nach einem der Ansprüche 54-79, **dadurch gekennzeichnet, dass** es sich bei den in diskreten Messbereichen aufgetragenen immobilisierten Proben enthaltenen, nachzuweisenden Analyten um Verbindungen aus der Gruppe handelt, welche cytosolische oder membrangebundene Zellproteine, insbesondere an den Prozessen der Signaltransduktion in Zellen beteiligte Proteine umfasst.

82. Analytische Plattform nach einem der Ansprüche 54-81, **dadurch gekennzeichnet, dass** die Evaneszentfeld-Sensorplattform eine dünne Metallschicht, gegebenenfalls auf einer darunter befindlichen Zwischenschicht mit Brechungsindex vorzugsweise< 1.5, wie beispielsweise Siliciumdioxid oder Magnesiumfluorid, umfasst, wobei die Dicke der Metallschicht und der eventuellen Zwischenschicht so ausgewählt ist, dass ein Oberflächenplasmon bei der Wellenlänge eines eingestrahlten Anregungslichts und / oder bei der Wellenlänge einer erzeugten Lumineszenz angeregt werden kann.

83. Analytische Plattform nach Anspruch 82, **dadurch gekennzeichnet, dass** das Metall ausgewählt ist aus der Gruppe, welche Gold und Silber umfasst.

84. Analytische Plattform nach Anspruch 82, **dadurch gekennzeichnet, dass** die Metallschicht eine Dicke zwischen 10 nm und 1000 nm, bevorzugt zwischen 30 nm und 200 nm, hat.

85. Analytische Plattform nach einem der Ansprüche 54-84, **dadurch gekennzeichnet, dass** die Evaneszentfeld-Sensorplattform als fester Träger einen optischen Wellenleiter, umfassend eine oder mehrere Schichten, umfasst.

86. Analytische Plattform nach Anspruch 85, **dadurch gekennzeichnet, dass** die Evaneszentfeld-Sensorplattform als fester Träger einen durchgehenden oder in diskrete wellenleitende Bereiche aufgeteilten planaren optischen Wellenleiter, umfassend eine oder mehrere Schichten, umfasst.

87. Analytische Plattform nach Anspruch 86, **dadurch gekennzeichnet, dass** die Evaneszentfeld-Sensorplattform als fester Träger einen planaren optischen Dünnschichtwellenleiter mit einer im wesentlichen optisch transparenten, wellenleitenden Schicht auf einer zweiten, ebenfalls im wesentlichen optisch transparenten Schicht (b) mit niedrigerem Brechungsindex als die wellenleitenden Schicht und gegebenenfalls einer ebenfalls im wesentlichen optisch transparenten Zwischenschicht zwischen der wellenleitenden Schicht und der zweiten Schicht mit ebenfalls niedrigerem Brechungsindex als die wellenleitenden Schicht umfasst.

88. Analytische Plattform nach einem der Ansprüche 54-87, **dadurch gekennzeichnet, dass** eine wellenleitende Schicht der Evaneszenzfeld-Sensorplattform mit einem oder mehreren optischen Koppelelementen in optischem Kontakt steht, welche die Einkopplung von Anregungslicht von einer oder mehreren Lichtquellen in die besagte wellenleitende Schicht ermöglichen, wobei besagte optische Koppelelemente ausgewählt sind aus der Gruppe von Prismenkopplern, evaneszenten Kopplern mit zusammengebrachten optischen Wellenleitern mit überlappenden evaneszenten Feldern, Stirnflächenkopplern mit vor einer Stirnseite der besagten wellenleitenden Schicht der Evaneszentfeld-Sensorplattform angeordneten fokussierenden Linsen, vorzugsweise Zylinderlinsen, und Gitterkopplern.

89. Analytische Plattform nach Anspruch 88, **dadurch gekennzeichnet, dass** in einer wellenleitenden Schicht der Evaneszentfeld-Sensorplattform eine oder mehrere Gitterstrukturen zur Einkopplung ausgeprägt sind, welche die Einkopplung von Anregungslicht von einer oder mehreren Lichtquellen ermöglichen.

90. Analytische Plattform nach einem der Ansprüche 54-88, **dadurch gekennzeichnet, dass** in einer wellenleitenden Schicht der Evaneszentfeld-Sensorplattform eine oder mehrere Gitterstrukturen zur Auskopplung mit gleicher oder unterschiedlicher Gitterperiode und Gittertiefe wie Gitterstrukturen zur Einkopplung ausgeprägt sind, welche die Auskopplung von in besagter wellenleitender Schicht geführtem Licht ermöglichen.

91. Verwendung eines Verfahrens nach einem der Ansprüche 1-53 oder einer analytischen Plattform nach einem der Ansprüche 54-90 zu quantitativen und / oder qualitativen Analysen zur Bestimmung chemischer, biochemischer oder biologischer Analyten in Screeningverfahren in der Pharmaforschung, der Kombinatorischen Chemie, der Klinischen und Präklinischen Entwicklung, zu Echtzeitbindungsstudien und zur Bestimmung kinetischer Parameter im Affinitätsscreening und in der Forschung, zu qualitativen und quantitativen Analytbestimmungen, sowie zum Nachweis von Antikörpern, Antigenen, Pathogenen oder Bakterien in der pharmazeutischen Produktforschung und -entwicklung, der Human- und Veterinärdiagnostik, der Agrochemischen Produktforschung und -entwicklung, der symptomatischen und präsymptomatischen Pflanzendiagnostik, zur Patientenstratifikation in der pharmazeutischen Produktentwicklung und für die therapeutische Medikamentenauswahl, zum Nachweis von Pathogenen, Schadstoffen und Erregern in der Lebensmittel- und Umweltanalytik.

## Claims

1. Method for assaying a multiplicity of samples for compounds present in said samples as analytes, which are biologically relevant as participants of specific binding reactions, **characterized in that**
- said samples or fractions of said samples containing the analytes to be detected as a first multiplicity of specific binding partners are applied directly, or after additional dilutions of said fractions, in at least one one- or two-dimensional array in discrete measurement areas on a evanescent field sensor platform which acts as solid support, with different samples or fractions or different dilutions of samples or fractions being arranged in different discrete measurement areas,
- one or more detection substances, as a second multiplicity of specific binding partners, for specific detection of one or more analytes present in said samples or fractions thereof from said first multiplicity of specific binding partners are contacted in a single step or multiple steps of a specific binding reaction with the samples or fractions or dilutions thereof applied in said discrete measurement areas,
- changes in optoelectronic signals as a result of detection substances binding to analytes present in discrete measurement areas are measured in a space-dissolved manner in the evanescent field of the evanescent field sensor platform, and,
from the relative magnitude of the changes in said optoelectronic signals from the particular measurement areas, the presence of the analytes to be specifically detected there is determined in a qualitative and/or quantitative manner.

2. Method according to Claim 1, **characterized in that** a separation method for dividing the sample into one or more said fractions from the group of methods consisting of centrifugation, high pressure liquid chromatography and micro high pressure liquid chromatography by means of the method of normal phase, reverse phase, ion exchange chromatography or hydrophobic interaction chromatography, size exclusion chromatography, gel chromatography, electrophoresis, capillary electrophoresis, electrochromatography, free-flow electrophoresis.

3. Method according to either of Claims 1-2, **characterized in that** a fraction of a sample is diluted by at least a factor of 10 prior to application to said evanescent field sensor platform which acts as solid support.

4. Method according to either of Claims 1-2, **characterized in that** a fraction of a sample is diluted by at least a factor of 30 prior to application to said evanescent field sensor platform which acts as solid support.

5. Method according to any of Claims 1-4, **characterized in that** the samples are selected from the group of extracts of healthy or diseased cells, extracts of animal or human tissue or of plant tissue, and of bodily fluids or their components.

6. Method according to any of Claims 1-4, **characterized in that** the samples are selected from the group which comprises extracts of stimulated or untreated cells and extracts of healthy or diseased tissue.

7. Method according to any of Claims 1-6, **characterized in that** a sample to be assayed was removed from an organism or a tissue or cell assemblage or cell by means of a method from the group of tissue sections, biopsies or laser capture micro dissection.

8. Method according to any of Claims 1-7, **characterized in that** an immobilized sample comprises the material of fewer than 20 000 cells.

9. Method according to any of Claims 1-8, **characterized in that** an immobilized sample comprises the material of fewer than 1000 cells.

10. Method according to any of Claims 1-9, **characterized in that** analytes present in an immobilized sample are in a native or denatured form.

11. Method according to any of Claims 1-9, **characterized in that** the analytes, nucleic acids or proteins present in an immobilized sample are in a denatured form after treatment with urea, with the epitopes of said analytes being freely accessible for binding of their particular detection substances.

12. Method according to any of Claims 1-11, **characterized in that** the relative total amounts of one or more compounds as analytes, which total amounts are present in an immobilized sample, are determined by way of the sum of the occurrence thereof in a phosphorylated or non-phosphorylated form and/or glycosylated and/or non-glycosylated form.

13. Method according to any of Claims 1-11, **characterized in that** the relative amounts of one or more compounds as analytes, which amounts are present in an immobilized sample, are determined in each case by the occurrence thereof in a phosphorylated and/or non-phosphorylated form and/or glycosylated and/or non-glycosylated form, for one or more of said forms.

14. Method according to any of Claims 1-11, **characterized in that** the degree of activation of one or more analytes present in an immobilized sample is determined.

15. Method according to any of Claims 1-11, **characterized in that** the degree of phosphorylation and/or degree of glycosylation of one or more analytes present in an immobilized sample is determined.

16. Method according to any of Claims 1-15, **characterized in that** differences of less than 20%, preferably of less than 10%, of the relative amounts of one or more compounds in a phosphorylated and/or non-phosphorylated form and/or glycosylated and/or non-glycosylated form as analytes, which relative amounts are present in an immobilized sample and in one or more comparative samples, are detected for one or more of said forms.

17. Method according to any of Claims 1-16, **characterized in that** said immobilized sample and one or more comparative samples have been removed from the same site of origin at different times, and that alterations as a function of time of the relative amounts of one or more compounds in a phosphorylated and/or non-phosphorylated form and/or glycosylated and/or non-glycosylated form as analytes, which relative amounts are present in said samples, are determined.

18. Method according to any of Claims 1-17, **characterized in that** different samples were removed from the same organism or the same cell culture.

19. Method according to Claim 18, **characterized in that** different samples were removed at various positions of the same organism.

20. Method according to any of Claims 1-17, **characterized in that** different samples were removed from various organisms or various cell cultures.

21. Method according to any of Claims 1-20, **characterized in that** the evanescent field sensor platform comprises an adhesion-promoting layer to which said fractions of samples or dilutions of these fractions are applied, in order to improve the adhesive capacity of the immobilized sample to be applied in discrete measurement areas.

22. Method according to Claim 21, **characterized in that** said adhesion-promoting layer has a thickness of less than 200 nm, preferably less than 20 nm.

23. Method according to either of Claims 21-22, **characterized in that** said adhesion-promoting layer comprises compounds from the group consisting of silanes, functionalized silanes, epoxides, functionalized, charged or polar polymers, and self-assembled passive or functionalized monolayers or multiple layers, thiols, alkyl phosphates and alkyl phosphonates, multifunctional block copolymers.

24. Method according to either of Claims 21-22, **characterized in that** said adhesion-promoting layer comprises compounds from the group consisting of organophosphoric acids of the general formula I(A)
Y-B-OPO₃H₂ (IB)
or of organophosphonic acids of the general formula I(B)
Y-B-PO₃H₂ (IB),
and salts thereof, in which B is an alkyl, alkenyl, alkynyl, aryl, aralkyl, hetaryl or hetarylalkyl radical, and Y is hydrogen or a functional group from among hydroxy, carboxy, amino, mono- or dialkylamino optionally substituted by lower alkyl, thiol, or a negative acid group from among ester, phosphate, phosphonate, sulphate, sulphonate, maleimide, succinimidyl, epoxy or acrylate.

25. Method according to any of Claims 1-24, **characterized in that** one or more immobilized samples are mixed with a solution of polymers or polymerizable monomers, where appropriate in the presence of initiators, or of chemical crosslinkers, before being applied to the evanescent field sensor platform which acts as solid support.

26. Method according to Claim 25, **characterized in that** said solution of polymers or polymerizable monomers or chemical crosslinkers is selected from the group comprising solutions of polysaccharides.

27. Method according to either of Claims 25-26, **characterized in that** the mixture of the one or more immobilized samples with a solution of polymers or polymerizable monomers results in the immobilization of a three-dimensional web structure with sample components incorporated therein which are accessible to detection substances in the subsequent step of a bioaffinity reaction, on the evanescent field sensor platform which acts as solid support.

28. Method according to any of Claims 1-20, **characterized in that** the immobilized samples are applied to the evanescent field sensor platform in discrete measurement areas directly or in a spatially selective manner on an adhesion-promoting layer applied thereto, with the aid of a method which is selected from the group of methods consisting of inkjet spotting, mechanical spotting by means of pin, spring or capillary, micro contact printing, fluidic contacting of the measurement areas with said samples by delivering them in parallel or crossed microchannels, under the influence of pressure differences or electric or electromagnetic potentials, and photochemical or photolithographic immobilization methods.

29. Method according to any of Claims 1-28, **characterized in that** areas are applied between the discrete measurement areas in order to minimize unspecific binding of detection substances between the spatially separated measurement areas over the analytes and other ingredients of the immobilized sample applied, and components that do not bind the analytes over the detection substances for said analytes are also applied.

30. Method according to Claim 29, **characterized in that** said non-binding components are selected from the groups consisting of albumins, in particular bovine serum albumin or human serum albumin, casein, unspecific, polyclonal or monoclonal, foreign-species antibodies or antibodies empirically unspecific for the analyte(s) to be detected, detergents, fragmented natural or synthetic DNA that does not hybridize with polynucleotides to be analysed, or else uncharged but hydrophilic polymers.

31. Method according to any of Claims 1-23, **characterized in that** the analytes to be detected and present in immobilized samples applied in discrete measurement areas are compounds from the group consisting of proteins, for example mono- or polyclonal antibodies, and antibody fragments, peptides, enzymes, glycopeptides, oligosaccharides, lectins, antigens for antibodies, proteins functionalized with additional binding sites, and nucleic acids.

32. Method according to Claim 31, **characterized in that** the analytes to be detected and present in immobilized samples applied in discrete measurement areas are compounds from the group consisting of cytosolic or membrane-bound cellular proteins, in particular protein kinases involved in the processes of signal transduction in cells.

33. Method according to any of Claims 1-32, **characterized in that** the changes in optoelectronic signals to be measured in a space-dissolved manner are caused, as a consequence of detection substances binding to analytes present in discrete measurement areas in the immobilized samples, by local changes in the resonance conditions for generating a surface plasmon in a thin metal layer as part of said evanescent field sensor platform.

34. Method according to Claim 33, **characterized in that** said changes in the resonance conditions consist of a change in the resonance angle for illumination with an excitation light for generating a surface plasmon in a thin metal layer as part of said evanescent field sensor platform.

35. Method according to Claim 33, **characterized in that** said changes in resonance conditions consist of a change in the resonance wavelength of an incident excitation light for generating a surface plasmon in a thin metal layer as part of said evanescent field sensor platform.

36. Method according to any of Claims 1-35, **characterized in that** the changes in optoelectronic signals to be measured in a space-dissolved manner are caused, as a consequence of detection substances binding to analytes present in discrete measurement areas in the immobilized samples, by local changes in the effective refractive index in these areas on said evanescent field sensor platform.

37. Method according to any of Claims 1-32, **characterized in that** the changes in optoelectronic signals to be measured in a space-dissolved manner, are caused as a consequence of detection substances binding to analytes in discrete measurement areas in the immobilized samples, by local changes of one or more luminescences of luminescence-capable molecules present within the evanescent field of said evanescent field sensor platform.

38. Method according to Claim 37, **characterized in that** said changes in one or more luminescences stem from luminescent-capable molecules or luminescent-capable nanoparticles which are bound as luminescent labels to one or more detection substances for the analytes present in discrete measurement areas.

39. Method according to Claim 38, **characterized in that** two or more luminescent labels with different emission wavelengths and/or different excitation spectra, preferably with different emission wavelengths and the same excitation wavelength, are employed for analyte detection.

40. Method according to either of Claims 38-39, **characterized in that** two or more luminescent labels with different emission decay rates are employed for analyte detection.

41. Method according to either of Claims 39-40, **characterized in that** different analytes in an immobilized sample are detected using two or more luminescent labels.

42. Method according to any of Claims 39-41, **characterized in that** different analytes in a measurement area are detected using two or more luminescent labels.

43. Method according to any of Claims 37-42, **characterized in that** the illumination with excitation light is carried out in pulses of between 1 fsec and 10 minutes, and the emission light from the measurement areas is measured in a time-resolved manner.

44. Method according to any of Claims 36-43, **characterized in that** the evanescent field sensor platform which acts as solid support comprises an optical waveguide, comprising one or more layers.

45. Method according to Claim 44, **characterized in that** the evanescent field sensor platform which acts as solid support comprises a planar optical waveguide which is either continuous or divided in discrete wave-guiding areas and comprises one or more layers.

46. Method according to Claim 45, **characterized in that** the evanescent field sensor platform which acts as solid support comprises a planar optical thin-film waveguide having an essentially optically transparent, wave-guiding layer on a second, likewise essentially optically transparent layer with a lower refractive index than layer and optionally a likewise essentially optically transparent intermediate layer between the wave-guiding layer and the second layer, likewise with a lower refractive index than the wave-guiding layer.

47. Method according to any of Claims 1-46, **characterized in that** excitation light of one or more light sources is coupled into a wave-guiding layer of the evanescent field sensor platform via one or more optical coupling elements selected from the group consisting of prism couplers, evanescent couplers with united optical waveguides with overlapping evanescent fields, end face couplers with focussing lenses, preferably cylindrical lenses, arranged before an end side of said wave-guiding layer, and grating couplers.

48. Method according to Claim 47, **characterized in that** excitation light is coupled into a wave-guiding layer of the evanescent field sensor platform with the aid of one or more grating structures for coupling which are fashioned in said wave-guiding layer.

49. Method according to any of Claims 1-48, **characterized in that** light guided in a wave-guiding layer of the evanescent field sensor platform is coupled out with the aid of one or more grating structures for coupling out which are fashioned in said wave-guiding layer and which have identical or different period and grating depth with respect to coupling-in grating structures.

50. Method according to any of Claims 48-49, **characterized in that** excitation light of one or more light sources is coupled via a grating structure for coupling in excitation light into a wave-guiding layer of said evanescent field sensor platform and is guided by way of a guided wave to measurement areas on the evanescent field sensor platform, that furthermore the luminescence of luminescence-capable molecules which is generated in the evanescent field of said guided wave is recorded in a space-dissolved manner by one or more detectors and the relative concentration of one or more analytes is determined from the relative intensity of said luminescence signals.

51. Method according to any of Claims 37-50, **characterized in that** changes in the effective refractive index are determined on the measurement areas in addition to the determination of one or more luminescences.

52. Method according to any of Claims 33-51, **characterized in that** the one or more luminescences and/or determinations of the light signals at the excitation wavelength are carried out in a polarization-selective manner.

53. Method according to any of Claims 47-52, **characterized in that** the one or more luminescences are measured at a different polarization than that of the excitation light.

54. Analytical platform for assaying a multiplicity of samples for compounds present in said samples as analytes, which are biologically relevant as participants in bioaffinity reactions, comprising
- an evanescent field sensor platform which acts as solid support,
- at least one one- or two-dimensional array of discrete measurement areas with binding partners immobilized therein on said evanescent field sensor platform for detecting said analytes in a bioaffinity reaction, **characterized in that**
- said discrete measurement areas were generated by applying said samples or fractions of said samples directly or after additional dilutions of said samples or of their fractions, with the analytes to be detected therein, as a first multiplicity of specific binding partners,
- different samples or fractions or different dilutions of said samples or of their fractions are arranged in different discrete measurement areas, and
- said one or more immobilized binding partners, which form said first multiplicity of specific binding partners, are the one or more analytes themselves which are present in the samples to be assayed.

55. Analytical platform according to Claim 54, **characterized in that** one or more samples or fractions of a sample are diluted by at least a factor of 10 prior to application to the evanescent field sensor platform which acts as solid support, and different dilutions of a fraction were applied in different discrete measurement areas on said evanescent field sensor platform.

56. Analytical platform according to Claim 54, **characterized in that** one or more samples or fractions of a sample are diluted by at least a factor of 30 prior to application to the evanescent field sensor platform which acts as solid support, and different dilutions of a fraction were applied in different discrete measurement areas on said evanescent field sensor platform.

57. Analytical platform according to any of Claims 54-56, **characterized in that** the samples are selected from the group of extracts of healthy or diseased cells, extracts of animal or human tissue or of plant tissue, and of bodily fluids or their components.

58. Analytical platform according to any of Claims 54-56, **characterized in that** the samples are selected from the group which comprises extracts of stimulated or untreated cells and extracts of healthy or diseased tissue.

59. Analytical platform according to any of Claims 54-58, **characterized in that** a sample to be assayed was removed from an organism or a tissue or cell assemblage or cell by means of a method from the group of tissue sections, biopsies or laser capture micro dissection.

60. Analytical platform according to any of Claims 54-59, **characterized in that** an immobilized sample comprises the material of fewer than 20 000 cells.

61. Analytical platform according to any of Claims 54-60, **characterized in that** an immobilized sample comprises the material of fewer than 1000 cells.

62. Analytical platform according to any of Claims 54-61, **characterized in that** analytes present in an immobilized sample are in a native or denatured form.

63. Analytical platform according to any of Claims 54-61, **characterized in that** the analytes present in an immobilized sample are in a denatured form after treatment with urea, with the epitopes of said analytes being freely accessible for binding of their particular detection substances.

64. Analytical platform according to any of Claims 54-63, **characterized in that** different applied samples were removed from the same organism or the same cell culture.

65. Analytical platform according to Claim 64, **characterized in that** different applied samples were removed at various positions of the same organism.

66. Analytical platform according to any of Claims 54-63, **characterized in that** different applied samples were removed from various organisms or various cell cultures.

67. Analytical platform according to any of Claims 54-66, **characterized in that** the evanescent field sensor platform comprises an adhesion-promoting layer to which said samples a fractions of samples or dilutions of these fractions are applied, in order to improve the adhesive capacity of the immobilized samples applied in discrete measurement areas.

68. Analytical platform according to Claim 67, **characterized in that** said adhesion-promoting layer has a thickness of less than 200 nm, preferably less than 20 nm.

69. Analytical platform according to either of Claims 67-68, **characterized in that** said adhesion-promoting layer comprises compounds from the group consisting of silanes, functionalized silanes, epoxides, functionalized, charged or polar polymers, and self-assembled passive or functionalized monolayers or multiple layers, thiols, alkyl phosphates and alkyl phosphonates, multifunctional block copolymers.

70. Analytical platform according to either of Claims 67-68, **characterized in that** said adhesion-promoting layer comprises compounds from the group consisting of organophosphoric acids of the general formula I(A)
Y-B-OPO₃H₂ (IA)
or of organophosphonic acids of the general formula I(B)
Y-B-PO₃H₂ (IB),
and salts thereof, in which B is an alkyl, alkenyl, alkynyl, aryl, aralkyl, hetaryl or hetarylalkyl radical, and Y is hydrogen or a functional group from among hydroxy, carboxy, amino, mono- or dialkylamino optionally substituted by lower alkyl, thiol, or a negative acid group from among ester, phosphate, phosphonate, sulphate, sulphonate, maleimide, succinimidyl, epoxy or acrylate.

71. Analytical platform according to any of Claims 54-70, **characterized in that** one or more immobilized samples are mixed with a solution of polymers or polymerizable monomers, where appropriate in the presence of initiators, or of chemical crosslinkers, before being applied to the evanescent field sensor platform which acts as solid support.

72. Analytical platform according to Claim 71, **characterized in that** said solution of polymers or polymerizable monomers or chemical crosslinkers is selected from the group comprising solutions of polysaccharides.

73. Analytical platform according to either of Claims 71-72, **characterized in that** the mixture of the one or more immobilized samples with a solution of polymers or polymerizable monomers, optionally in the presence of initiators or of chemical crosslinkers, results in the immobilization of a three-dimensional web structure with sample components incorporated therein which are accessible to detection substances in the subsequent step of a bioaffinity reaction, on the evanescent field sensor platform which acts as solid support.

74. Analytical platform according to Claims 54-73, **characterized in that** an array comprises more than 50, preferably more than 500, particularly preferably more than 5000, measurement areas.

75. Analytical platform according to any of Claims 54-74, **characterized in that** the measurement areas of an array are arranged in a density of more than 10, preferably of more than 100, particularly preferably of more than 1000, measurement areas per square centimetre.

76. Analytical platform according to any of Claims 54-75, **characterized in that** a multiplicity of arrays of measurement areas are arranged on the evanescent field sensor platform which acts as solid support.

77. Analytical platform according to Claim 76, **characterized in that** at least 5, preferably at least 50, arrays of measurement areas are arranged on the evanescent field sensor platform which acts as solid support.

78. Analytical platform according to any of Claims 54-77, **characterized in that** areas between the discrete measurement areas are applied between the spatially separated measurement areas over the analytes and other ingredients of the immobilized samples applied, and components that do not bind the analytes over the detection substances for said analytes are also applied.

79. Analytical platform according to Claim 78, **characterized in that** said non-binding components are selected from the groups consisting of albumins, in particular bovine serum albumin or human serum albumin, casein, unspecific, polyclonal or monoclonal, foreign-species antibodies or antibodies empirically unspecific for the analyte(s) to be detected, detergents, fragmented natural or synthetic DNA that does not hybridize with polynucleotides to be analysed, or else uncharged but hydrophilic polymers.

80. Analytical platform according to any of Claims 54-79, **characterized in that** the analytes to be detected and present in immobilized samples applied in discrete measurement areas are compounds from the group consisting of proteins and nucleic acids.

81. Analytical platform according to any of Claims 54-79, **characterized in that** the analytes to be detected and present in immobilized samples applied in discrete measurement areas are compounds from the group consisting of cytosolic or membrane-bound cellular proteins, in particular proteins involved in the processes of signal transduction in cells.

82. Analytical platform according to any of Claims 54-81, **characterized in that** the evanescent field sensor platform comprises a thin metal layer, where appropriate on an intermediate layer arranged thereunder and having a refractive index of preferably < 1.5, such as silicon dioxide or magnesium fluoride for example, wherein the thickness of said metal layer and of the possible intermediate layer is selected in such a way that a surface plasmon can be excited at the wavelength of an incident excitation light and/or at the wavelength of a generated luminescence.

83. Analytical platform according to Claim 82, **characterized in that** the metal is selected from the group comprising gold and silver.

84. Analytical platform according to Claim 82, **characterized in that** the metal layer has a thickness of between 10 nm and 1000 nm, preferably between 30 nm and 200 nm.

85. Analytical platform according to any of Claims 54-84, **characterized in that** the evanescent field sensor platform which acts as solid support comprises an optical waveguide, comprising one or more layers.

86. Analytical platform according to Claim 85, **characterized in that** the evanescent field sensor platform which acts as solid support comprises a planar optical waveguide which is either continuous or divided in discrete wave-guiding areas and comprises one or more layers.

87. Analytical platform according to Claim 86, **characterized in that** the evanescent field sensor platform which acts as solid support comprises a planar optical thin-film waveguide having an essentially optically transparent, wave-guiding layer on a second, likewise essentially optically transparent layer (b) with a lower refractive index than the wave-guiding layer and optionally a likewise essentially optically transparent intermediate layer between the wave-guiding layer and the second layer, likewise with a lower refractive index than the wave-guiding layer.

88. Analytical platform according to any of Claims 54-87, **characterized in that** a wave-guiding layer of said evanescent field sensor platform is in optical contact with one or more optical coupling elements which enable excitation light of one or more light sources to be coupled into said wave-guiding layer, said optical coupling elements being selected from the group of prism couplers, evanescent couplers with united optical waveguides with overlapping evanescent fields, end face couplers with focussing lenses, preferably cylindrical lenses, arranged before an end side of said wave-guiding layer of the evanescent field sensor platform, and grating couplers.

89. Analytical platform according to Claim 88, **characterized in that** one or more grating structures which enable excitation light of one or more light sources to be coupled in are fashioned in a wave-guiding layer of the evanescent field sensor platform.

90. Analytical platform according to any of Claims 54-88, **characterized in that** one or more coupling-out grating structures with an identical or different grating period and grating depth with respect to coupling-in grating structures are fashioned in a wave-guiding layer of the evanescent field sensor platform and enable light guided in said wave-guiding layer to be coupled out.

91. Use of a method according to any of Claims 1-53 or of an analytical platform according to any of Claims 54-90 for quantitative and/or qualitative analyses for determining chemical, biochemical or biological analytes in screening methods in pharmaceutical research, combinatorial chemistry, clinical and preclinical development, for real-time binding studies and for determining kinetic parameters in affinity screening and in research, for qualitative and quantitative analyte determinations, and for detecting antibodies, antigens, pathogens or bacteria in pharmaceutical product research and development, human and veterinary diagnosis, agrochemical product research and development, symptomatic and presymptomatic plant diagnosis, for patient stratification in pharmaceutical product development and for therapeutic medicament selection, for detecting pathogens, harmful substances and causative agents in foodstuffs analysis and environmental analysis.

## Revendications

1. Procédé pour étudier un grand nombre d'échantillons, portant sur des composés biologiquement pertinents en tant que participants à des réactions de liaison spécifique, et contenus dans les échantillons, **caractérisé en ce que**
- lesdits échantillons, ou fragments desdits échantillons, avec les analytes à détecter qui y sont contenus, sont appliqués, en tant que première pluralité de partenaires de liaison spécifique, directement ou après des dilutions supplémentaires des fractions, selon au moins une matrice unidimensionnelle ou bidimensionnelle, dans des domaines de mesure discrets sur une plate-forme formant capteur à champ évanescent servant de support solide, différents échantillons ou différentes fractions ou différentes dilutions d'échantillons ou de fractions étant disposés dans des domaines de mesure discrets différents,
- une ou plusieurs substances à détecter, en tant que deuxième pluralité de partenaires de liaison spécifique, sont, pour la détection spécifique d'un ou plusieurs analytes contenus dans les échantillons ou dans les fractions de ceux-ci, constitués de ladite première pluralité de partenaires de liaison spécifique, dans le cadre d'une étape unique ou de plusieurs étapes d'une réaction de liaison spécifique, mises en contact avec les échantillons, ou fractions, ou dilutions de ceux-ci, appliqués dans lesdits domaines de mesure discrets,
- les modifications des signaux optoélectroniques, consécutives à la liaison de substances à détecter à des analytes contenus dans des domaines de mesure discrets, sont mesurées avec résolution locale dans le champ évanescent de la plate-forme formant capteur à champ évanescent, et
à partir de la grandeur relative des modifications desdits signaux optoélectroniques provenant des différents domaines de mesure, on détermine qualitativement et/ou quantitativement la présence des analytes qui doivent y faire l'objet d'une détection spécifique.

2. Procédé selon la revendication 1, **caractérisé en ce qu'**un procédé de séparation, pour fractionner l'échantillon en une ou plusieurs desdites fractions, choisi dans le groupe des procédés qui est formé de la centrifugation, de la chromatographie en phase liquide haute pression et de la microchromatographie en phase liquide haute pression par la méthode de la phase normale, de la phase inverse, de la chromatographie d'interaction hydrophobe ou par échange d'ions, de la chromatographie d'exclusion de taille, de la chromatographie sur gel, de l'électrophorèse, de l'électrophorèse capillaire, de l'électrochromatographie, de l'électrophorèse à écoulement libre.

3. Procédé selon l'une des revendications 1-2, **caractérisé en ce qu'**une fraction d'un échantillon est, avant application sur ladite plate-forme formant capteur à champ évanescent servant de support solide, diluée au moins au 1:10.

4. Procédé selon l'une des revendications 1-2, **caractérisé en ce qu'**une fraction d'un échantillon est, avant application sur ladite plate-forme formant capteur à champ évanescent, servant de support solide, diluée au moins au 1:30.

5. Procédé selon l'une des revendications 1-4, **caractérisé en ce que** les échantillons sont choisis dans le groupe des extraits de plantes saines ou pathologiques, des extraits de tissu animal ou humain, ou de tissus végétaux, ainsi que de liquides corporels ou de leurs constituants.

6. Procédé selon l'une des revendications 1-4, **caractérisé en ce que** les échantillons sont choisis dans le groupe qui comprend des extraits de cellules stimulées ou non traitées, et des extraits de tissus sains ou pathologiques.

7. Procédé selon l'une des revendications 1-6, **caractérisé en ce qu'**un échantillon à étudier a été prélevé d'un organisme ou d'une structure tissulaire ou cellulaire ou d'une cellule, à l'aide d'une méthode du groupe consistant en les coupes tissulaires, les biopsies ou la microdissection par capture laser.

8. Procédé selon l'une des revendications 1-7, **caractérisé en ce qu'**un échantillon immobilisé comprend la matière de moins de 20 000 cellules.

9. Procédé selon l'une des revendications 1-8, **caractérisé en ce qu'**un échantillon immobilisé comprend la matière de moins de 1 000 cellules.

10. Procédé selon l'une des revendications 1-9, **caractérisé en ce que** les analytes contenus dans un échantillon immobilisé se présentent sous forme native ou dénaturée.

11. Procédé selon l'une des revendications 1-9, **caractérisé en ce que** les analytes, acides nucléiques ou protéines contenus dans un échantillon immobilisé se présentent, après traitement à l'urée, sous une forme dénaturée, les épitopes de chacun desdits analytes étant accessibles librement à une liaison de leurs substances à détecter.

12. Procédé selon l'une des revendications 1-11, **caractérisé en ce que** les quantités totales relatives, contenues dans un échantillon immobilisé, d'un ou plusieurs composés servant d'analytes, sont déterminées par la somme de leurs occurrences sous forme phosphorylée ou non phosphorylée et/ou sous forme glycolysée et/ou non glycolysée.

13. Procédé selon l'une des revendications 1-11, **caractérisé en ce que** les quantités relatives, contenues dans un échantillon immobilisé, d'un ou plusieurs composés servant d'analytes, sont chacune déterminées par leurs occurrences sous forme phosphorylée et/ou non phosphorylée et/ou sous forme glycolysée et/ou non glycolysée, pour une ou plusieurs desdites formes.

14. Procédé selon l'une des revendications 1-11, **caractérisé en ce qu'**on détermine le degré d'activation d'un ou plusieurs analytes contenus dans un échantillon immobilisé.

15. Procédé selon l'une des revendications 1-11, **caractérisé en ce qu'**on détermine le degré de phosphorylation et/ou le degré de glycolysation d'un ou plusieurs analytes contenus dans un échantillon immobilisé.

16. Procédé selon l'une des revendications 1-15, **caractérisé en ce qu'**on détecte des différences inférieures à 20 %, de préférence inférieures à 10 %, des quantités relatives, contenues dans un échantillon immobilisé ou dans un ou plusieurs échantillons comparatifs, d'un ou plusieurs composés sous forme phosphorylée et/ou non phosphorylée et/ou sous forme glycolysée et/ou non glycolysée, servant d'analytes, pour une ou plusieurs desdites formes.

17. Procédé selon l'une des revendications 1-16, **caractérisé en ce que** ledit échantillon immobilisé, et un ou plusieurs échantillons comparatifs, sont prélevés du même site d'origine à des instants différents, et que l'on détermine les modifications dans le temps des quantités relatives, contenues dans ces échantillons, d'un ou plusieurs composés sous forme phosphorylée et/ou non phosphorylée et/ou sous forme glycolysée et/ou non glycolysée, servant d'analytes.

18. Procédé selon l'une des revendications 1-17, **caractérisé en ce que** des échantillons différents ont été prélevés du même organisme ou de la même culture cellulaire.

19. Procédé selon la revendication 18, **caractérisé en ce que** des échantillons différents ont été prélevés sur différentes positions du même organisme.

20. Procédé selon l'une des revendications 1-17, **caractérisé en ce que** différents échantillons de différents organismes ou de différentes cultures cellulaires ont été prélevés.

21. Procédé selon l'une des revendications 1-20, **caractérisé en ce que** la plate-forme formant capteur à champ évanescent comprend, pour améliorer l'adhésivité de l'échantillon immobilisé devant être appliqué sur des domaines de mesure discrets, une couche promotrice d'adhérence, sur laquelle lesdites fractions d'échantillons ou de dilutions de ces fractions sont appliquées.

22. Procédé selon la revendication 21, **caractérisé en ce que** ladite couche promotrice d'adhérence a une épaisseur inférieure à 200 nm, de préférence inférieure à 20 nm.

23. Procédé selon l'une des revendications 21-22, **caractérisé en ce que** ladite couche promotrice d'adhérence comprend des composés du groupe consistant en les silanes, les silanes fonctionnalisés, les époxydes, les polymères fonctionnalisés, chargés ou polaires, et les mono- ou multicouches auto-organisées, passives ou fonctionnalisées, les thiols, les alkylphosphates et -phosphonates, les copolymères à blocs multifonctionnels.

24. Procédé selon l'une des revendications 21-22, **caractérisé en ce que** ladite couche promotrice d'adhérence comprend des composés du groupe des acides organophosphoriques de formule générale I(A)
Y-B-OPO₃H₂ (IB)
ou des acides organophosphoniques de formule générale I(B)
Y-B-PO₃H₂ (IB)
et leurs sels, où B désigne un radical alkyle, alcényle, alcynyle, aryle, aralkyle, hétéroaryle ou hétéroarylalkyle, et Y représente un atome d'hydrogène ou un groupe fonctionnel de la série hydroxy, carboxy, amino, mono- ou dialkylamino éventuellement substitué par un ou plusieurs substituants alkyle inférieur, ou un groupe acide négatif de la série esters, phosphates, phosphonates, sulfates, sulfonates, maléimide, succinimydyle, époxy ou acrylate.

25. Procédé selon l'une des revendications 1-24, **caractérisé en ce qu'**on mélange un ou plusieurs échantillons immobilisés, avant leur application sur la plate-forme formant capteur à champ évanescent, servant de support solide, à une solution de polymères ou de monomères polymérisables, éventuellement en présence d'amorceurs ou d'agents de réticulation chimique.

26. Procédé selon la revendication 25, **caractérisé en ce que** ladite solution de polymères ou de monomères polymérisables ou d'agents de réticulation chimique est choisie dans le groupe qui comprend les solutions de polysaccharides.

27. Procédé selon l'une des revendications 25-26, **caractérisé en ce que** le mélange du ou des plusieurs échantillons immobilisés à une solution de polymères ou de monomères polymérisables se traduit par l'immobilisation d'une structure en réseau tridimensionnel, avec des constituants d'échantillons, qui sont incorporés, accessibles à des substances à détecter au cours de l'étape suivante d'une réaction de bioaffinité, sur la plate-forme formant capteur à champ évanescent, servant de support solide.

28. Procédé selon l'une des revendications 1-20, **caractérisé en ce que** les échantillons immobilisés sont appliqués dans des domaines de mesure discrets sur la plate-forme formant capteur à champ évanescent, directement ou sur une couche promotrice d'adhérence appliquée par-dessus, d'une manière spatialement sélective, à l'aide d'un procédé qui est choisi dans le groupe des procédés consistant en le procédé "Inkjet spotting", le dépôt mécanique au crayon, à la plume ou par capillaire, le "Micro contact printing", la mise en contact fluidique des domaines de mesure avec lesdits échantillons, grâce à leur amenée dans des microcanaux parallèles ou croisés, sous l'action de différences de pression ou de potentiels électriques ou électromagnétiques, ainsi que les procédés d'immobilisation photochimiques ou photolithographiques.

29. Procédé selon l'une des revendications 1-28, **caractérisé en ce que** des domaines sont appliqués entre les domaines de mesure discrets pour minimiser la liaison non spécifique de substances à détecter entre les domaines de mesure spatialement séparés par comparaison auxdits analytes et autres constituants de l'échantillon immobilisé appliqué, et des composants qui ne lient pas les analytes par comparaison aux substances à détecter pour lesdits analytes sont également appliqués.

30. Procédé selon la revendication 29, **caractérisé en ce que** les composants ne les liant pas sont choisis dans le groupe consistant en les albumines, en particulier la sérumalbumine bovine ou la sérumalbumine humaine, la caséine, les anticorps non spécifiques, polyclonaux ou monoclonaux, étrangers à l'espèce ou empiriquement non spécifiques pour le ou les analytes à détecter, les détergents, l'ADN fragmenté, naturel ou synthétique, qui ne s'hybrident pas aux polynucléotides à analyser, ou aussi les polymères non chargés, mais hydrophiles.

31. Procédé selon l'une des revendications 1-23, **caractérisé en ce que**, pour ce qui concerne les analytes à détecter, qui sont contenus dans des échantillons immobilisés et appliqués dans des domaines de mesure discrets, il s'agit de composés du groupe consistant en les protéines, par exemple les anticorps et fragments d'anticorps monoclonaux ou polyclonaux, les peptides, les enzymes, les glycopeptides, les oligosaccharides, les lectines, les antigènes d'anticorps, les protéines fonctionnalisées et comportant des sites de liaison supplémentaires, ainsi que les acides nucléiques.

32. Procédé selon la revendication 31, **caractérisé en ce que**, pour ce qui concerne les analytes à détecter, contenus dans des échantillons immobilisés et appliqués par domaines de mesure discrets, il s'agit de composés du groupe consistant en les protéines cellulaires cytosoliques ou liées à la membrane, en particulier des protéine-kinases participant aux processus de transduction du signal dans les cellules.

33. Procédé selon l'une des revendications 1-32, **caractérisé en ce que** les modifications devant subir une mesure à résolution locale de signaux optoélectroniques sont provoquées en conséquence de la liaison de substances à détecter à des analytes contenus par domaines de mesure discrets dans les échantillons immobilisés, sous l'effet de modifications locales des conditions de résonance, pour produire un plasmon de surface dans une couche métallique mince, servant de partie de ladite plate-forme formant capteur à champ évanescent.

34. Procédé selon la revendication 33, **caractérisé en ce que** lesdites modifications des conditions de résonance consistent en une modification de l'angle de résonance, pour irradiation d'une lumière d'excitation destinée à produire un plasmon de surface dans une couche métallique mince servant de partie de ladite plate-forme formant capteur à champ évanescent.

35. Procédé selon la revendication 33, **caractérisé en ce que** lesdites modifications des conditions de résonance consistent en une modification de la longueur d'onde de résonance d'une lumière d'excitation incidente, pour produire un plasmon de surface dans une couche métallique mince servant de partie de ladite plate-forme formant capteur à champ évanescent.

36. Procédé selon l'une des revendications 1-35, **caractérisé en ce que** les modifications devant subir une mesure à résolution locale de signaux optoélectroniques sont provoquées en conséquence de la liaison de substances à détecter à des analytes contenus dans des domaines de mesure discrets dans les échantillons immobilisés, sous l'effet de modifications locales de l'indice de réfraction effectif dans ces domaines sur ladite plate-forme formant capteur à champ évanescent.

37. Procédé selon l'une des revendications 1-32, **caractérisé en ce que** les modifications devant subir une mesure avec une résolution locale de signaux optoélectroniques sont provoquées en conséquence de la liaison de substances à détecter à des analytes contenus dans des domaines de mesure discrets dans les échantillons immobilisés, sous l'effet de modifications locales d'une ou plusieurs luminescences de molécules pouvant subir une luminescence, se trouvant à l'intérieur du champ évanescent de ladite plate-forme formant capteur à champ évanescent.

38. Procédé selon la revendication 37, **caractérisé en ce que** lesdites modifications d'une ou plusieurs luminescences proviennent de molécules ou de nanoparticules pouvant subir une luminescence, qui en tant que marqueurs de luminescence sont liées à une ou plusieurs substances à détecter pour les analytes contenus dans les domaines de mesure discrets.

39. Procédé selon la revendication 38, **caractérisé en ce que**, pour la détection des analytes, on utilise deux marqueurs de luminescence, ou plus, ayant des longueurs d'onde d'émission et/ou des spectres d'excitation différents, ayant de préférence des longueurs d'onde d'émission différentes et des longueurs d'onde d'excitation identiques.

40. Procédé selon l'une des revendications 38-39, **caractérisé en ce que**, pour la détection des analytes, on utilise deux marqueurs de luminescence, ou plus, ayant des temps de diminution de l'émission différents.

41. Procédé selon l'une des revendications 39-40, **caractérisé en ce que**, pour détecter des analytes différents dans un échantillon immobilisé, on utilise deux marqueurs de luminescence, ou plus.

42. Procédé selon l'une des revendications 39-41, **caractérisé en ce que**, pour détecter des analytes différents dans un domaine de mesure, on utilise deux marqueurs de luminescence, ou plus.

43. Procédé selon l'une des revendications 37-42, **caractérisé en ce que** l'irradiation de la lumière d'excitation a lieu par impulsions ayant une durée comprise entre 1 fs et 10 minutes, et la lumière d'émission est mesurée, avec une résolution dans le temps, à partir des domaines de mesure.

44. Procédé selon l'une des revendications 36-43, **caractérisé en ce que** la plate-forme formant capteur à champ évanescent, servant de support solide, comprend un guide d'onde optique, qui comprend une ou plusieurs couches.

45. Procédé selon la revendication 44, **caractérisé en ce que** la plate-forme formant capteur à champ évanescent, servant de support solide, comprend un guide d'onde optique, plan, continu ou subdivisé en domaines guides d'onde, comprenant une ou plusieurs couches.

46. Procédé selon la revendication 45, **caractérisé en ce que** la plate-forme formant capteur à champ évanescent comprend, servant de support solide, comprend un guide d'onde optique à couche mince, plan, comportant pour l'essentiel une couche guide d'onde, optiquement transparente, sur une deuxième couche, elle aussi pour l'essentiel optiquement transparente, servant de couche, et éventuellement une couche intermédiaire, elle aussi pour l'essentiel optiquement transparente, entre la couche guide d'onde et la deuxième couche, qui elle aussi a un indice de réfraction inférieur à celui de la couche guide d'onde.

47. Procédé selon l'une des revendications 1-46, **caractérisé en ce qu'**une lumière d'excitation, provenant d'une ou plusieurs sources de lumière, est injectée dans une couche guide d'onde de la plate-forme formant capteur à champ évanescent, par l'intermédiaire d'un ou plusieurs éléments de couplage optique, qui sont choisis dans le groupe consistant en les coupleurs à prisme, les coupleurs évanescents avec guides d'ondes optiques réunis et champs évanescents en chevauchement, les coupleurs à face frontale, comportant des lentilles de focalisation, disposées en avant d'une face frontale de la couche guide d'onde, de préférence des lentilles cylindriques, et les coupleurs à réseau.

48. Procédé selon la revendication 47, **caractérisé en ce que** l'injection de la lumière d'excitation dans une couche guide d'onde de la plate-forme formant capteur à champ évanescent a lieu à l'aide d'une ou plusieurs structures en réseau destinées à l'injection, qui sont estampées dans ladite couche guide d'onde.

49. Procédé selon l'une des revendications 1-48, **caractérisé en ce que** l'éjection de la lumière guidée dans une couche guide d'onde de la plate-forme formant capteur à champ évanescent a lieu à l'aide d'une ou plusieurs structures en réseau servant à l'éjection, qui sont estampées dans ladite couche guide d'onde, et qui ont la même période et la même profondeur de réseau, que celles des structures en réseau servant à l'injection, ou une période et une profondeur de réseau qui en sont différentes.

50. Procédé selon l'une des revendications 48-49, **caractérisé en ce que** la lumière d'excitation est injectée par une ou plusieurs sources de lumière, en passant par une structure en réseau destinée à l'injection d'une lumière d'excitation, dans une couche guide d'onde de ladite plate-forme formant capteur à champ évanescent, et est envoyée sous forme d'une onde guidée sur des domaines de mesure se trouvant sur la plate-forme formant capteur à champ évanescent, qu'en outre la luminescence produite dans le champ évanescent de ladite onde guidée est détectée, avec résolution locale, par des molécules pouvant subir une luminescence, à l'aide d'un ou plusieurs détecteurs, et on détermine la concentration relative d'un ou plusieurs analytes, à partir de l'intensité relative de ces signaux de luminescence.

51. Procédé selon l'une des revendications 37-50, **caractérisé en ce que**, outre la détermination d'une ou plusieurs luminescences, on détermine les modifications de l'indice de réfraction effectif sur les domaines de mesure.

52. Procédé selon l'une des revendications 33-51, **caractérisé en ce que** la ou les plusieurs luminescences et/ou déterminations de signaux lumineux ont lieu, à la longueur d'onde d'excitation, avec sélectivité de polarisation.

53. Procédé selon l'une des revendications 47-52, **caractérisé en ce que** la ou les plusieurs luminescences sont mesurées en présence d'une polarisation différente de celle de la lumière d'excitation.

54. Plate-forme analytique pour étudier un grand nombre d'échantillons, pour ce qui est de composés contenus dans les échantillons, biologiquement pertinents en tant que participants à des réactions de bioaffinité, servant d'analytes, comprenant :
- une plate-forme formant capteur à champ évanescent, servant de support solide,
- au moins une matrice uni- ou bidimensionnelle de domaines de mesure discrets, dans laquelle se trouvent des partenaires de liaison immobilisés sur ladite plate-forme formant capteur à champ évanescent, pour détecter lesdits analytes dans le cadre d'une réaction de bioaffinité, **caractérisée en ce que**
- lesdits domaines de mesure discrets sont produits par application desdits échantillons ou de fractions desdits échantillons, directement ou après des dilutions supplémentaires de ces échantillons ou de leurs fractions, avec les analytes à détecter qui y sont contenus, servant d'une première pluralité de partenaires de liaison spécifique,
- différents échantillons ou fractions, ou différentes dilutions des échantillons ou de leurs fractions, sont disposés dans des domaines de mesure discrets différents,
- pour ce qui concerne le ou les plusieurs partenaires de liaison immobilisés, qui forment ladite première pluralité de partenaires de liaison spécifique, il s'agit du ou des plusieurs analytes proprement dits, qui sont contenus dans les échantillons à étudier.

55. Plate-forme analytique selon la revendication 54, **caractérisée en ce qu'**un ou plusieurs échantillons ou fractions d'un échantillon sont, avant application sur la plate-forme formant capteur à champ évanescent, servant de support solide, dilués au moins au 1:10, et que différentes dilutions d'une fraction sont, dans des domaines de mesure discrets différents, appliqués sur ladite plate-forme formant capteur à champ évanescent.

56. Plate-forme analytique selon la revendication 54, **caractérisée en ce qu'**un ou plusieurs échantillons ou fractions d'un échantillon sont, avant application sur la plate-forme formant capteur à champ évanescent, servant de support solide, dilués au moins au 1:30, et que différentes dilutions d'une fraction sont, dans des domaines de mesure discrets différents, appliqués sur ladite plate-forme formant capteur à champ évanescent.

57. Plate-forme analytique selon l'une des revendications 54-56, **caractérisée en ce que** les échantillons sont choisis dans le groupe consistant en les extraits de cellules saines ou pathologiques, les extraits de tissus animaux ou humains, ou de tissus végétaux, ainsi que les fluides corporels ou les constituants de ceux-ci.

58. Plate-forme analytique selon l'une des revendications 54-56, **caractérisée en ce que** les échantillons sont choisis dans le groupe consistant en les extraits de cellules stimulées ou non traitées et les extraits de tissus sains ou pathologiques.

59. Plate-forme analytique selon l'une des revendications 54-58, **caractérisée en ce que** les échantillons à étudier sont prélevés d'un organisme ou d'une structure tissulaire ou cellulaire ou de cellules par une méthode choisie dans le groupe consistant en les coupes tissulaires, les biopsies ou la microdissection par capture laser.

60. Plate-forme analytique selon l'une des revendications 54-59, **caractérisée en ce qu'**un échantillon immobilisé comprend la matière de moins de 20 000 cellules.

61. Plate-forme analytique selon l'une des revendications 54-60, **caractérisée en ce qu'**un échantillon immobilisé comprend la matière de moins de 1 000 cellules.

62. Plate-forme analytique selon l'une des revendications 54-61, **caractérisée en ce que** les analytes contenus dans un échantillon immobilisé se présentent sous forme native ou dénaturée.

63. Plate-forme analytique selon l'une des revendications 54-61, **caractérisée en ce que** les analytes contenus dans les échantillons immobilisés se présentent, après traitement à l'urée, sous forme dénaturée, les épitopes de chacun desdits analytes étant librement accessibles à la liaison de ses substances à détecter.

64. Plate-forme analytique selon l'une des revendications 54-63, **caractérisée en ce qu'**on a prélevé différents échantillons appliqués du même organisme ou de la même culture cellulaire.

65. Plate-forme analytique selon la revendication 64, **caractérisée en ce qu'**on a prélevé différents échantillons appliqués, sur différentes positions du même organisme.

66. Plate-forme analytique selon l'une des revendications 54-63, **caractérisée en ce que** différents échantillons appliqués ont été prélevés de différents organismes ou de différentes cultures cellulaires.

67. Plate-forme analytique selon l'une des revendications 54-66, **caractérisée en ce que** la plate-forme formant capteur à champ évanescent comprend, pour améliorer l'adhésivité des échantillons immobilisés appliqués dans des domaines de mesure discrets, une couche promotrice d'adhérence, sur laquelle sont appliqués lesdits échantillons ou lesdites fractions ou dilutions.

68. Plate-forme analytique selon la revendication 67, **caractérisée en ce que** ladite couche promotrice d'adhérence a une épaisseur inférieure à 200 nm, de préférence inférieure à 20 nm.

69. Plate-forme analytique selon l'une des revendications 67-68, **caractérisée en ce que** ladite couche promotrice d'adhérence comprend des composés du groupe consistant en les silanes, les silanes fonctionnalisés, les époxydes, les polymères fonctionnalisés, chargés ou polaires, et les mono- ou multicouches auto-organisées, passives ou fonctionnalisées, les thiols, les alkylphosphates et -phosphonates, les copolymères à blocs multifonctionnels.

70. Plate-forme analytique selon l'une des revendications 67-68, **caractérisée en ce que** ladite couche promotrice d'adhérence comprend des composés du groupe des acides organophosphoriques de formule générale I(A)
Y-B-OPO₃H₂ (IA)
ou des acides organophosphoniques de formule générale I (B)
Y-B-PO₃H₂ (IB)
et leurs sels, où B désigne un radical alkyle, alcényle, alcynyle, aryle, aralkyle, hétéroaryle ou hétéroarylalkyle, et Y représente un atome d'hydrogène ou un groupe fonctionnel de la série hydroxy, carboxy, amino, mono- ou dialkylamino éventuellement substitué par un ou plusieurs substituants alkyle inférieur, ou un groupe acide négatif de la série esters, phosphates, phosphonates, sulfates, sulfonates, maléimide, succinimydyle, époxy ou acrylate.

71. Plate-forme analytique selon l'une des revendications 54-70, **caractérisée en ce qu'**on mélange un ou plusieurs échantillons immobilisés, avant leur application sur la plate-forme formant capteur à champ évanescent, servant de support solide, à une solution de polymères ou de monomères polymérisables, éventuellement en présence d'amorceurs ou d'agents de réticulation chimique.

72. Plate-forme analytique selon la revendication 71, **caractérisée en ce que** ladite solution de polymères ou de monomères polymérisables ou d'agents de réticulation chimique est choisie dans le groupe qui comprend les solutions de polysaccharides.

73. Plate-forme analytique selon l'une des revendications 71-72, **caractérisée en ce que** le mélange du ou des plusieurs échantillons immobilisés à une solution de polymères ou de monomères polymérisables se traduit par l'immobilisation d'une structure en réseau tridimensionnel, avec des constituants d'échantillons, qui sont incorporés, accessibles à des substances à détecter au cours de l'étape suivante d'une réaction de bioaffinité, sur la plate-forme formant capteur à champ évanescent, servant de support solide.

74. Plate-forme analytique selon l'une des revendications 54-73, **caractérisée en ce qu'**une matrice comprend plus de 50, de préférence plus de 500 et d'une manière particulièrement préférée plus de 5 000 domaines de mesure.

75. Plate-forme analytique selon l'une des revendications 54-74, **caractérisée en ce que** les domaines de mesure d'une matrice sont disposés selon une densité de plus de 10, de préférence de plus de 100, d'une manière particulièrement préférée de plus de 1 000 domaines de mesure par centimètre carré.

76. Plate-forme analytique selon l'une des revendications 54-75, **caractérisée en ce qu'**une pluralité de matrices de domaines de mesure sont disposées sur la plate-forme formant capteur à champ évanescent, servant de support solide.

77. Plate-forme analytique selon la revendication 76, **caractérisée en ce qu'**au moins 5, de préférence au moins 50 matrices de domaines de mesure sont disposées sur la plate-forme formant capteur à champ évanescent, servant de support solide.

78. Plate-forme analytique selon l'une des revendications 54-77, **caractérisée en ce que** des domaines entre les domaines de mesure discrets sont appliqués entre les domaines de mesure spatialement séparés par comparaison auxdits analytes et autres constituants de l'échantillon immobilisé appliqué, et des composants qui ne lient pas les analytes par comparaison aux substances à détecter pour lesdits analytes sont également appliqués.

79. Plate-forme analytique selon la revendication 78, **caractérisée en ce que** les composants ne les liant pas sont choisis dans le groupe consistant en les albumines, en particulier la sérumalbumine bovine ou la sérumalbumine humaine, la caséine, les anticorps non spécifiques, polyclonaux ou monoclonaux, étrangers à l'espèce ou empiriquement non spécifiques pour le ou les analytes à détecter, les détergents, l'ADN fragmenté, naturel ou synthétique, qui ne s'hybrident pas aux polynucléotides à analyser, ou aussi les polymères non chargés, mais hydrophiles.

80. Plate-forme analytique selon l'une des revendications 54-79, **caractérisée en ce que**, pour ce qui concerne les analytes à détecter, contenus dans des échantillons immobilisés et appliqués dans des domaines de mesure discrets, il s'agit de composés du groupe consistant en les protéines et les acides nucléiques.

81. Plate-forme analytique selon l'une des revendications 54-79, **caractérisée en ce que**, pour ce qui concerne les analytes à détecter, contenus dans des échantillons immobilisés et appliqués par domaines de mesure discrets, il s'agit de composés du groupe consistant en les protéines cellulaires cytosoliques ou liées à la membrane, en particulier des protéine-kinases participant aux processus de transduction du signal dans les cellules.

82. Plate-forme analytique selon l'une des revendications 54-81, **caractérisée en ce que** la plate-forme formant capteur à champ évanescent comprend une couche métallique mince, de préférence sur une couche intermédiaire disposée en dessous, ayant un indice de réfraction de préférence < 1,5, telle que par exemple du dioxyde de silicium ou du fluorure de magnésium, l'épaisseur de la couche métallique et de l'éventuelle couche intermédiaire étant choisie de façon qu'un plasmon de surface puisse être excité à la longueur d'onde d'une lumière d'excitation irradiée et/ou à la longueur d'onde d'une luminescence produite.

83. Plate-forme analytique selon la revendication 82, **caractérisée en ce que** la couche métallique est choisie dans le groupe comprenant l'or et l'argent.

84. Plate-forme analytique selon la revendication 82, **caractérisée en ce que** la couche métallique a une épaisseur comprise entre 10 nm et 1 000 nm, de préférence entre 30 nm et 200 nm.

85. Plate-forme analytique selon l'une des revendications 54-84, **caractérisée en ce que** la plate-forme formant capteur à champ évanescent, servant de support solide, comprend un guide d'onde optique, comprenant une ou plusieurs couches.

86. Plate-forme analytique selon la revendication 85, **caractérisée en ce que** la plate-forme formant capteur à champ évanescent, servant de support solide, comprend un guide d'onde optique plan, continu ou subdivisé en domaines guides d'ondes discrets, comprenant une ou plusieurs couches.

87. Plate-forme analytique selon la revendication 86, **caractérisée en ce que** la plate-forme formant capteur à champ évanescent, servant de support solide, comprend un guide d'onde à couche mince optique plan, comportant une couche guide d'onde pour l'essentiel optiquement transparente sur une deuxième couche (b), elle aussi pour l'essentiel optiquement transparente, ayant un indice de réfraction inférieur à celui de la couche guide d'onde, et éventuellement une couche intermédiaire, elle aussi pour l'essentiel optiquement transparente, entre la couche guide d'onde et la deuxième couche, ayant un indice de réfraction lui aussi inférieur à celui de la couche guide d'onde.

88. Plate-forme analytique selon l'une des revendications 54-87, **caractérisée en ce qu'**une couche guide d'onde de la plate-forme formant capteur à champ évanescent est en contact optique avec un ou plusieurs éléments de couplage optique, qui permettent l'injection d'une lumière d'injection, provenant d'une ou plusieurs sources de lumière, dans ladite couche guide d'onde, lesdits éléments de couplage optique étant choisis dans le groupe des coupleurs à prisme, des coupleurs évanescents comportant des guides d'ondes optiques réunis et des champs évanescents en chevauchement, les coupleurs à face frontale, ayant des lentilles, de préférence des lentilles cylindriques, de focalisation, disposées en avant d'une face frontale de ladite couche guide d'onde de la plate-forme formant capteur à champ évanescent, et les coupleurs à réseau.

89. Plate-forme analytique selon la revendication 88, **caractérisée en ce que**, dans une couche guide d'onde de la plate-forme formant capteur à champ évanescent, on a estampé une ou plusieurs structures en réseau pour injection, qui permettent l'injection d'une lumière d'excitation provenant d'une ou plusieurs sources de lumière.

90. Plate-forme analytique selon l'une des revendications 54-88, **caractérisée en ce que**, dans une couche guide d'onde de la plate-forme formant capteur à champ évanescent, une ou plusieurs structures en réseau sont estampées, pour une éjection avec une période de réseau et une profondeur de réseau identiques ou différentes de celles des structures de réseau destinées à l'injection, qui permettent l'éjection d'une lumière guidée dans ladite couche guide d'onde.

91. Utilisation d'un procédé selon l'une des revendications 1-53 ou d'une plate-forme analytique selon l'une des revendications 54-90 pour l'analyse quantitative et/ou qualitative destinée à déterminer des analytes chimiques, biochimiques ou biologiques dans le cadre d'un procédé de criblage en recherche pharmacologique, en chimie combinatoire, en développement clinique ou préclinique, pour des études de liaison en temps réel et pour déterminer des paramètres cinétiques dans le cadre d'un criblage par affinité et en recherche, pour des déterminations qualitatives et quantitatives d'analytes, ainsi que pour la détection d'anticorps, d'antigènes, de micro-organismes pathogènes ou de bactéries dans la recherche et le développement de produits pharmaceutiques, en diagnostic humain ou vétérinaire, en recherche et développement de produits agrochimiques, en diagnostic végétal symptomatique et présymptomatique, en stratification des patients lors du développement de produits pharmaceutiques, et pour le choix de médicaments thérapeutiques, pour la détection de micro-organismes pathogènes, de substances nuisibles et d'organismes pathogènes lors de l'analyse de produits alimentaires et de l'environnement.
